# EUROPEAN PATENT APPLICATION

(11) **EP 4 567 038 A1**
(43) Date of publication of application: **11.06.2025**
(21) Application number: 23851793.2
(22) Date of filing: 08.08.2023
(51) Int. Cl.: C07D 487/04, A61K 31/498

(54) **NOVEL PRMT5 INHIBITOR AND USE THEREOF**

(30) Priority: 09.08.2022 CN 202210951874; 26.09.2022 CN 202211173943; 22.11.2022 CN 202211473887; 17.01.2023 CN 202310080068; 14.04.2023 CN 202310402301; 10.07.2023 CN 202310842267
(71) Applicant: Shanghai Apeiron Therapeutics Company Limited, Shanghai 201210 (CN)
(72) Inventor: YAO, Bing, Shanghai 201210 (CN); GU, Xiaohui, Shanghai 201210 (CN)
(74) Representative: Bayramoglu et al.
(86) International application number: PCT/CN2023/111604
(87) International publication number: WO 2024/032572

(57) **Abstract**

The present invention describes a novel molecule having protein arginine methyltransferase 5 inhibitory activity, and synthetic and use methods of the compound. Specifically, the present invention describes the compound of formula (I) or pharmaceutically acceptable salts, hydrates or solvates thereof, and synthetic and use methods of the compound.

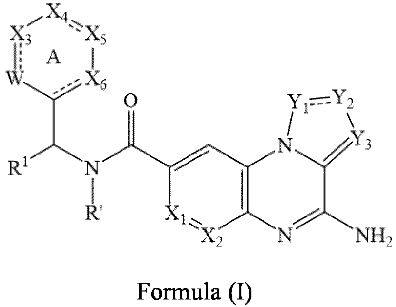

## Description

The present application claims the priority to the following Chinese patent applications:
(1) A Chinese patent application filed to State Intellectual Property Office of the People's Republic of China on August 9, 2022 with the application number of 202210951874.X, titled "Novel PRMT5 inhibitor and use thereof";
(2) A Chinese patent application filed to State Intellectual Property Office of the People's Republic of China on September 26, 2022 with the application number of 202211173943.5, titled "Novel PRMT5 inhibitor and use thereof";
(3) A Chinese patent application filed to State Intellectual Property Office of the People's Republic of China on November 22, 2022 with the application number of 202211473887.7, titled "Novel PRMT5 inhibitor and use thereof";
(4) A Chinese patent application filed to State Intellectual Property Office of the People's Republic of China on January 17, 2023 with the application number of 202310080068.4, titled "Novel PRMT5 inhibitor and use thereof";
(5) A Chinese patent application filed to State Intellectual Property Office of the People's Republic of China on April 14, 2023 with the application number of 202310402301.6, titled "Novel PRMT5 inhibitor and use thereof";
(6) A Chinese patent application filed to State Intellectual Property Office of the People's Republic of China on July 10, 2023 with the application number of 202310842267.4, titled "Novel PRMT5 inhibitor and use thereof", the entire contents of which are incorporated herein by reference.

### TECHNICAL FIELD

This application belongs to the field of drug synthesis, and particularly to a PRMT5 inhibitor and use thereof.

### BACKGROUND TECHNOLOGY

Epigenetic alterations are key mediators that drive and maintain the malignant phenotype of tumors. Changes in DNA methylation, histone acetylation and methylation, noncoding RNA, and post-translational modifications are all epigenetic driving forces of cancer development, independent of changes in the DNA sequence. Arginine methylation is an important kind of post-translational modifications that affect cell growth and proliferation, apoptosis, angiogenesis and metastasis by regulating transcription and post-transcriptional RNA processing. There are three types of methylarginine, ω-NG, N'G-asymmetric dimethylarginine (ADMA) and ω-NG, N'G-symmetric dimethylarginine (SDMA). This modification is catalyzed by the protein arginine methyltransferase (PRMT) family, which transfers methyl from S-adenosylmethionine (AdoMet) to histone and nonhistone arginine side chains. Nine PRMT genes are annotated in the human genome, and classified as Type I (PRMT1, 2, 3, 4, 6 and 8), Type II (PRMT5 and PRMT9), and Type III enzyme (PRMT7) based on the type of methylarginine produced. PRMT5 is primarily a type II enzyme that catalyzes the symmetric dimethylation of arginine. PRMT5 was first discovered in a two-hybrid experiment that test the proteins interacting with Janus tyrosine kinase (Jak2).

PRMT5 is a universal transcriptional repressor that forms complexes with other transcription factors, including BRG1 and Hbrm, Blimp1 and Snail. PRMT5 participates in different cell biological processes through methylation of substrates in the cytoplasm and nucleus, including histone H4 residue Arg3 (H4R3) and H3 residue Arg8 (H3R8). H4R3 methylation is associated with transcriptional repression, while H3R8 methylation is considered to be associated with both transcriptional activation and transcriptional repression. In addition to direct induction of inhibitory histone labeling by PRMT5, the role of this enzyme in gene silencing is mediated through the formation of multiple inhibitory protein complexes including NuRD fractions, HDACs, MDB proteins and DNA methyltransferase. PRMT5 affects its substrate specificity by interacting with some binding proteins. The core component of this protein complex is MEP50. MEP50 is required for the enzymatic activity of PRMT5. Studies have found that PRMT5 can methylate proteins participating RNA splicing, such as SmD3, which can be used to track the chemical activity of PRMT5 in cellular organism.

PRMT5 plays an important role in tumorigenesis. Studies have found that PRMT5 expression is up-regulated in a variety of tumors, including lymphoma, lung cancer, breast cancer and colorectal cancer. In addition, PRMT5 expression is increased in the samples of mantle cell lymphoma (MCL) patient, and PRMT5 knockout can inhibit MCL cell proliferation, indicating that PRMT5 plays an important role in MCL. Overexpression of PRMT5 promotes cell proliferation which is inhibited by PRMT5 knockout in the cell lines of melanoma, breast and lung cancer. Therefore, PRMT5 is a potential target for cancer therapy.

Loss of methylthioadenosine phosphorylase (MTAP) makes the cells selectively dependent on PRMT5 and its binding protein of WDR77. MTAP is often lost due to its proximity to the tumor suppressor gene of CDKN2A which is generally missing. The concentration of methionine adenosine (MTA, the metabolite cleaved by MTAP) is increased in the cells with MTAP deficiency. MTA has a similar structure to S-adenosylmethionine (SAM). With increase of concentration, MTA acts as an intrinsic selective inhibitor to inhibit the binding of SAM to PRMT5, thus inhibiting the methyltransferase activity of PRMT5.

The most significant structural difference between MTAP-deficient cancer cells and MTAP wild-type cancer cells is the PRMT5-MTA complex produced in MTAP-deficient cancer cells due to accumulation of MTA concentration. The inhibitor developed for the PRMT5-MTA complex can selectively target cancer cells with MTAP deficiency, with little effect on normal cells, which greatly improves the therapeutic index. Thus, the identification and development of small molecules that inhibit PRMT5 activity would be useful as a therapeutic method for treating various PRMT5-related diseases or disorders (e.g., cancer).

### CONTENT OF THE INVENTION

In order to solve the technical problem of the present invention, the present invention provides a type of novel structural compounds with excellent inhibitory activity against PRMT5.

Specifically, the present invention provides a compound of formula (I), and a pharmaceutically acceptable salt, an ester, a prodrug, a stereoisomer or an isotopic derivative thereof. 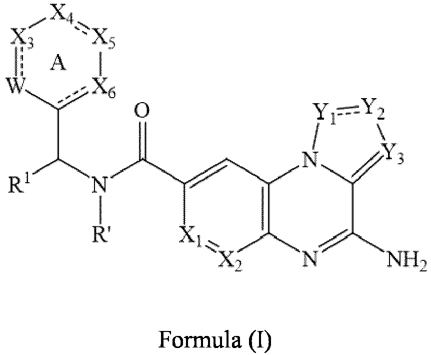
wherein W represents N or CR^{W};
wherein X₃ represents N or CR^{X3}; X₄ represents N or CR^{X4}; X₅ represents N or CR^{X5}; and
X₆ represents N or CR^{X6};
wherein, when X₃ represents CR^{X3}, R^{X3} represents hydrogen, deuterium, C₁-C₆ alkyl, deuterated C₁-C₆ alkyl, C₂-C₆ alkenyl, C₂-C₆ alkynyl, halogen, -OR^{a}, -SR^{a}, -S(O)₂R^{a}, - S(O)R^{a}, -P(O) R^{a}R^{b}, -CN, -SF₅, -NR^{a}R^{b}, C₁-C₆ haloalkyl, C₁-C₆ haloalkoxy, hydroxy(C₁-C₆ alkyl) or C₃-C₁₀ cycloalkyl, C₆-C₁₀ cycloalkenyl, 4- to 10-membered heterocycloalkyl, 6- to 10-membered heterocycloalkenyl, C₆-C₁₀ aryl, 5- to 10-membered heteroaryl, wherein the said C₃-C₁₀ cycloalkyl, C₆-C₁₀ cycloalkenyl, 4- to 10-membered heterocycloalkyl, 6- to 10-membered heterocycloalkenyl, C₆-C₁₀ aryl and 5- to 10-membered heteroaryl can be furtherly substituted with 0-4 substituents selected from: deuterium, halogen, C₁-C₆ alkyl, C₂-C₆ alkenyl, C₂-C₆ alkynyl, C₃-C₁₀ cycloalkyl, -OR^{a}, oxo, hydroxy(C₁-C₆ alkyl), NR^{a}R^{b}, - CN, C₁-C₆ haloalkyl, C₁-C₆ haloalkoxy, -SO₃R^{a}, -SR^{a}, -SF₅, -C(O)R^{a}, -C(O)OR^{a}, -OC(O)R^{a}, OC(O)NR^{a}R^{b}, -NR^{a}COR^{b} or -CONR^{a}R^{b};
wherein, when X₄ represents CR^{X4}, R^{X4} represents hydrogen, deuterium, C₁-C₆ alkyl, deuterated C₁-C₆ alkyl, C₂-C₆ alkenyl, C₂-C₆ alkynyl, halogen, -OR^{a}, -SR^{a}, -S(O)₂R^{a}, - S(O)R^{a}, -P(O) R^{a}R^{b}, -CN, -SF₅, -NR^{a}R^{b}, C₁-C₆ haloalkyl, C₁-C₆ haloalkoxy, hydroxy(C₁-C₆ alkyl) or C₃-C₁₀ cycloalkyl, C₆-C₁₀ cycloalkenyl, 4- to 10-membered heterocycloalkyl, 6- to 10-membered heterocycloalkenyl, C₆-C₁₀ aryl, 5- to 10-membered heteroaryl, wherein the said C₃-C₁₀ cycloalkyl, C₆-C₁₀ cycloalkenyl, 4- to 10-membered heterocycloalkyl, 6- to 10-membered heterocycloalkenyl, C₆-C₁₀ aryl and 5- to 10-membered heteroaryl can be furtherly substituted with 0-4 substituents selected from: deuterium, halogen, C₁-C₆ alkyl, C₂-C₆ alkenyl, C₂-C₆ alkynyl, C₃-C₆ cycloalkyl, -OR^{a}, oxo, hydroxy(C₁-C₆ alkyl), NR^{a}R^{b}, - CN, C₁-C₆ haloalkyl, C₁-C₆ haloalkoxy, -SO₃R^{a}, -SR^{a}, -SF₅, -C(O)R^{a}, -C(O)OR^{a}, -OC(O)R^{a}, OC(O)NR^{a}R^{b}, -NR^{a}COR^{b} or -CONR^{a}R^{b};
wherein, when X₅ represents CR^{X5}, R^{X5} represents hydrogen, deuterium, C₁-C₆ alkyl, deuterated C₁-C₆ alkyl, C₂-C₆ alkenyl, C₂-C₆ alkynyl, halogen, -OR^{a}, -SR^{a}, -S(O)₂R^{a}, - S(O)R^{a}, -P(O) R^{a}R^{b}, -CN, -SF₅, -NR^{a}R^{b}, C₁-C₆ haloalkyl, C₁-C₆ haloalkoxy, hydroxy(C₁-C₆ alkyl) or C₃-C₁₀ cycloalkyl, C₆-C₁₀ cycloalkenyl, 4- to 10-membered heterocycloalkyl, 6- to 10-membered heterocycloalkenyl, C₆-C₁₀ aryl, 5- to 10-membered heteroaryl, wherein the said C₃-C₁₀ cycloalkyl, C₆-C₁₀ cycloalkenyl, 4- to 10-membered heterocycloalkyl, 6- to 10-membered heterocycloalkenyl, C₆-C₁₀ aryl and 5- to 10-membered heteroaryl can be furtherly substituted with 0-4 substituents selected from: deuterium, halogen, C₁-C₆ alkyl, C₂-C₆ alkenyl, C₂-C₆ alkynyl, C₃-C₁₀ cycloalkyl, -OR^{a}, oxo, hydroxy(C₁-C₆ alkyl), NR^{a}R^{b}, - CN, C₁-C₆ haloalkyl, C₁-C₆ haloalkoxy, -SO₃R^{a}, -SR^{a}, -SF₅, -C(O)R^{a}, -C(O)OR^{a}, -OC(O)R^{a}, OC(O)NR^{a}R^{b}, -NR^{a}COR^{b} or -CONR^{a}R^{b};
wherein, when X₆ represents CR^{X6}, R^{X6} represents hydrogen, deuterium, C₁-C₆ alkyl, deuterated C₁-C₆ alkyl, C₂-C₆ alkenyl, C₂-C₆ alkynyl, halogen, -OR^{a}, -SR^{a}, -S(O)₂R^{a}, - S(O)R^{a}, -P(O) R^{a}R^{b}, -CN, -SF₅, -NR^{a}R^{b}, C₁-C₆ haloalkyl, C₁-C₆ haloalkoxy, hydroxy(C₁-C₆ alkyl) or C₃-C₁₀ cycloalkyl, C₆-C₁₀ cycloalkenyl, 4- to 10-membered heterocycloalkyl, 6- to 10-membered heterocycloalkenyl, C₆-C₁₀ aryl, 5- to 10-membered heteroaryl, wherein the said C₃-C₁₀ cycloalkyl, C₆-C₁₀ cycloalkenyl, 4- to 10-membered heterocycloalkyl, 6- to 10-membered heterocycloalkenyl, C₆-C₁₀ aryl and 5- to 10-membered heteroaryl can be furtherly substituted with 0-4 substituents selected from: deuterium, halogen, C₁-C₆ alkyl, C₂-C₆ alkenyl, C₂-C₆ alkynyl, C₃-C₁₀ cycloalkyl, -OR^{a}, oxo, hydroxy(C₁-C₆ alkyl), NR^{a}R^{b}, - CN, C₁-C₆ haloalkyl, C₁-C₆ haloalkoxy, -SO₃R^{a}, -SR^{a}, -SF₅, -C(O)R^{a}, -C(O)OR^{a}, -OC(O)R^{a}, OC(O)NR^{a}R^{b}, -NR^{a}COR^{b} or -CONR^{a}R^{b};
wherein, the ring A may further optionally fuse a 5- to 6-membered saturated or unsaturated ring at the chemical bond between X₃ and X₄, and may further contain 0-3 heteroatoms selected from O, N, and S; further, the ring may be substituted with 0-3 substitutions selected from the group consisting of: deuterium, C₁-C₆ alkyl, deuterated C₁-C₆ alkyl, C₂-C₆ alkenyl, C₂-C₆ alkynyl, halogen, -OR^{a}, -SR^{a}, -S(O)₂R^{a}, -S(O)R^{a}, -P(O)R^{a}R^{b}, -CN, -SF₅, - NR^{a}R^{b}, C₁-C₆ haloalkyl, C₁-C₆ haloalkoxy, hydroxy(C₁-C₆ alkyl), or C₃-C₁₀ cycloalkyl, C₆-C₁₀ cycloalkenyl, 4- to 10-membered heterocycloalkyl, 6- to 10-membered heterocycloalkenyl, C₆-C₁₀ aryl, or 5- to 10-membered heteroaryl, wherein the said C₃-C₁₀ cycloalkyl, C₆-C₁₀ cycloalkenyl, 4- to 10-membered heterocycloalkyl, 6- to 10-membered heterocycloalkenyl, C₆-C₁₀ aryl, or 5- to 10-membered heteroaryl can be furtherly substituted with 0-4 substituents selected from the group consisting of: deuterated, halogen, C₁-C₆ alkyl, C₂-C₆ alkenyl, C₂-C₆ alkynyl, C₃-C₁₀ cycloalkyl, -OR^{a}, oxo, hydroxy(C₁-C₆ alkyl), NR^{a}R^{b}, -CN, C₁-C₆ haloalkyl, C₁-C₆ haloalkoxy, -SO₅R^{a}, -SR^{a}, -SF₅, -C(O)R^{a}, - C(O)OR^{a}, -OC(O)R^{a}, -OC(O)NR^{a}R^{b}, -NR^{a}COR^{b}, and -CONR^{a}R^{b};
wherein, the ring A may further optionally fuse a 5- to 6-membered saturated or unsaturated ring at the chemical bond between X₄ and X₅, and may further contain 0-3 heteroatoms selected from O, N, and S; further, the ring may be substituted with 0-3 substitutions selected from the group consisting of: deuterium, C₁-C₆ alkyl, deuterated C₁-C₆ alkyl, C₂-C₆ alkenyl, C₂-C₆ alkynyl, halogen, -OR^{a}, -SR^{a}, -S(O)₂R^{a}, -S(O)R^{a}, -P(O)R^{a}R^{b}, -CN, -SF₅, - NR^{a}R^{b}, C₁-C₆ haloalkyl, C₁-C₆ haloalkoxy, hydroxy(C₁-C₆ alkyl), or C₃-C₁₀ cycloalkyl, C₆-C₁₀ cycloalkenyl, 4- to 10-membered heterocycloalkyl, 6- to 10-membered heterocycloalkenyl, C₆-C₁₀ aryl, or 5- to 10-membered heteroaryl, wherein the said C₃-C₁₀ cycloalkyl, C₆-C₁₀ cycloalkenyl, 4- to 10-membered heterocycloalkyl, 6- to 10-membered heterocycloalkenyl, C₆-C₁₀ aryl, or 5- to 10-membered heteroaryl can be furtherly substituted with 0-4 substituents selected from the group consisting of: deuterated, halogen, C₁-C₆ alkyl, C₂-C₆ alkenyl, C₂-C₆ alkynyl, C₃-C₁₀ cycloalkyl, -OR^{a}, oxo, hydroxy(C₁-C₆ alkyl), NR^{a}R^{b}, -CN, C₁-C₆ haloalkyl, C₁-C₆ haloalkoxy, -SO₄R^{a}, -SR^{a}, -SF₅, -C(O)R^{a}, - C(O)OR^{a}, -OC(O)R^{a}, OC(O)NR^{a}R^{b}, -NR^{a}COR^{b}, and -CONR^{a}R^{b};
wherein, the ring A may further optionally fuse a 5- to 6-membered saturated or unsaturated ring at the chemical bond between X₅ and X₆, and may further contain 0-3 heteroatoms selected from O, N, and S; further, the ring may be substituted with 0-3 substitutions selected from the group consisting of: deuterium, C₁-C₆ alkyl, deuterated C₁-C₆ alkyl, C₂-C₆ alkenyl, C₂-C₆ alkynyl, halogen, -OR^{a}, -SR^{a}, -S(O)₂R^{a}, -S(O)R^{a}, -P(O)R^{a}R^{b}, -CN, -SF₅, - NR^{a}R^{b}, C₁-C₆ haloalkyl, C₁-C₆ haloalkoxy, hydroxy(C₁-C₆ alkyl), or C₃-C₁₀ cycloalkyl, C₆-C₁₀ cycloalkenyl, 4- to 10-membered heterocycloalkyl, 6- to 10-membered heterocycloalkenyl, C₆-C₁₀ aryl, or 5- to 10-membered heteroaryl substituted with 0-4 substituents selected from the group consisting of: deuterated, halogen, C₁-C₆ alkyl, C₂-C₆ alkenyl, C₂-C₆ alkynyl, C₃-C₁₀ cycloalkyl, -OR^{a}, oxo, hydroxy(C₁-C₆ alkyl), NR^{a}R^{b}, -CN, C₁-C₆ haloalkyl, C₁-C₆ haloalkoxy, -SO₅R^{a}, -SR^{a}, -SF₅, -C(O)R^{a}, -C(O)OR^{a}, -OC(O)R^{a}, OC(O)NR^{a}R^{b}, -NR^{a}COR^{b}, and -CONR^{a}R^{b};
wherein, R' represents C₁-C₆ alkyl, C₃-C₁₀ cycloalkyl, 4- to 10-membered heterocycloalkyl, C₆-C₁₀ aryl, or 5- to 10-membered heteroaryl, wherein the said C₁-C₆ alkyl, C₃-C₁₀ cycloalkyl, 4- to 10-membered heterocycloalkyl, C₆-C₁₀ aryl, or 5- to 10-membered heteroaryl can be furtherly substituted with 0-3 substituents optionally selected from the group consisting of: deuterated, halogen, C₁-C₆ alkyl, C₂-C₆ alkenyl, C₂-C₆ alkynyl, C₃-C₆ cycloalkyl, 3- to 6-membered saturated or unsaturated aliphatic monoheterocyclic group, - OR^{a}, oxo, hydroxy(C₁-C₆ alkyl), NR^{a}R^{b}, -CN, C₁-C₆ haloalkyl, C₁-C₆ haloalkoxy, -SO₃R^{a}, - SR^{a}, -S(O)₂R^{a}, -S(O)R^{a}, -SF₅, -C(O)R^{a}, -C(O)OR^{a}, -OC(O)R^{a}, -OC(O)NR^{a}R^{b}, -NR^{a}COR^{b}, and -CONR^{a}R^{b};
preferably, R' represents -CHR²R³;
wherein, R¹ represents hydrogen, deuterium, halogen, C₁-C₆ alkyl, deuterated C₁-C₆ alkyl, C₁-C₆ haloalkyl, hydroxy(C₁-C₆ alkyl), or C₃-C₁₀ cycloalkyl;
wherein, R² and R³ respectively represent hydrogen, deuterium, -OR^{a}, halogen, -CN, C₁-C₆ alkyl, C₁-C₆ haloalkyl, C₁-C₆ haloalkoxy, hydroxy(C₁-C₆ alkyl), or C₃-C₁₀ cycloalkyl, 4- to 10-membered heterocycloalkyl, C₆-C₁₀ aryl, or 5- to 10-membered heteroaryl, wherein the said C₃-C₁₀ cycloalkyl, 4- to 10-membered heterocycloalkyl, C₆-C₁₀ aryl, or 5- to 10-membered heteroaryl can be furtherly substituted with 0-3 substituents optionally selected from the group consisting of: deuterated, halogen, C₁-C₆ alkyl, C₂-C₆ alkenyl, C₂-C₆ alkynyl, C₃-C₆ cycloalkyl, -OR^{a}, oxo, hydroxy(C₁-C₆ alkyl), NR^{a}R^{b}, -CN, C₁-C₆ haloalkyl, C₁-C₆ haloalkoxy, -SO₃R^{a}, -SR^{a}, -S(O)₂R^{a}, -S(O)R^{a}, -SF₅, -C(O)R^{a}, -C(O)OR^{a}, -OC(O)R^{a}, - OC(O)NR^{a}R^{b}, -NR^{a}COR^{b}, and -CONR^{a}R^{b};
wherein, when W represents CR^{W}, R^{W} is selected from hydrogen, deuterium, C₁-C₆ alkyl, deuterated C₁-C₆ alkyl, halogen, -OR^{a}, -SR^{a}, -S(O)₂R^{a}, -S(O)R^{a}, -CN, -SF₅, -NR^{a}R^{b}, C₁-C₆ haloalkyl, hydroxy(C₁-C₆ alkyl), and C₁-C₆ haloalkoxy;
wherein X₁ represents N or CR^{X1};
wherein X₂ represents N or CR^{X2};
wherein Y₁ represents CR^{Y1}R^{Y1'}, NR^{Y1}, O, S, or Se;
wherein Y₂ represents CR^{Y2}R^{Y2'}, NR^{Y2}, O, S, or Se;
wherein Y₃ represents CR^{Y3}R^{Y3'}, NR^{Y3}, O, S, or Se;
wherein R^{X1} and R^{X2} respectively represent hydrogen, deuterium, C₁-C₆ alkyl, deuterated C₁-C₆ alkyl, C₁-C₆ haloalkyl, C₁-C₆ haloalkoxy, hydroxy(C₁-C₆ alkyl), C₂-C₆ alkenyl, C₂-C₆ alkynyl, -OR^{a}, -SR^{a}, -S(O)₂R^{a}, -S(O)R^{a}, -CN, -OC(O)R^{a}, -OCONR^{a}R^{b}, halogen, -SO₃R^{a}, - NR^{a}R^{b}, or -SF₅;
wherein R^{Y1}, R^{Y1'}, R^{Y2}, R^{Y2'}, R^{Y3}, and R^{Y3'} respectively represent absent, hydrogen, deuterium, C₁-C₆ alkyl, deuterated C₁-C₆ alkyl, C₁-C₆ haloalkyl, C₁-C₆ haloalkoxy, hydroxy(C₁-C₆ alkyl), C₂-C₆ alkenyl, C₂-C₆ alkynyl, C₃-C₆ cycloalkyl, -OR^{a}, -SR^{a}, -S(O)₂R^{a}, -S(O)R^{a}, -CN, -OC(O)R^{a}, -OCONR^{a}R^{b}, halogen, -SO₃R^{a}, -NR^{a}R^{b}, or -SF₅;
wherein, -̅ -̅ -̅ represents a single bond or a double bond; and
wherein R^{a} and R^{b} respectively represent hydrogen, deuterium, halogen, C₁-C₆ alkyl, C₃-C₆ cycloalkyl, C₁-C₆ haloalkyl, or R^{a} and R^{b} can be taken together with the atom to which they are attached form a 3- to 14-membered saturated or unsaturated ring, wherein the ring may optionally contain 0-2 heteroatoms selected from O, S, and N.

In additional embodiment, the present invention provides a compound, its pharmaceutically acceptable salts, esters, prodrugs, stereoisomers or isotopic derivatives thereof having the structure of formula (II): 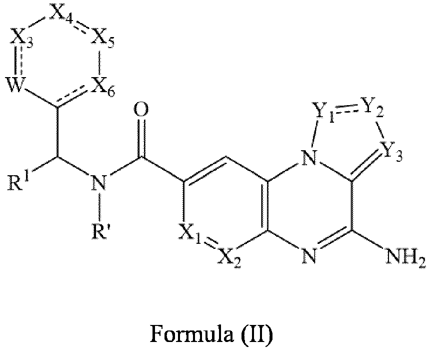
wherein W represents N or CR^{W};
wherein X₃ represents N or CR^{X3}; X₄ represents N or CR^{X4}; X₅ represents N or CR^{X5}; and
X₆ represents N or CR^{X6};
wherein, when X₃ represents CR^{X3}, R^{X3} represents hydrogen, deuterium, C₁-C₆ alkyl, deuterated C₁-C₆ alkyl, C₂-C₆ alkenyl, C₂-C₆ alkynyl, halogen, -OR^{a}, -SR^{a}, -S(O)₂R^{a}, - S(O)R^{a}, -P(O) R^{a}R^{b}, -CN, -SF₅, -NR^{a}R^{b}, C₁-C₆ haloalkyl, C₁-C₆ haloalkoxy, hydroxy(C₁-C₆ alkyl) or C₃-C₁₀ cycloalkyl, C₆-C₁₀ cycloalkenyl, 4- to 10-membered heterocycloalkyl, 6- to 10-membered heterocycloalkenyl, C₆-C₁₀ aryl, 5- to 10-membered heteroaryl, wherein the said C₃-C₁₀ cycloalkyl, C₆-C₁₀ cycloalkenyl, 4- to 10-membered heterocycloalkyl, 6- to 10-membered heterocycloalkenyl, C₆-C₁₀ aryl and 5- to 10-membered heteroaryl can be furtherly substituted with 0-4 substituents selected from: deuterium, halogen, C₁-C₆ alkyl, C₂-C₆ alkenyl, C₂-C₆ alkynyl, C₃-C₁₀ cycloalkyl, -OR^{a}, oxo, hydroxy(C₁-C₆ alkyl), NR^{a}R^{b}, - CN, C₁-C₆ haloalkyl, C₁-C₆ haloalkoxy, -SO₃R^{a}, -SR^{a}, -SF₅, -C(O)R^{a}, -C(O)OR^{a}, -OC(O)R^{a}, OC(O)NR^{a}R^{b}, -NR^{a}COR^{b} or -CONR^{a}R^{b};
wherein, when X₄ represents CR^{X4}, R^{X4} represents hydrogen, deuterium, C₁-C₆ alkyl, deuterated C₁-C₆ alkyl, C₂-C₆ alkenyl, C₂-C₆ alkynyl, halogen, -OR^{a}, -SR^{a}, -S(O)₂R^{a}, - S(O)R^{a}, -P(O) R^{a}R^{b}, -CN, -SF₅, -NR^{a}R^{b}, C₁-C₆ haloalkyl, C₁-C₆ haloalkoxy, hydroxy(C₁-C₆ alkyl) or C₃-C₁₀ cycloalkyl, C₆-C₁₀ cycloalkenyl, 4- to 10-membered heterocycloalkyl, 6- to 10-membered heterocycloalkenyl, C₆-C₁₀ aryl, 5- to 10-membered heteroaryl, wherein the said C₃-C₁₀ cycloalkyl, C₆-C₁₀ cycloalkenyl, 4- to 10-membered heterocycloalkyl, 6- to 10-membered heterocycloalkenyl, C₆-C₁₀ aryl and 5- to 10-membered heteroaryl can be furtherly substituted with 0-4 substituents selected from: deuterium, halogen, C₁-C₆ alkyl, C₂-C₆ alkenyl, C₂-C₆ alkynyl, C₃-C₆ cycloalkyl, -OR^{a}, oxo, hydroxy(C₁-C₆ alkyl), NR^{a}R^{b}, - CN, C₁-C₆ haloalkyl, C₁-C₆ haloalkoxy, -SO₃R^{a}, -SR^{a}, -SF₅, -C(O)R^{a}, -C(O)OR^{a}, -OC(O)R^{a}, OC(O)NR^{a}R^{b}, -NR^{a}COR^{b} or -CONR^{a}R^{b};
wherein, when X₅ represents CR^{X5}, R^{X5} represents hydrogen, deuterium, C₁-C₆ alkyl, deuterated C₁-C₆ alkyl, C₂-C₆ alkenyl, C₂-C₆ alkynyl, halogen, -OR^{a}, -SR^{a}, -S(O)₂R^{a}, - S(O)R^{a}, -P(O) R^{a}R^{b}, -CN, -SF₅, -NR^{a}R^{b}, C₁-C₆ haloalkyl, C₁-C₆ haloalkoxy, hydroxy(C₁-C₆ alkyl) or C₃-C₁₀ cycloalkyl, C₆-C₁₀ cycloalkenyl, 4- to 10-membered heterocycloalkyl, 6- to 10-membered heterocycloalkenyl, C₆-C₁₀ aryl, 5- to 10-membered heteroaryl, wherein the said C₃-C₁₀ cycloalkyl, C₆-C₁₀ cycloalkenyl, 4- to 10-membered heterocycloalkyl, 6- to 10-membered heterocycloalkenyl, C₆-C₁₀ aryl and 5- to 10-membered heteroaryl can be furtherly substituted with 0-4 substituents selected from: deuterium, halogen, C₁-C₆ alkyl, C₂-C₆ alkenyl, C₂-C₆ alkynyl, C₃-C₁₀ cycloalkyl, -OR^{a}, oxo, hydroxy(C₁-C₆ alkyl), NR^{a}R^{b}, - CN, C₁-C₆ haloalkyl, C₁-C₆ haloalkoxy, -SO₃R^{a}, -SR^{a}, -SF₅, -C(O)R^{a}, -C(O)OR^{a}, -OC(O)R^{a}, OC(O)NR^{a}R^{b}, -NR^{a}COR^{b} or -CONR^{a}R^{b};
wherein, when X₆ represents CR^{X6}, R^{X6} represents hydrogen, deuterium, C₁-C₆ alkyl, deuterated C₁-C₆ alkyl, C₂-C₆ alkenyl, C₂-C₆ alkynyl, halogen, -OR^{a}, -SR^{a}, -S(O)₂R^{a}, - S(O)R^{a}, -P(O) R^{a}R^{b}, -CN, -SF₅, -NR^{a}R^{b}, C₁-C₆ haloalkyl, C₁-C₆ haloalkoxy, hydroxy(C₁-C₆ alkyl) or C₃-C₁₀ cycloalkyl, C₆-C₁₀ cycloalkenyl, 4- to 10-membered heterocycloalkyl, 6- to 10-membered heterocycloalkenyl, C₆-C₁₀ aryl, 5- to 10-membered heteroaryl, wherein the said C₃-C₁₀ cycloalkyl, C₆-C₁₀ cycloalkenyl, 4- to 10-membered heterocycloalkyl, 6- to 10-membered heterocycloalkenyl, C₆-C₁₀ aryl and 5- to 10-membered heteroaryl can be furtherly substituted with 0-4 substituents selected from: deuterium, halogen, C₁-C₆ alkyl, C₂-C₆ alkenyl, C₂-C₆ alkynyl, C₃-C₁₀ cycloalkyl, -OR^{a}, oxo, hydroxy(C₁-C₆ alkyl), NR^{a}R^{b}, - CN, C₁-C₆ haloalkyl, C₁-C₆ haloalkoxy, -SO₃R^{a}, -SR^{a}, -SF₅, -C(O)R^{a}, -C(O)OR^{a}, -OC(O)R^{a}, OC(O)NR^{a}R^{b}, -NR^{a}COR^{b} or -CONR^{a}R^{b};
wherein, R' represents C₁-C₆ alkyl, C₃-C₁₀ cycloalkyl, 4- to 10-membered heterocycloalkyl, C₆-C₁₀ aryl, or 5- to 10-membered heteroaryl, wherein the said C₁-C₆ alkyl, C₃-C₁₀ cycloalkyl, 4- to 10-membered heterocycloalkyl, C₆-C₁₀ aryl, or 5- to 10-membered heteroaryl can be furtherly substituted with 0-3 substituents optionally selected from the group consisting of: deuterated, halogen, C₁-C₆ alkyl, C₂-C₆ alkenyl, C₂-C₆ alkynyl, C₃-C₆ cycloalkyl, 3- to 6-membered saturated or unsaturated aliphatic monoheterocyclic group, - OR^{a}, oxo, hydroxy(C₁-C₆ alkyl), NR^{a}R^{b}, -CN, C₁-C₆ haloalkyl, C₁-C₆ haloalkoxy, -SO₃R^{a}, - SR^{a}, -S(O)₂R^{a}, -S(O)R^{a}, -SF₅, -C(O)R^{a}, -C(O)OR^{a}, -OC(O)R^{a}, -OC(O)NR^{a}R^{b}, -NR^{a}COR^{b}, and -CONR^{a}R^{b};
preferably, R' represents -CHR²R³;
wherein, R¹ represents hydrogen, deuterium, halogen, C₁-C₆ alkyl, deuterated C₁-C₆ alkyl, C₁-C₆ haloalkyl, hydroxy(C₁-C₆ alkyl), or C₃-C₁₀ cycloalkyl;
wherein, R² and R³ respectively represent hydrogen, deuterium, -OR^{a}, halogen, -CN, -C₁-C₆ alkyl, C₁-C₆ haloalkyl, C₁-C₆ haloalkoxy, hydroxy(C₁-C₆ alkyl) or C₃-C₁₀ cycloalkyl, 4- to 10-membered heterocycloalkyl, C₆-C₁₀ aryl, or 5- to 10-membered heteroaryl, wherein the said C₃-C₁₀ cycloalkyl, 4- to 10-membered heterocycloalkyl, C₆-C₁₀ aryl, or 5- to 10-membered heteroaryl can be furtherly substituted with 0-3 substituents optionally selected from the group consisting of: deuterated, halogen, C₁-C₆ alkyl, C₂-C₆ alkenyl, C₂-C₆ alkynyl, C₃-C₆ cycloalkyl, -OR^{a}, oxo, hydroxy(C₁-C₆ alkyl), NR^{a}R^{b}, -CN, C₁-C₆ haloalkyl, C₁-C₆ haloalkoxy, -SO₃R^{a}, -SR^{a}, -S(O)₂R^{a}, -S(O)R^{a}, -SF₅, -C(O)R^{a}, -C(O)OR^{a}, -OC(O)R^{a}, - OC(O)NR^{a}R^{b}, -NR^{a}COR^{b} and -CONR^{a}R^{b};
wherein, when W represents CR^{W}, R^{W} is selected from hydrogen, deuterium, C₁-C₆ alkyl, halogen, -OR^{a}, -SR^{a}, -S(O)₂R^{a}, -S(O)R^{a}, -CN, -SF₅, -NR^{a}R^{b}, C₁-C₆ haloalkyl, hydroxy(C₁-C₆ alkyl), and C₁-C₆ haloalkoxy;
wherein X₁ represents N or CR^{X1};
wherein X₂ represents N or CR^{X2};
wherein Y₁ represents CR^{Y1}R^{Y1'}, NR^{Y1}, O, S, or Se;
wherein Y₂ represents CR^{Y2}R^{Y2'}, NR^{Y2}, O, S, or Se;
wherein Y₃ represents CR^{Y3}R^{Y3'}, NR^{Y3}, O, S, or Se;
wherein R^{X1} and R^{X2} respectively represent hydrogen, deuterium, C₁-C₆ alkyl, deuterated C₁-C₆ alkyl, C₁-C₆ haloalkyl, C₁-C₆ haloalkoxy, hydroxy(C₁-C₆ alkyl), C₂-C₆ alkenyl, C₂-C₆ alkynyl, -OR^{a}, -SR^{a}, -S(O)₂R^{a}, -S(O)R^{a}, -CN, -OC(O)R^{a}, -OCONR^{a}R^{b}, halogen, -SO₃R^{a}, - NR^{a}R^{b}, or -SF₅;
wherein R^{Y1}, R^{Y1'}, R^{Y2}, R^{Y2'}, R^{Y3}, and R^{Y3'} respectively represent absent, hydrogen, deuterium, C₁-C₆ alkyl, deuterated C₁-C₆ alkyl, C₁-C₆ haloalkyl, C₁-C₆ haloalkoxy, hydroxy(C₁-C₆ alkyl), C₂-C₆ alkenyl, C₂-C₆ alkynyl, C₃-C₆ cycloalkyl, -OR^{a}, -SR^{a}, -S(O)₂R^{a}, -S(O)R^{a}, -CN, -OC(O)R^{a}, -OCONR^{a}R^{b}, halogen, -SO₃R^{a}, -NR^{a}R^{b}, or -SF₅;
wherein, -̅ -̅ -̅ represents a single bond or a double bond; and
wherein R^{a} and R^{b} respectively represent hydrogen, deuterium, halogen, C₁-C₆ alkyl, C₃-C₆ cycloalkyl, C₁-C₆ haloalkyl, or R^{a} and R^{b} can be taken together with the atom to which they are attached form a 3- to 14-membered saturated or unsaturated ring, wherein the ring may optionally contain 0-2 heteroatoms selected from O, S, and N.

In a preferred embodiment of formula I or II, wherein W represents CH or N.

In a preferred embodiment of the formula I or II, wherein X₁ represents CR^{X1} or N, wherein R^{X1} represents hydrogen, deuterium, halogen, -CN, C₁-C₆ alkyl, deuterated C₁-C₆ alkyl, C₁-C₆ alkoxy, C₁-C₆ haloalkyl.

In a preferred embodiment of formula I or II, wherein X₂ represents CH or CD.

In a preferred embodiment of formula I or II, wherein X₃ represents CH, CD or N.

In a preferred embodiment of formula I or II, wherein X₄ represents CR^{X4} or N, wherein R^{X4} represents hydrogen, deuterium, C₁-C₆ alkyl, deuterated C₁-C₆ alkyl, C₁-C₆ alkoxy, C₁-C₆ haloalkyl, C₁-C₆ haloalkoxy, halogen, SF₅, -SO₃R^{a}, -SR^{a}, or cyano or C₃-C₁₀ cycloalkyl, C₆-C₁₀ cycloalkenyl, 4- to 10-membered heterocycloalkyl, 6- to 10-membered heterocycloalkenyl, C₆-C₁₀ aryl, and 5- to 10-membered heteroaryl, wherein the said C₃-C₁₀ cycloalkyl, C₆-C₁₀ cycloalkenyl, 4- to 10-membered heterocycloalkyl, 6- to 10-membered heterocycloalkenyl, C₆-C₁₀ aryl, and 5- to 10-membered heteroaryl can be furtherly substituted with 0-4 substituents selected from: deuterated, halogen, C₁-C₆ alkyl, C₂-C₆ alkenyl, C₂-C₆ alkynyl, C₃-C₆ cycloalkyl, -OR^{a}, oxo, hydroxy(C₁-C₆ alkyl), NR^{a}R^{b}, - P(O)R^{a}R^{b}, -CN, C₁-C₆ haloalkyl, C₁-C₆ haloalkoxy, -SO₃R^{a}, -SR^{a}, -SF₅, -C(O)R^{a}, -C(O)OR^{a}, -OC(O)R^{a}, OC(O)NR^{a}R^{b}, -NR^{a}COR^{b} or -CONR^{a}R^{b}.

In a preferred embodiment of the formula I or II, wherein X₅ represents CR^{X5} or N, wherein R^{X5} represents hydrogen, deuterium, C₁-C₆ alkyl, C₁-C₆ alkoxy, C₁-C₆ haloalkyl, C₁-C₆ haloalkoxy, halogen, SF₅, or cyano.

In a preferred embodiment of formula I or II, wherein X₆ represents CH, CD or N.

In a preferred embodiment of formula I or II, wherein the chemical bond between Y₁ and Y₂ is a double bond.

In a preferred embodiment of formula I or II, wherein Y₁ represents CR^{Y1}, wherein R^{Y1} represents hydrogen, deuterium, C₁-C₆ alkyl, deuterated C₁-C₆ alkyl, C₃-C₆ cycloalkyl, C₁-C₆ alkoxy, C₁-C₆ haloalkyl, C₁-C₆ haloalkoxy, halogen, SF₅, -S(O)₂CH₃ or cyano.

In a preferred embodiment of formula I or II, wherein Y₂ represents N.

In a preferred embodiment of formula I or II, wherein Y₃ represents CH or CD.

In a preferred embodiment of formula I or II, wherein R' represents -CHR²R³, wherein R² and R³ each independently represent hydrogen, deuterium, C₁-C₆ alkyl, or C₃-C₁₀ cycloalkyl, 4- to 10-membered heterocycloalkyl, C₆-C₁₀ aryl, 5- to 10-membered heteroaryl, wherein the said C₃-C₁₀ cycloalkyl, 4- to 10-membered heterocycloalkyl, C₆-C₁₀ aryl, 5- to 10-membered heteroaryl can be furtherly substituted with 0-3 substituents selected from: halogen, C₁-C₆ alkyl, C₂-C₆ alkenyl, C₂-C₆ alkynyl, C₃-C₆ cycloalkyl, -OR^{a}, oxo, hydroxy(C₁-C₆ alkyl), NR^{a}R^{b}, -CN, C₁-C₆ haloalkyl, C₁-C₆ haloalkoxy, -SO₃R^{a}, -SR^{a}, - S(O)₂R^{a}, -S(O)R^{a}, -SF₅, -C(O)R^{a}, -C(O)OR^{a}, -OC(O)R^{a}, -OC(O)NR^{a}R^{b}, -NR^{a}COR^{b} or - CONR^{a}R^{b}.

In a preferred embodiment of formula I or II, wherein R' represents -CHR²R³, wherein R² represents hydrogen, deuterium, C₁-C₆-alkyl; and R³ represent hydrogen, C₁-C₆ alkyl, or C₃-C₁₀ cycloalkyl, 4- to 10-membered heterocycloalkyl, C₆-C₁₀ aryl, 5- to 10-membered heteroaryl, wherein the said C₃-C₁₀ cycloalkyl, 4- to 10-membered heterocycloalkyl, C₆-C₁₀ aryl, 5- to 10-membered heteroaryl can be furtherly substituted with 0-3 substituents selected from the group consisting of: halogen, C₁-C₆ alkyl, C₂-C₆ alkenyl, C₂-C₆ alkynyl, C₃-C₆ cycloalkyl, -OR^{a}, oxo, hydroxy(C₁-C₆ alkyl), NR^{a}R^{b}, -CN, C₁-C₆ haloalkyl, C₁-C₆ haloalkoxy, -SO₃R^{a}, -SR^{a}, -S(O)₂R^{a}, -S(O)R^{a}, -SF₅, -C(O)R^{a}, -C(O)OR^{a}, -OC(O)R^{a}, - OC(O)NR^{a}R^{b}, -NR^{a}COR^{b} or -CONR^{a}R^{b}.

In a preferred embodiment of formula I or II, wherein R' represents -CHR²R³, wherein R² represents hydrogen, deuterium, C₁-C₆ alkyl; R³ represents C₃-C₁₀ cycloalkyl, 4- to 10-membered heterocycloalkyl, C₆-C₁₀ aryl, and 5- to 10-membered heteroaryl, wherein the said C₃-C₁₀ cycloalkyl, 4- to 10-membered heterocycloalkyl, C₆-C₁₀ aryl, 5- to 10-membered heteroaryl can be furtherly substituted with 0-3 substituents selected from: deuterated, halogen, C₁-C₆ alkyl, C₂-C₆ alkenyl, C₂-C₆ alkynyl, C₃-C₆ cycloalkyl, -OR^{a}, oxo, hydroxy(C₁-C₆ alkyl), NR^{a}R^{b}, -S(O)₂R^{a}, -S(O)R^{a}, -CN, C₁-C₆ haloalkyl, C₁-C₆ haloalkoxy, - SO₃R^{a}, -SR^{a}, -S(O)₂R^{a}, -S(O)R^{a}, -SF₅, -C(O)R^{a}, -C(O)OR^{a}, -OC(O)R^{a}, -OC(O)NR^{a}R^{b}, - NR^{a}COR^{b} or -CONR^{a}R^{b}.

In a preferred embodiment of formula I or II, wherein R' represents -CHR²R³, wherein R² represents hydrogen, deuterium, C₁-C₆ alkyl; R³ represents the following groups: 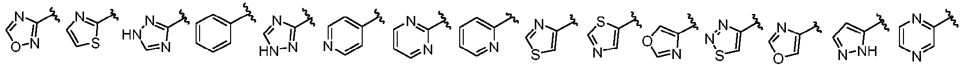 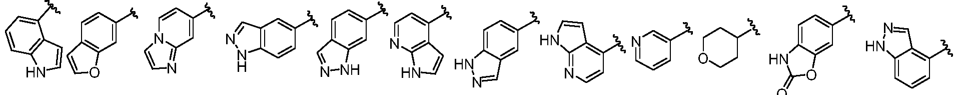 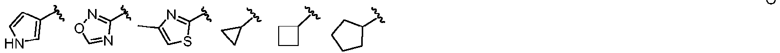 wherein, the above groups can be furtherly substituted with 0-3 deuterated, halogen, C₁-C₆ alkyl, C₂-C₆ alkenyl, C₂-C₆ alkynyl, C₃-C₆ cycloalkyl, -OR^{a}, oxo, hydroxy(C₁-C₆ alkyl), NR^{a}R^{b}, -S(O)₂R^{a}, -S(O)R^{a}, -CN, C₁-C₆ haloalkyl, C₁-C₆ haloalkoxy, -SO₃R^{a}, -SR^{a}, -SF₅, C(O)R^{a}, -C(O)OR^{a}, -OC(O)R^{a}, -OC(O)NR^{a}R^{b}, -NR^{a}COR^{b} or -CONR^{a}R^{b} substituted.

In a preferred embodiment of formula I or II, wherein R' represents C₁-C₆ alkyl substituted with 0-3 substituents selected from: deuterated, halogen, C₁-C₆ alkyl, hydroxy(C₁-C₆ alkyl), -OR^{a}, -CN, NR^{a}R^{b}, C₁-C₆ haloalkyl, C₁-C₆ haloalkoxy.

In a preferred embodiment of formula I or II, wherein R' represents C₁-C₆ alkyl substituted with 0-3 substituents selected from the group consisting of: deuterated, halogen, C₁-C₆ alkyl, hydroxy(C₁-C₆ alkyl), -OR^{a}, -CN, NR^{a}R^{b}, C₁-C₆ haloalkyl, C₃-C₁₀ haloalkoxy.

In a preferred embodiment of formula I or II, wherein R' represents cyclopropyl, cyclobutyl, cyclopentyl, or cyclohexyl, wherein the said cyclopropyl, cyclobutyl, cyclopentyl, or cyclohexyl can be furtherly substituted with 0-3 substituents selected from: deuterated, halogen, C₁-C₆ alkyl, hydroxy(C₁-C₆ alkyl), -OR^{a}, -CN, NR^{a}R^{b}, C₁-C₆ haloalkyl, and C₁-C₆ haloalkoxy.

In a preferred embodiment of formula I or II, wherein R' represents a 4- to 10-membered heterocycloalkyl, C₆-C₁₀ aryl, or 5- to 10-membered heteroaryl, wherin the said 4- to 10-membered heterocycloalkyl, C₆-C₁₀ aryl, or 5- to 10-membered heteroaryl can be furtherly substituted with 0-3 substituents selected from: deuterated, halogen, C₁-C₆ alkyl, hydroxy(C₁-C₆ alkyl), -OR^{a}, -CN, NR^{a}R^{b}, C₁-C₆ haloalkyl, and C₁-C₆ haloalkoxy.

In a preferred embodiment of formula I or II, wherein R' represents following groups: 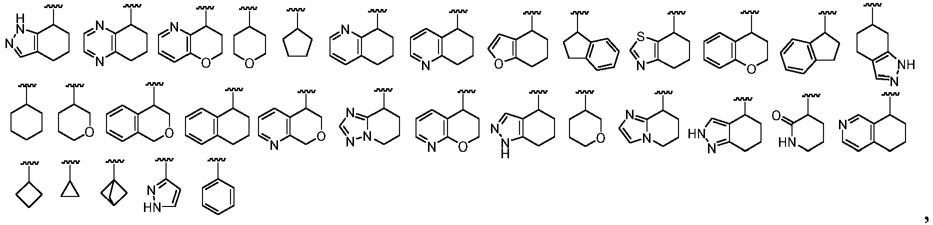 wherein, the above groups can be furtherly substituted with 0-3 substituents selected from the group consisting of: deuterated, halogen, C₁-C₆ alkyl, hydroxy(C₁-C₆ alkyl), -OR^{a}, -CN, NR^{a}R^{b}, C₁-C₆ haloalkyl.

In a preferred embodiment of formula I or II, wherein R' represents any one of the following groups:

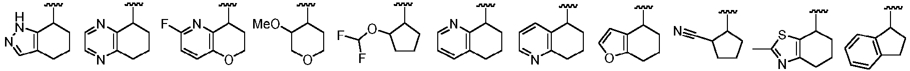

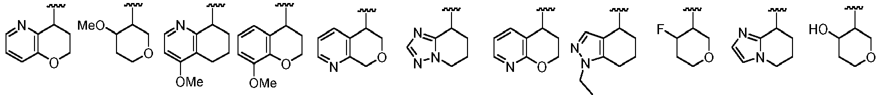

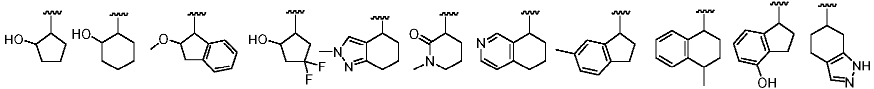

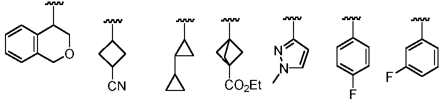

Specifically, the present invention provides the following compounds:

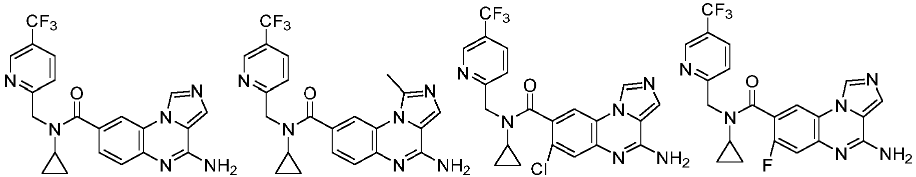

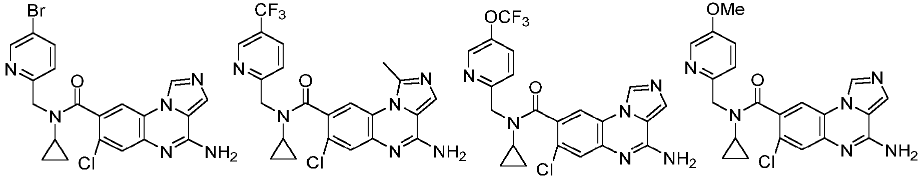

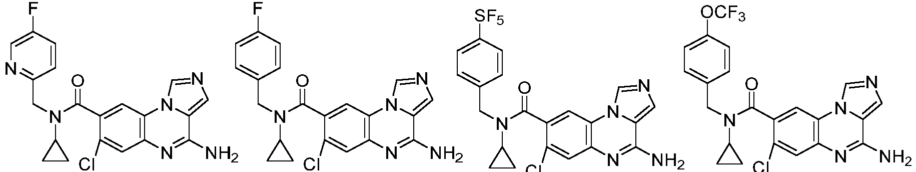

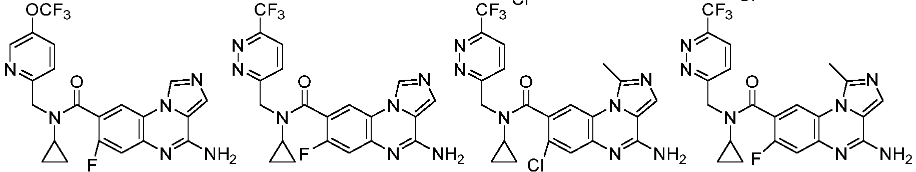

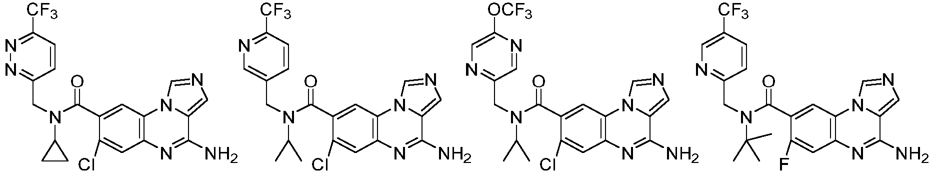

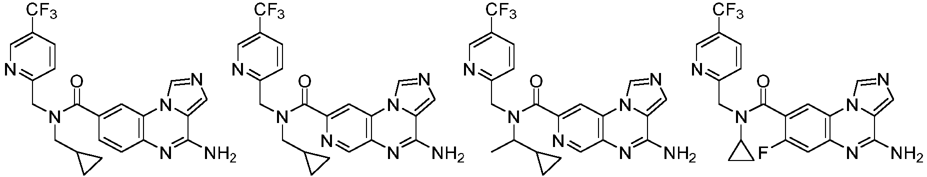

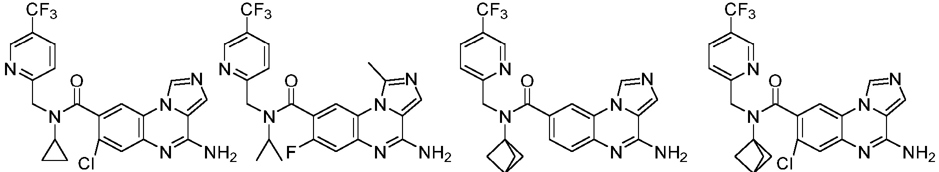

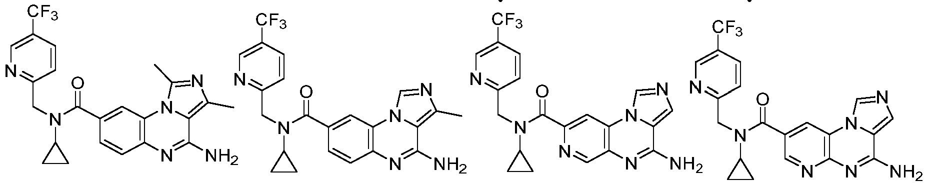

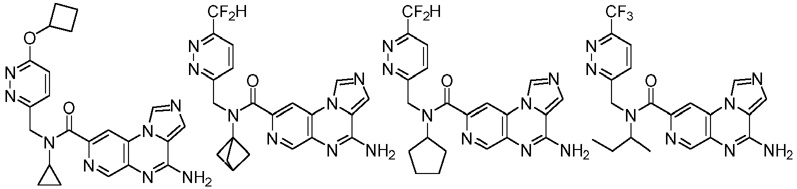

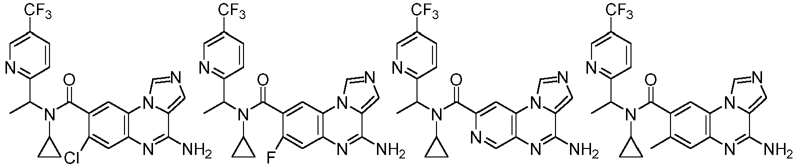

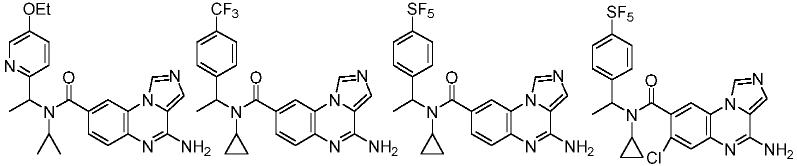

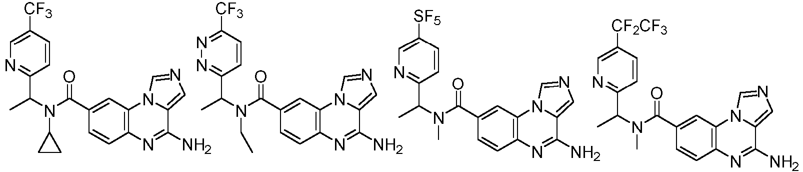

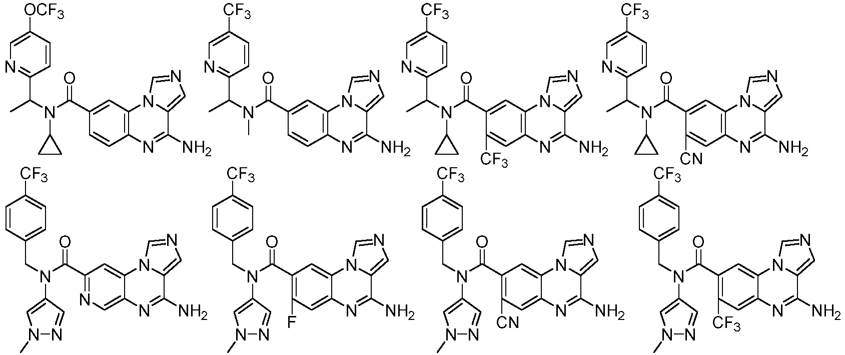

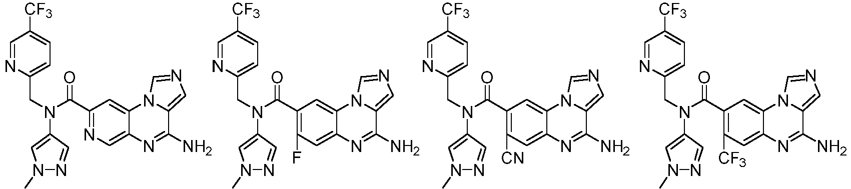

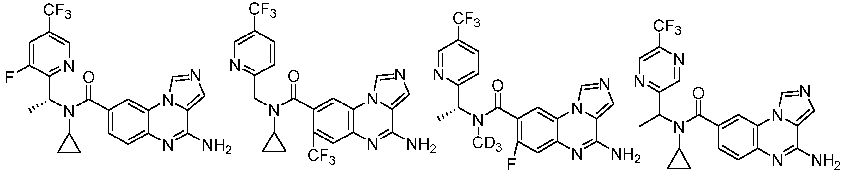

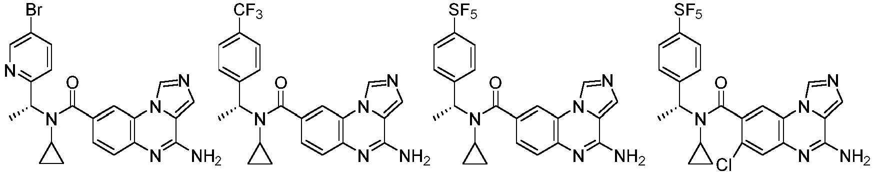

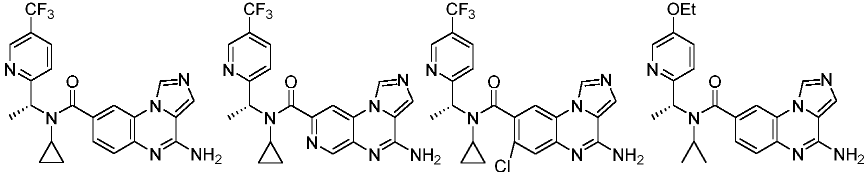

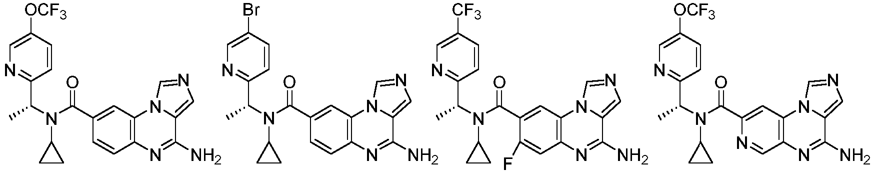

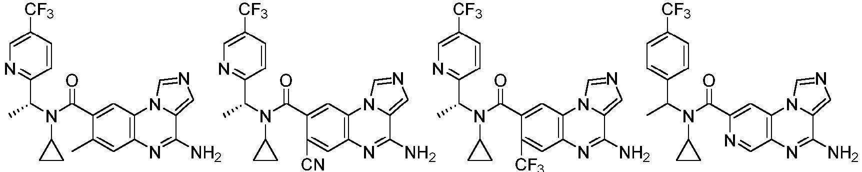

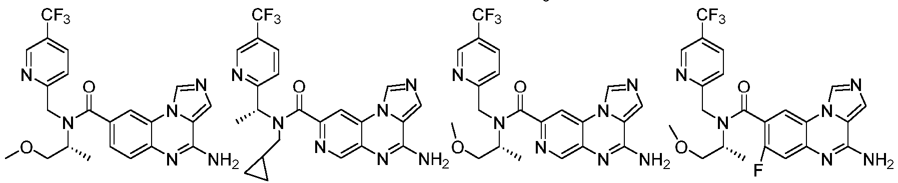

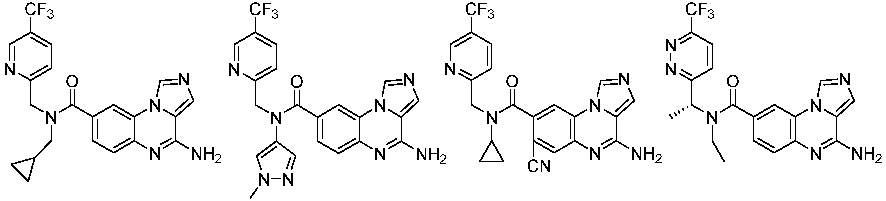

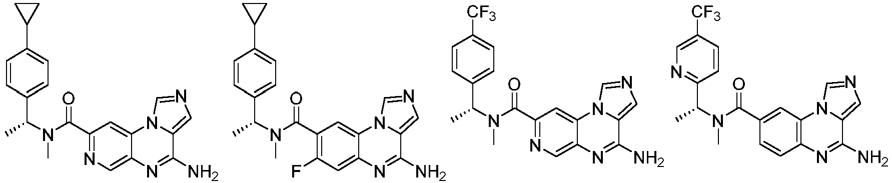

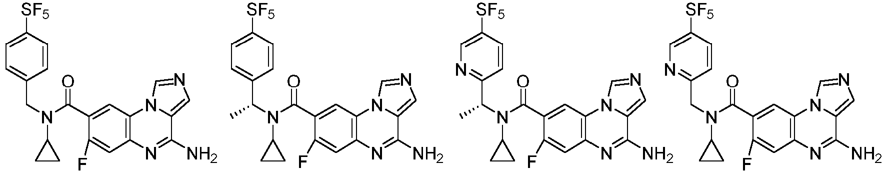

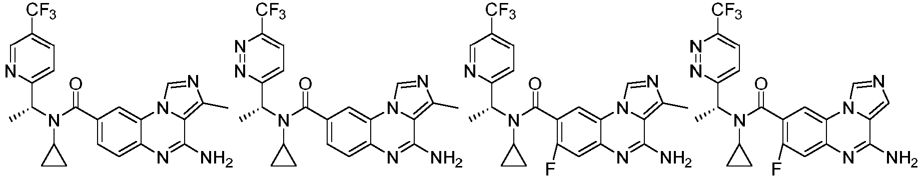

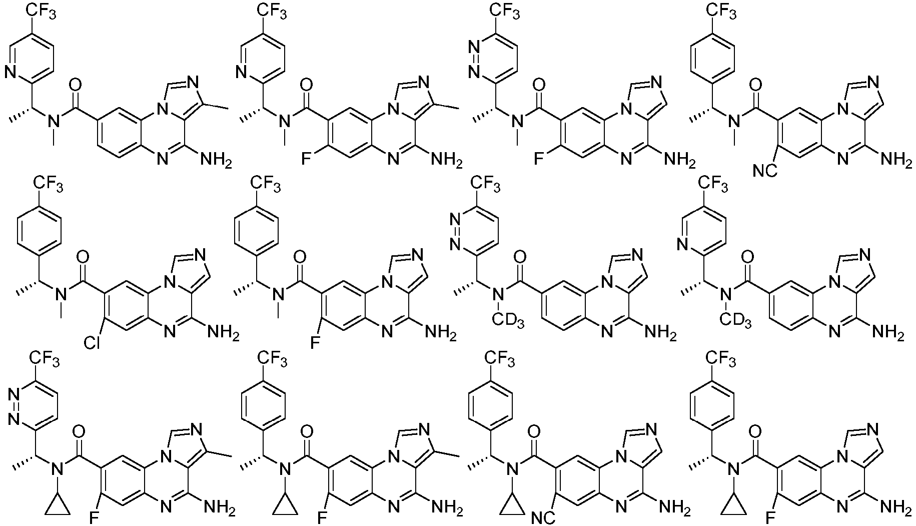

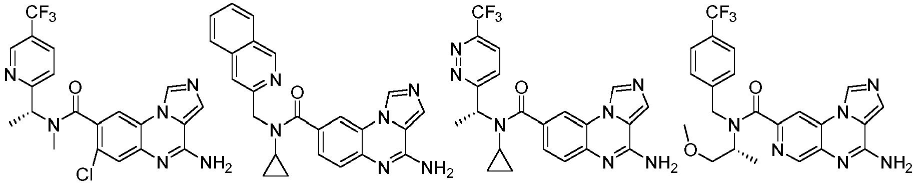

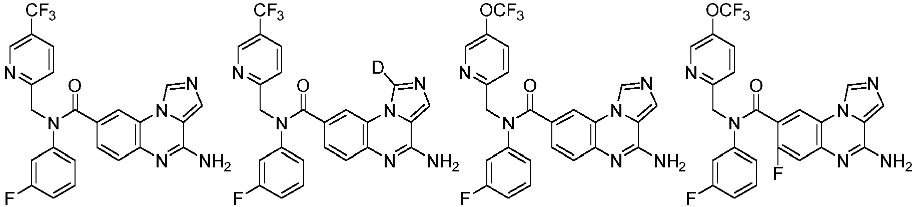

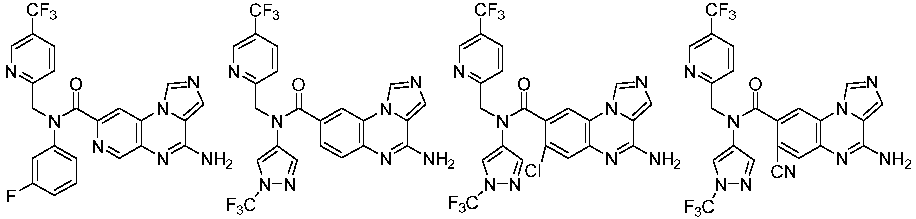

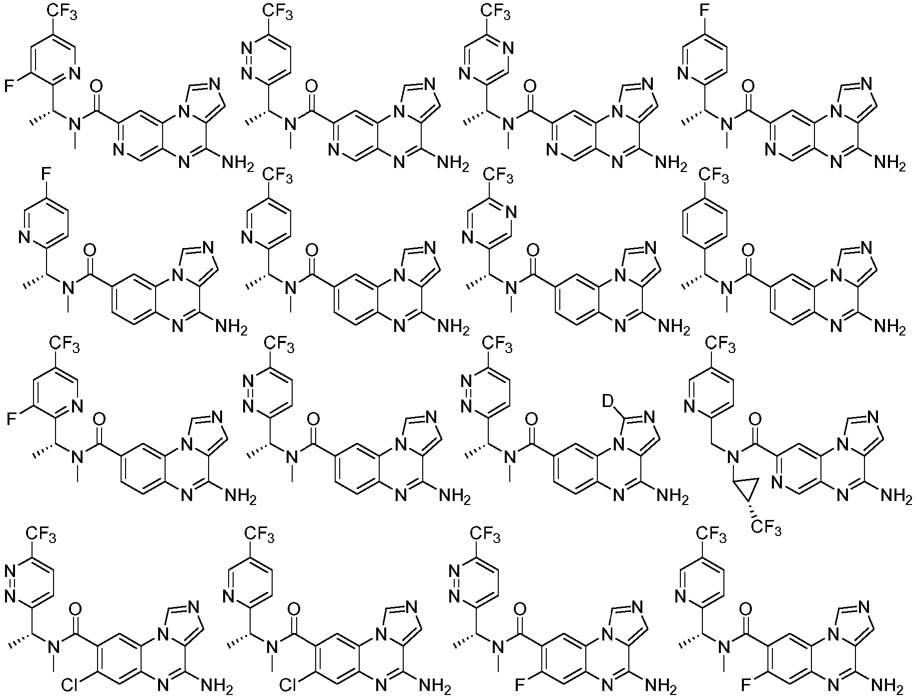

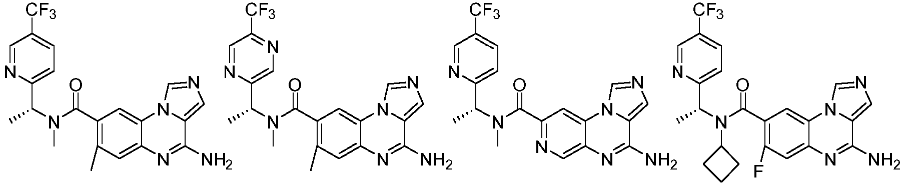

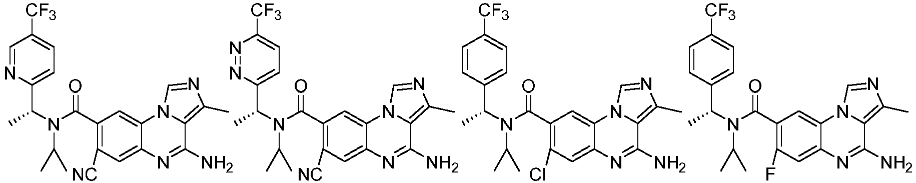

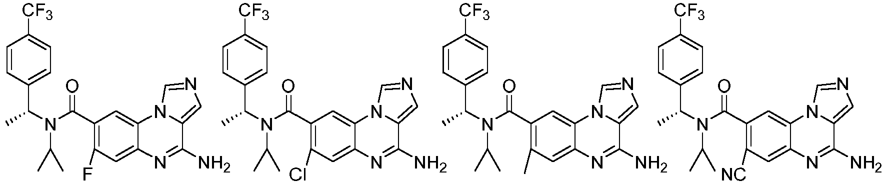

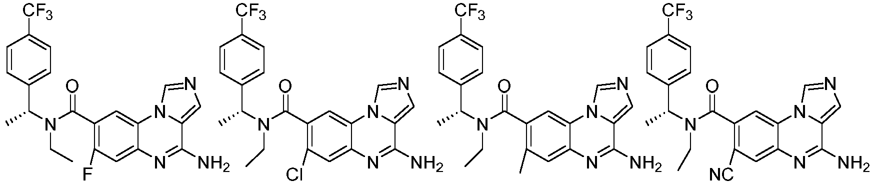

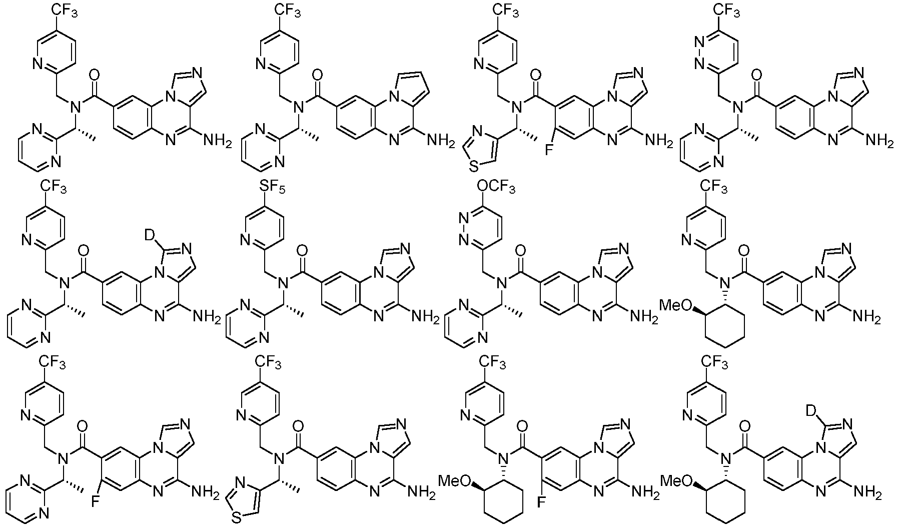

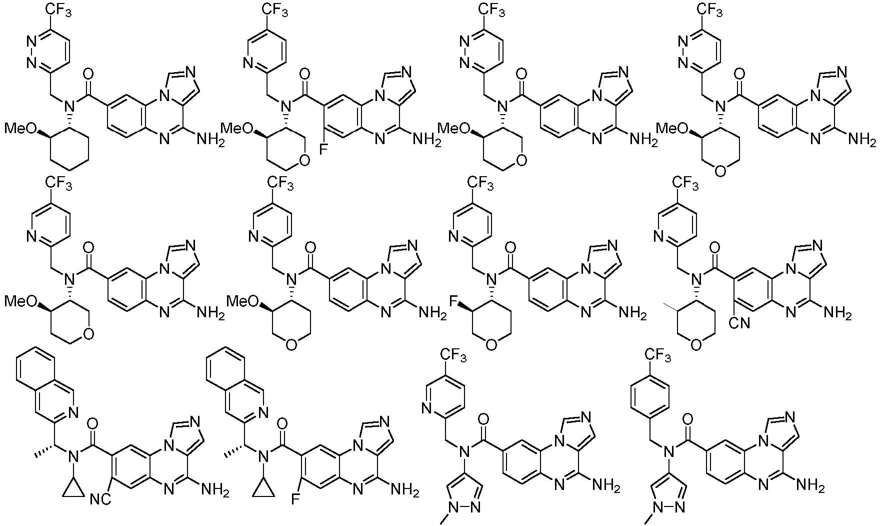

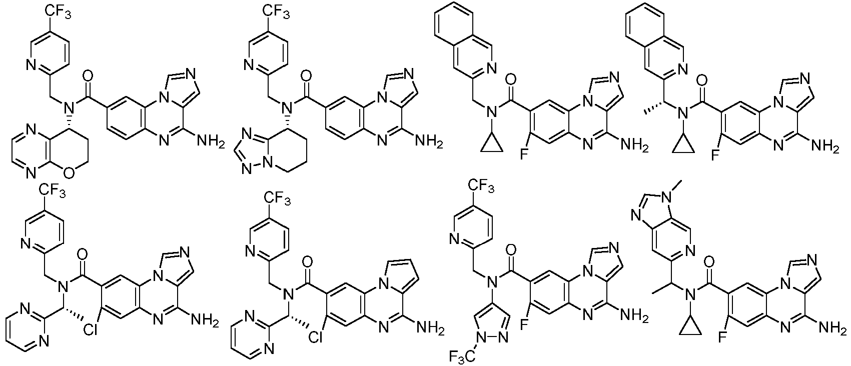

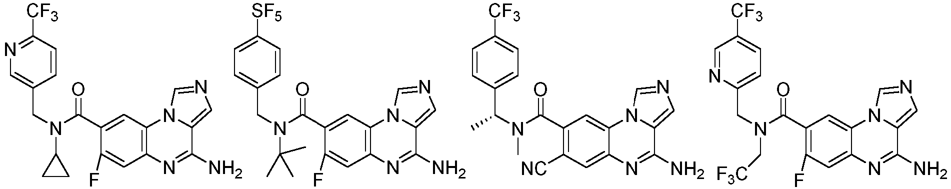

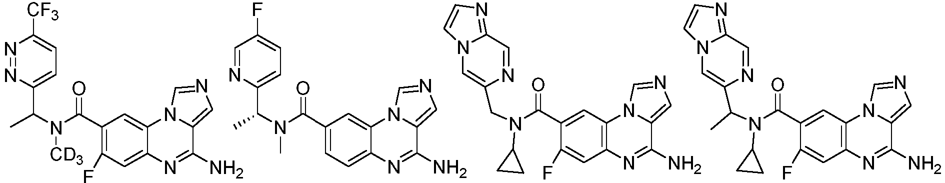

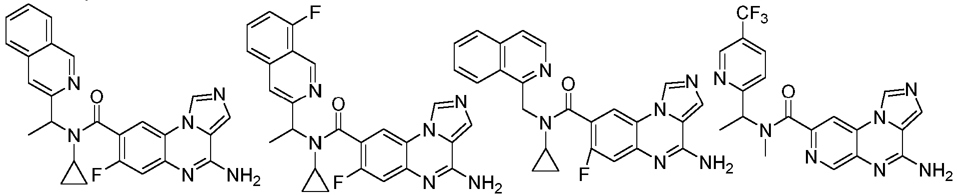

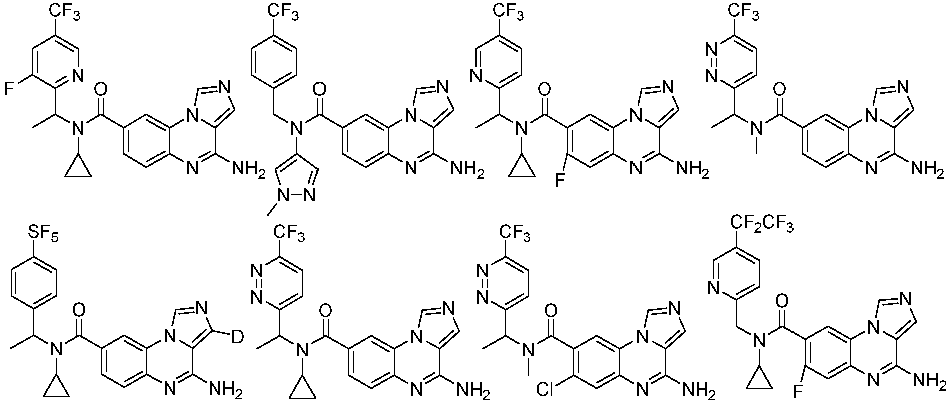

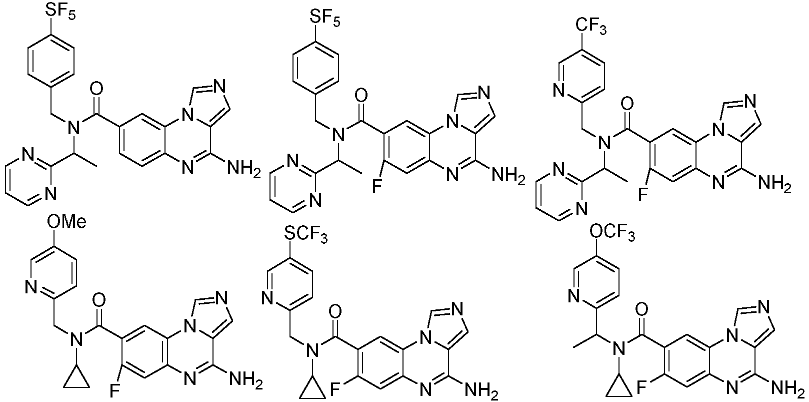

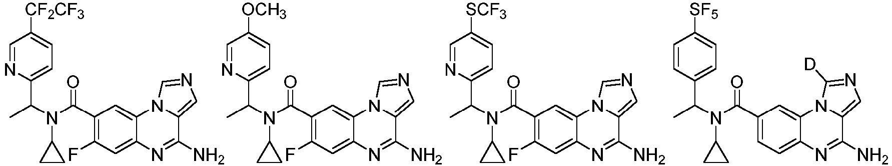

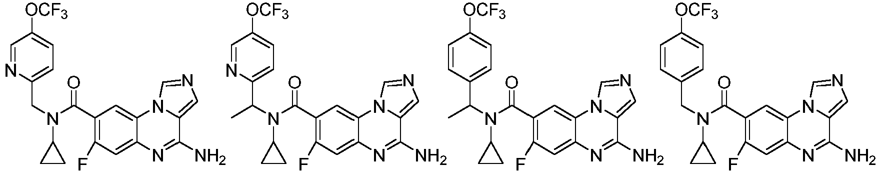

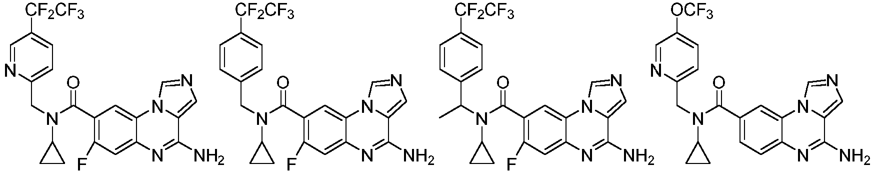

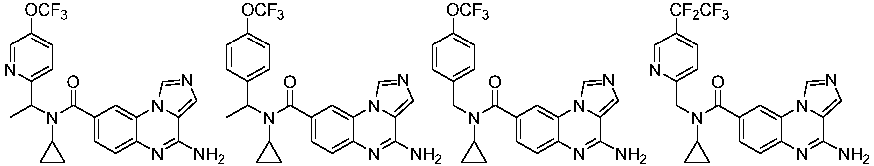

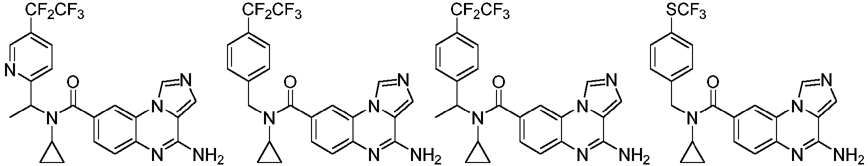

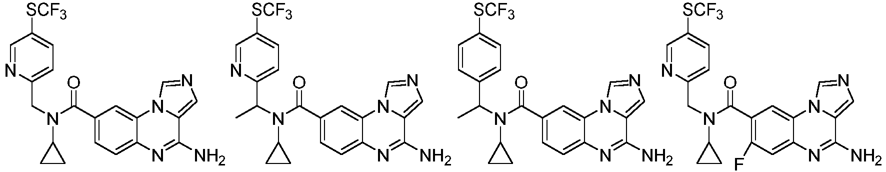

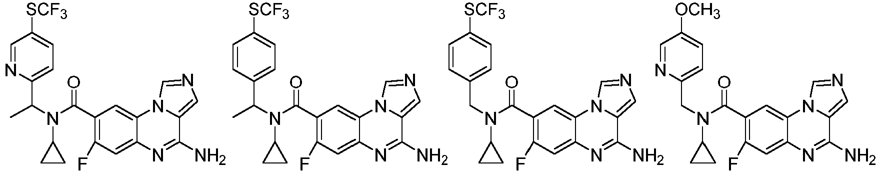

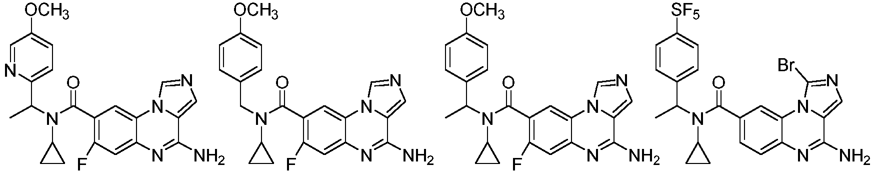

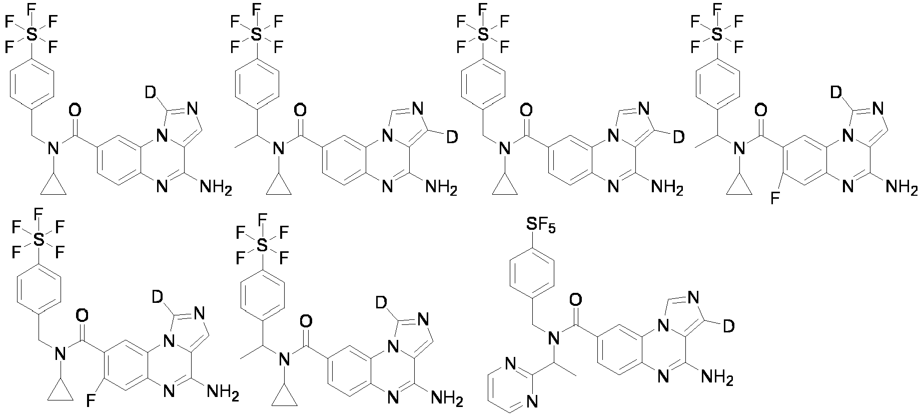

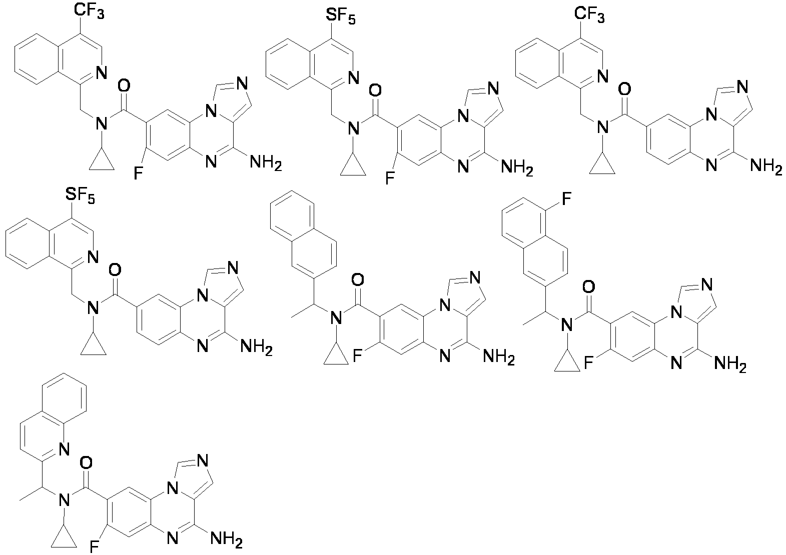

In the above preferred embodiments of the present invention, at least one or more hydrogen atoms in said isotopic derivative are deuterium atoms.

In additional embodiment, the present invention provides a pharmaceutical composition, comprising any one of the compounds mentioned above in the present invention, the pharmaceutically acceptable salt, the ester, the prodrug, the stereoisomer or the isotopic derivative and a pharmaceutically acceptable carriers.

Unless otherwise specified, the compounds of the present invention may encompass, in addition to their specific structures, pharmaceutically acceptable salts, stereoisomers, isotope isomers (e.g., deuterates), solvates, hydrates, prodrugs and metabolites thereof. Thus, the pharmaceutically acceptable salts, stereoisomers, isotope isomers, solvates, hydrates, prodrugs, and metabolites of these compounds are also considered within the scope of protection.

Preferably, the pharmaceutical composition of the present invention may further comprise a second active substance which is an anti-tumor drug including one or more of chemotherapeutic drugs, targeted tumor therapy drugs and tumor therapy antibody drugs.

In another embodiment, the present invention also provides a method for treating diseases by inhibiting the action of PRMT5 with a compound and pharmaceutically acceptable salts, esters, prodrugs, stereoisomers or isotopic derivatives thereof. Preferably, said diseases are tumors.

### Definition:

Unless otherwise specified, the term "alkyl" itself or as part of another substituent refers to linear (i.e. unbranched) or branched, or cyclic hydrocarbyl, or combinations thereof, which may be saturated, mono- or polyunsaturated and may include divalent or multivalent groups having a defined number of carbon atoms (i.e., C₁-C₁₀ refers to one to ten carbon atoms). Examples of saturated hydrocarbyl include, but are not limited to, groups such as methyl, ethyl, n-propyl, isopropyl, n-butyl, t-butyl, isobutyl, sec-butyl, cyclohexyl, cyclohexylmethyl, and cyclopropylmethyl, homologs and isomers such as n-pentyl, n-hexyl, n-heptyl, and n-octyl. An unsaturated alkyl has one or more double or triple bonds. Examples of unsaturated alkyl include, but are not limited to, vinyl, 2-propenyl, crotonyl, 2-isopentenyl, 2-(butadienyl), 2,4-pentadienyl, 3-(1,4-pentadienyl), ethynyl, 1- and 3-propynyl, 3-butynyl, as well as higher homologs and isomers. The alkyl defined as the hydrocarbyl is called "homoalkyl". The alkyl is optionally substituted with one or more halogen atoms.

The term "haloalkyl" refers to alkyl as defined above, wherein one or more hydrogen atoms are substituted with halogen atoms.

The term "alkylene" itself or as part of another substituent refers to a divalent group derived from alkyl, such as, but not limited to, -CH₂CH₂CH₂CH₂-, -CH₂CH=CHCH₂-, - CH₂C≡CCH₂- and -CH₂CH₂CH(CH₂CH₂CH₃)CH₂-. The alkyl (or the alkylene) usually has 1-24 carbon atoms, and groups having 10 or less carbon atoms are preferred in the present invention. "Low alkyl" or "low alkylene" refers to shorter chain alkyl or alkylene, usually having eight or fewer carbon atoms. The alkylene is optionally substituted with one or more halogen atoms.

The term "alkynyl" refers to a carbon chain containing at least one carbon-carbon triple bond, which may be linear or branched, or a combination thereof. Examples of the alkynyl include ethynyl, propargyl, 3-methyl-1-pentynyl, 2-heptynyl. The alkynyl is optionally substituted with one or more halogen atoms.

The term "cycloalkyl" refers to a monocyclic or bicyclic saturated carbon ring, each having 3-10 carbon atoms. "Fused analogue" of cycloalkyl means that a monocyclic ring is fused with aryl or heteroaryl, wherein the linking site is in the non-aromatic portion. Examples of the cycloalkyl and fused analogues thereof include cyclopropyl, cyclobutyl, cyclopentyl, cyclohexyl, tetrahydronaphthyl, decahydronaphthyl, dihydroindenyl. The cycloalkyl is optionally substituted with one or more halogen atoms. Further, the term "cycloalkyl" in present invention includes both bridged ring systems and spiro ring systems.

The term "alkoxy" refers to a straight-chain or branched alkoxy group having an indicated number of carbon atoms. C₁-C₆ alkoxy, for example, includes methoxyl, ethoxyl, propoxyl, isopropoxyl.

Unless otherwise specified, the term "heteroalkyl" itself or combination with another term refers to a stable straight-chain or branched, or cyclic hydrocarbon consisting of at least one carbon atom and at least one heteroatom selected from O, N, P, Si, S, or a combination thereof, wherein nitrogen atoms, phosphorus atoms or sulfur atoms may optionally be oxidized and the nitrogen atoms may optionally be quaternized. The heteroatoms O, N, P, S and Si may be placed at any position within the heteroalkyl or at positions where the alkyl is linked to the rest of the molecule. For example, the heteroatoms include, but are not limited to, -CH₂-CH₂-O-CH₃, -CH₂-CH₂-NH-CH₃, -CH₂-CH₂-N(CH₃)-CH₃, -CH₂-S-CH₂-CH₃, - CH₂-CH₂, -S(O)-CH₃, -CH₂-CH₂-S(O)₂-CH₃, -CH=CH-O-CH₃, -Si(CH₃)₃, -CH₂-CH=N-OCH₃, -CH=CH-N(CH₃)-CH₃, -O-CH₃, -O-CH₂-CH₃ and -CN. At most two or three heteroatoms may be contiguous. For example, -CH₂-NH-OCH₃ and -CH₂-O-Si(CH₃)₃. Similarly, the term "heteroalkylene" itself or combination with another term refers to a divalent group derived from heteroalkyl such as, but not limited to, -CH₂-CH₂-S-CH₂-CH₂- and -CH₂-S-CH₂-CH₂-NH-CH₂-. For heteroalkylene, the heteroatom may be at either or both ends of the chain (e.g., alkylene oxy, alkylene dioxy, alkylene amino, alkylene diamino). Furthermore, for alkylene and heteroalkylene linking groups, the direction in the molecular formula of the linking groups does not indicate the orientation of the linking groups. For example, the molecular formula -C(O)OR'- represents -C(O)OR'- and - R'OC(O)-. As mentioned above, the heteroalkyl as used herein includes those groups that are linked to the rest of the molecule via a heteroatom, for example -C(O)R', -C(O)NR', - NR'R", -OR', -SR' and/or -SO₂R'. Where reference is made to "heteroalkyl" followed by specific heteroalkyl such as -NR'R", it should be understood that the terms heteroalkyl and - NR'R" do not repeat and are not mutually exclusive. Instead, these specific heteroalkyl groups are referenced for greater clarity. Therefore, the term "heteroalkyl" should not be interpreted herein to exclude specific heteroalkyl such as -NR'R".

The term "cycloalkoxy" refers to cycloalkyl as defined above that is bonded to an oxygen atom, such as cyclopropoxy.

The term "haloalkoxy" refers to alkoxy as defined above in which one or more hydrogen atoms are substituted with halogen.

The term "aryl" refers to a monocycle or bicyclic aryl containing only carbon atoms. The "fused analogue" of aryl refers to the fusing of aryl with monocyclic cycloalkyl or monocyclic heterocyclyl in which the fusing point is located at the aryl. Examples of the aryl and fused ring analogues thereof include phenyl, naphthyl, indanyl, indenyl, tetrahydronaphthalene, 2,3-dihydrobenzofuryl, dihydrobenzopyranyl, 1,4-benzodioxalkyl.

The term "heteroaryl" refers to a monocyclic or bicyclic aryl containing at least one heteroatom selected from N, O and S. The "fused analogue" of heteroaryl refers to the fusing of heteroaryl with monocyclic cycloalkyl or monocyclic heterocyclyl in which the fusing point is located at the aryl. Examples of the heteroaryl include pyrrolyl, isozolyl, isothiazolyl, pyrazolyl, pyridinyl, oxazolyl, oxadiazolyl, thiadiazolyl, thiazolyl, imidazolyl, triazolyl, tetrazolyl, furyl, triazinyl, thienyl, pyrimidinyl, pyridazinyl, pyrazinyl, benzoxazolyl, benzothiazolyl, benzimidazolyl, benzofuranyl, benzothiopheneyl, furano(2,3-b)pyridinyl, quinolinyl, indolyl, isoquinolinyl.

"Substituted or unsubstituted": the defined alkyl, aryl and heteroaryl are unsubstituted or substituted with at least one substituent selected from the group consisting of substituents. The substituents are selected from the group consisting of: halogen atoms, alkyl having 1 to 6 carbon atoms, alkoxy having 1 to 6 carbon atoms, haloalkyl having 1 to 6 carbon atoms, haloalkoxy having 1 to 6 carbon atoms, -CN, alkynyl having 2 to 6 carbon atoms, alkanoyl having 1 to 6 carbon atoms, cycloalkyl having 3 to 7 ring atoms, heteroaryl, aryl, aralkyloxy having 7 to 10 carbon atoms, arylcarbonyl, aminocarbonyl, alkenyl having 2 to 5 carbon atoms, alkylthio having 1 to 6 carbon atoms, aminosulfinyl, aminosulphonyl, hydroxy, -SF₅, hydroxyalkyl having 1 to 4 carbon atoms, nitro, amino, carboxyl, alkoxycarbonyl having 2 to 5 carbon atoms, alkoxyalkyl having 1 to 4 carbon atoms, alkylsulfonyl having 1 to 4 carbon atoms, alkanoylamino having 1 to 4 carbon atoms, an alkanoyl (alkyl)amino having 1 to 6 carbon atoms, an alkanoylamidoalkyl group having 1 to 6 carbon atoms in each of the alkanoyl and alkyl moieties, an alkanoyl (alkyl)aminoalkyl having 1 to 6 carbon atoms in each of the alkanoyl and alkyl portions, alkanoyl sulfonamide having 1 to 4 carbon atoms, monoalkylaminocarbonyl or dialkylaminocarbonyl having 1 to 6 carbon atoms, monoalkylaminosulfinyl or dialkylaminesulfinyl having 1 to 6 carbon atoms, monoalkylaminosulfonyl or dialkylaminosulfonyl having 1 to 6 carbon atoms, aminealkyl having 1 to 4 carbon atoms, a monoalkylamino or dialkylaminogroup having 1 to 6 carbon atoms, a monoalkylaminoalkyl or dialkylaminoalkyl having 1 to 6 carbon atoms in each of the alkyl moieties, an aralkyl group having 7 to 10 carbon atoms, a heteroaralkyl group having 1 to 4 carbon atoms in the alkyl portion, heteroarylalkoxy having from 1 to 4 carbon atoms in the alkoxy portion and alkylsulfonamido having from 1 to 4 carbon atoms.

As used herein, the term "heterocycle"or "heterocyclic" or "heterocycloalkyl" or "heterocyclyl" refers to a saturated, partially saturated or unsaturated group (but not aromatic) having a monocyclic or condensed ring (including bridged and spiro systems in which there are 1 to 10 carbon atoms and 1 to 4 heteroatoms selected from nitrogen, sulfur, or oxygen, where one or more of the rings may be cycloalkyl, aryl, or heteroaryl, as long as the linking point passes through a nonaromatic ring. In an example, nitrogen atoms and/or sulfur atoms of the heterocyclic group are optionally oxidized to provide N-oxide, sulfinyl and sulfonyl portions. Examples of the "heterocyclyl" and fused analogues include pyrrolidinyl, piperidinyl, piperazinyl, imidazolidinyl, 2,3-dihydrofuran(2,3-b)pyridyl, benzoxazinyl, tetrahydroquinolinyl, tetrahydroisoquinolinyl, dihydroindolyl. The term also includes non-aromatic, partially unsaturated monocyclic rings such as 2- or 4-pyridones linked via a nitrogen atom or N-substituted -(1H,3H)-pyrimidine-2,4-diones (N-substituted uracils).

As used herein, the term "substituted heterocyclic" or "substituted heterocycloalkyl" or "substituted heterocyclyl" refers to a heterocyclic group substituted with 1 to 5 (e.g., 1 to 3) substituents that are identical to those defined by substituted cycloalkyl.

Unless otherwise specified, the term "halogenated" or "halo" or "halogen" itself or as part of another substituent refers to a fluorine, chlorine, bromine or iodine atom. In addition, the term "haloalkyl" refers to including monohaloalkyl and polyhaloalkyl. For example, the term "C₁-C₆ haloalkyl" refers to including, but not limited to, trifluoromethyl, 2,2,2-trifluoroethyl, 4-chlorobutyl, 3-bromopropyl.

"Prodrugs" refer to substances that are converted into the parent drug in vivo. In some cases, the prodrugs are often used because they are easier administered than the parent drugs. For example, the prodrugs may be bioavailable by oral administration while the parent drugs are not. The prodrugs may also have a higher solubility than the parent drugs in the pharmaceutical composition. An example of the prodrug, but not limited to, may be that any one of the compounds of Formula I is administered in the form of an ester (prodrug) to facilitate transport across cell membranes where water solubility is detrimental to migration and which subsequently metabolically hydrolyzes into the active substance (carboxylic acid) once within cells where water solubility is beneficial. Another example of the prodrug may be a short peptide (polyamino acid) that bonds to an acid group, where the peptide is metabolized to release the active portion.

### Optical isomers-diastereomers-geometrical isomers-tautomers:

The compounds of formula (I) contain asymmetric centers and are therefore used as racemates and racemic mixtures, single enantiomers, mixtures of diastereomers and single diastereomers. The present invention should comprise all these isomeric forms of the compounds of formula (I).

Some of the compounds described herein contain an olefinic (olefinic) double bond, which refers to including both E and Z geometrical isomers unless otherwise specified.

Some compounds of the present invention may comprise one or multiple ring systems, and thus cis- and trans-isomers may be present. The present invention aims to contain all of these cis- and trans-isomers.

Some of the compounds described herein may have different sites linked to a hydrogen atom, referred to as tautomers. Such examples may be ketones and enol forms thereof known as keto-enol tautomers. Individual tautomers as well as mixtures thereof are included in the compounds of the present invention.

The compounds of the present invention may be isolated into diastereoisomeric pairs of enantiomers, for example by fractional crystallization from a suitable solvent such as methanol or ethyl acetate or mixtures thereof. A pair of such enantiomers obtained can be separated into individual stereoisomers by conventional methods, for example using optically active amines or acids as resolution reagents or in a chiral HPLC column.

Alternatively, any enantiomers of the compounds of the present invention may be obtained by stereospecific synthesis using optically pure starting materials or reagents of known configuration.

Stable isotope-labeled analogues: one or more protons in the compounds of the present invention may be replaced with deuterium atoms, thereby providing deuterated analogues having improved pharmacological activity.

### Salts and dosage forms

It should be understood that, as used herein, reference to compounds of the present invention also includes pharmaceutically acceptable salts.

### Application

The compounds of the present invention can be used for treating PRMT5-associated diseases.

The compounds of the present invention can be prepared by the following reaction formula: Method A:

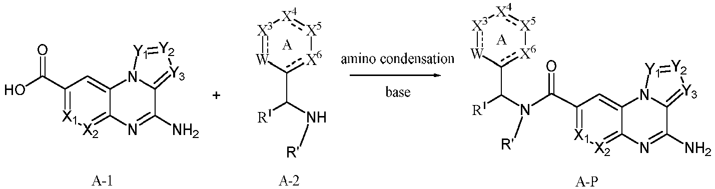

### Method A-SFC

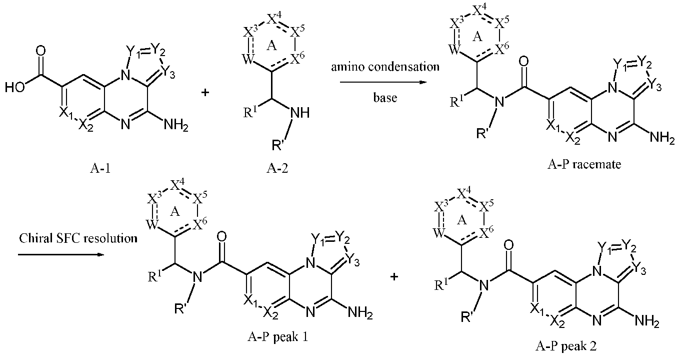

### Method B:

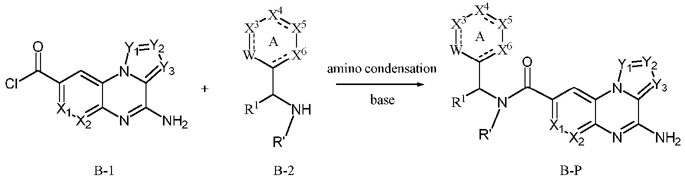

### Method B-SFC

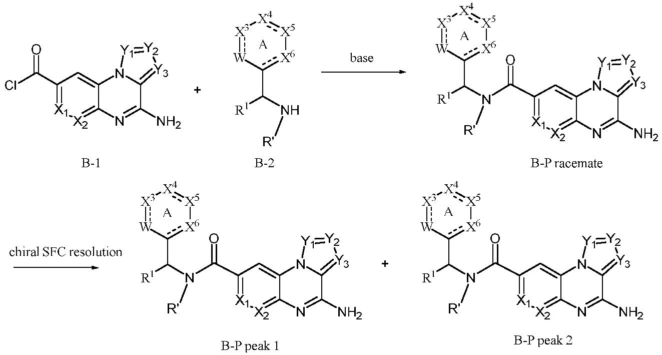

### Method C:

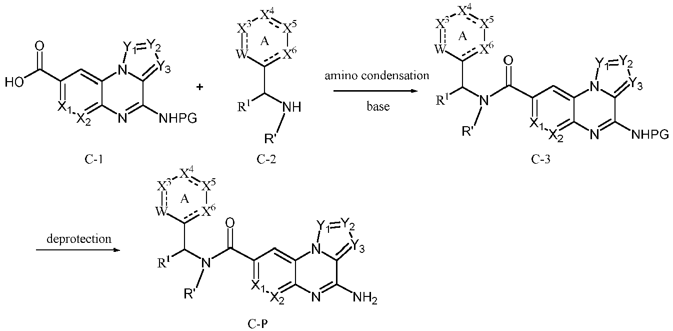

### Method C-SFC:

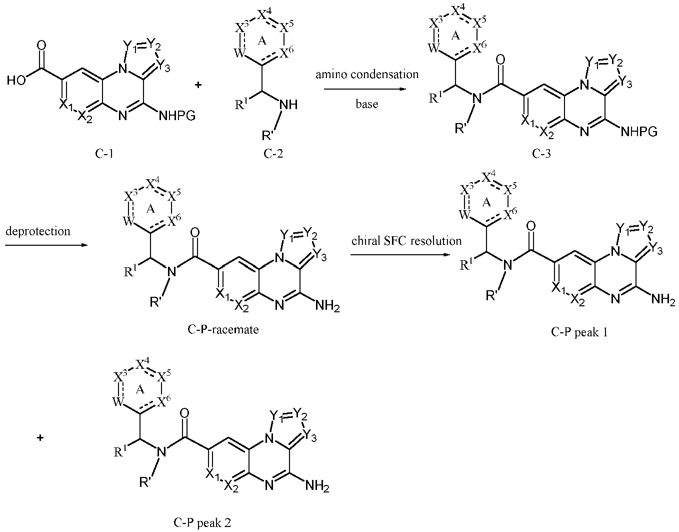

wherein R¹, R', ring A, W, X₁, X₃, X₃, X₄, X₅, X₆, Y₁, Y₂ and Y₃ are as defined in claim 1, and PG is a protecting group.

Method A: Compounds A-P can be obtained by the amino acid condensation reaction of carboxylic acid A-1 and amine A-2. The condensing agent may be HATU or PyBrOP, the base may be DIPEA or TEA, and the solvent may be DMF or DMAc. If the amine used is a racemate, it will be resolved by chiral SFC and the stereochemistry of the obtained isomer will be assigned arbitrarily to R or S.

Method B: Compound B-P may be obtained by the reaction of acid chloride B-1 and amine B-2. The alkali used may be Et3N, DIPEA or pyridine, the solvent used may be THF or 1,4-dioxane or DCM or DCE, and some catalysts such as DMPK may also be added to promote the reaction. Similarly, if the amine used is a racemate, then the product will be resolved by chiral SFC and the stereochemistry of the obtained isomer will be assigned arbitrarily to R or S.

Method C: Compound C-P may be obtained by an amine acid condensation reaction of carboxylic acid C-1 and amine C-2 and a one-step deprotection reaction, wherein the condensing agent used in the condensation reaction may be HATU or PyBrOP, the alkali used may be DIPEA or TEA, and the solvent used may be DMF or DMAc; The selected protecting group is PMB or Boc, the reagent for removing PMB protecting group used is TFA or 5% methanesulfonic acid+TFA, and the reagent for removing Boc protecting group used is TFA or HCl/EA, HCl/1,4-dioxane or TMSOTf+2,6-lutidine. Similarly, if the amine used is a racemate, then the product will be resolved by chiral SFC and the stereochemistry of the obtained isomer will be assigned arbitrarily to R or S.

### Analytical HPLC

Equipment: Agilent 1260; column specification: Agilent Poroshell HPH-C18 (3.0×50 mm, 2.7 µm); binary solvent system, mobile phase A: water (0.1% v/v ammonium bicarbonate), mobile phase B: acetonitrile; flow rate: 1 mL/min; gradient: from 10% B to 90% B; duration: 12 minutes; detector: DAD detector; wavelength: 254/220 nm;

### Preparation of HPLC-MS

HPLC equipment: Waters 2489; column specification: Ultimate µ XB-C18 (130A, 5 µm, 30 mm×150 mm); binary solvent system, mobile phase A: water (0.1% v/v ammonium bicarbonate), mobile phase B: acetonitrile; flow rate: 60-100 mL/min; gradient: from 10 % B to 90 % B; detector: DAD detector; wavelength: 254/220 nm;
Mass spectrometer: Agilent G6125B.

The compound of the present invention may be prepared by chemical synthesis, examples of which are shown below. It will be understood that the sequence of steps in the process described may vary, those specifically mentioned reagents, solvents and reaction conditions may be substituted, and readily reactable sites may be protected and deprotected if necessary.

The following abbreviations have the meanings shown below. ACN refers to acetonitrile; EA refers to ethyl acetate; CDI refers to N, N'-carbonyldiimidazole; DBU refers to 1,8-diazabicyclo[5.4.0]undec-7-ene; DIBAL-H represents diisobutylaluminium hydride; DIEA refers to diisopropylethylamine; DMAP refers to N,N-dimethylaminopyridine; DME refers to 1,2-dimethoxyethane; DMF refers to N,N-dimethylformamide; DMA and DMAc refers to N,N-dimethylformamide; DMPE refers to 1,2-bis(dimethylphosphino)ethane; DMSO means dimethylsulfoxide; DPPB means 1,4-bis(diphenylphosphino)butane; DPPE refers to 1,2-bis(diphenylphosphino)ethane; DPPF means 1,1'-bis(diphenylphosphino)ferrocene; DPPM refers to 1,1'-bis(diphenylphosphino)methane; DIAD means diisopropyl azodicarboxylate; EDCI represents 1-(3-dimethylaminopropyl)-3-ethylcarbodiimide; HATU represents 2-(7-aza-1H-benzotriazol-1-yl)-1,1,3,3-tetramethylurea hexafluorophosphate; HMPA refers to hexamethylphosphoramide; IPA refers to isopropyl alcohol; LDA refers to lithium diisopropylamide; LHMDS refers to lithium di(trimethylsilyl)amide; LAH represents lithium aluminium hydride; NCS refers to N-chlorosuccinimide; NaHMDS refers to sodium di(trimethylsilyl)amide; PyBOP refers to benzotriazol-1-yl-oxytripyrrolidinophosphobenzotriazole hexafluorophosphate; PyBrOP refers to bromo-tris-pyrrolidino-phosphonium hexafluorophosphate; TDA-I refers to tris(2-(2-methoxyethoxy)ethyl)amine; DCM refers to dichloromethane; TEA refers to triethylamine and TFA means trifluoroacetic acid; THF refers to tetrahydrofuran; NCS refers to N-chlorosuccinimide; NMM means N-methylmorpholine; NMP refers to N-methylpyrrolidone; PPh3 refers to triphenylphosphine; r.t. refers to room temperature; PMB means p-methoxybenzyl; Tosmic means p-toluenesulfonylmethyl isocyanide; (Boc)₂O refers to di-tert-butyl dicarbonate; PE refers to petroleum ether; o/n refers to overnight reaction.

The following preparations and examples illustrate the present invention, but do not limit the present invention in any way.

The features and advantages of the subject matter of the present invention will become more apparent from a detailed description of selected examples. As will be appreciated, the disclosed and claimed subject matter can be modified in various aspects, and all the modifications will not depart from the scope of the claims. Therefore, the description should be treated as illustrative in nature rather than restrictive. The entire scope of the subject matter of the present invention is set forth in the claims.

The present invention may be more easily understood with reference to the following examples, which are only used for illustrating the present invention instead of limiting the scope of the present invention.

### Intermediates

### Intermediate 1: 1-(pyrimidin-2-yl)-N-(5-(trifluoromethyl)pyridin-2-yl)methyl)ethyl-1-amine

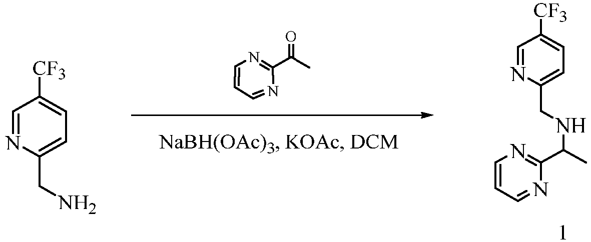

(5-(Trifluoromethyl)pyridin-2-yl)methanamine (5.0 g, 28 mmol) was dissolved in DCM (100 mL), 1-(pyrimidin-2-yl)ethyl-1-one (5.0 g, 42.0 mmol) and potassium acetate (3.3 g, 33.6 mmol) were added, and the mixture was stirred at room temperature for 1 h. Sodium triacetoxyborohydride (9.0 g, 42.0 mmol) was then added, and the mixture was continuously stirred for 5 h. Then the mixture was poured over ice water and extracted with DCM (100 mL × 2). The organic phases were combined and washed with a saturated brine solution (100 mL × 2), dried over anhydrous sodium sulfate, and filtered. The filtrate was concentrated under reduced pressure to give a crude product, which was purified by a flash chromatography machine (PE:EA=40:60) to give the target product, 1-(pyrimidin-2-yl)-N-(5-(trifluoromethyl)pyridin-2-yl)methyl)ethyl-1-amine (3.1 g, 10.9 mmol, yield: 39%) as a yellow oil.

LCMS (ESI) m/z: 283[M+H]⁺

### Intermediate 1A and Intermediate 1B: Synthesis of (S)-1-(pyrimidin-2-yl)-N-(5-(trifluoromethyl)pyridin-2-yl)methyl)ethyl-1-amine and (R)-1-(pyrimidin-2-yl)-N-(5-(trifluoromethyl)pyridin-2-yl)methyl)ethyl-1-amine

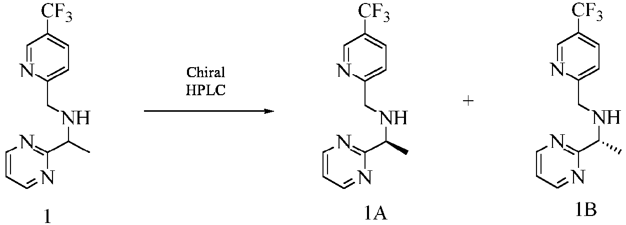

Racemate compound 1-(pyrimidin-2-yl)-*N*-(5-(trifluoromethyl)pyridin-2-yl)methyl)ethyl-1-amine (1 g, 3.5 mmol) was purified by Chiral HPLC (column: CHIRAL ART Cellulose-SB, 2 × 25 cm, 5 µm, mobile phase A: methyl t-butyl ether (0.1% diethylamine), mobile phase B: methanol, flow rate: 20 mL/min, elution gradient: 8% to 8% in 8 min for mobile phase B, detection wavelength: 220 nm), resulting in common intermediate 1A (S)-1-(pyrimidin-2-yl)-*N*-(5-(trifluoromethyl)pyridin-2-yl)methyl)ethyl-1-amine (450 mg, 1.6 mmol, 45%) and common intermediate 1B (*R*)-1-(pyrimidin-2-yl)-*N*-(5-(trifluoromethyl)pyridin-2-yl)methyl)ethyl-1-amine (500 mg, 1.7 mmol, 50%) as yellow oils.

LCMS (ESI) m/z: 283[M+H]⁺

### Intermediate 2: (1-methylpyrazol-4-yl) {[5-(trifluoromethyl)(2-pyridinyl)]methyl}amine

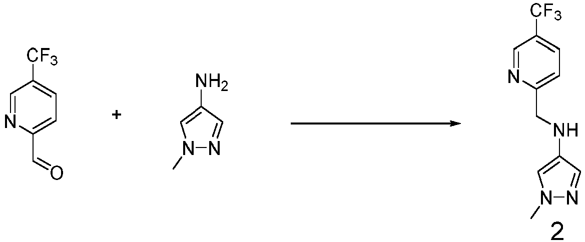

A mixture of 5-(trifluoromethyl)pyridin-2-carbaldehyde (5.00 g, 28.55 mmol), 1-methyl-1*H*-pyrazol-4-ylamine (3.33 g, 34.26 mmol), and DCM (50.00 mL) was stirred at room temperature overnight, and then sodium borohydride (1.62 g, 42.83 mmol) was added. The mixture was stirred at room temperature for 2 h, and then diluted with EA and washed continuously with a saturated sodium bicarbonate solution. The organic phases were collected, dried over anhydrous sodium sulfate and concentrated in vacuum to give (1-methylpyrazol-4-yl){[5-(trifluoromethyl)(2-pyridinyl)]methyl} amine (2.00 g, yield: 22%).

LCMS (ESI): 257 [M+H]⁺
The intermediate amines in the following table may be prepared using the synthesis steps described for **Intermediate 2** with replacement of the corresponding starting materials (aldehydes or amines) only:

| Intermediate No. | Chemical Structure | Chemical Name | m/z(ESI): (M+H)⁺ |
|---|---|---|---|
| 3 | 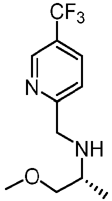 | (*R*)-1-methoxy-*N*-((5-(trifluoromethyl)pyridin-2-yl)methyl)propan-2-amine | 249.1 |
| 4 | 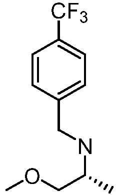 | (*R*)-2-methoxy-*N*-(4-(trifluoromethyl)phenyl)propane-2-amine | 248.2 |
| 5 | 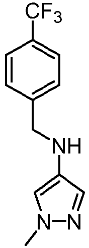 | 1-methyl-*N*-(4-(trifluoromethyl)benzyl)-1*H-*pyrazol-4-amine | 256.10 |

### Intermediate 6: N-(6-(trifluoromethyl)pyridazin-3-ylmethyl)cyclopropanamine

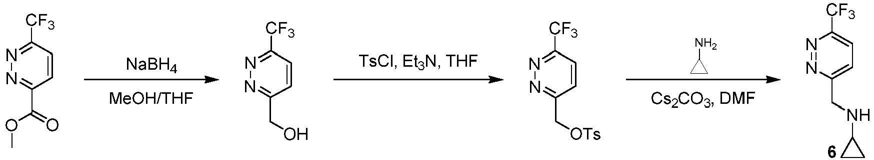

### Step I: 6-(trifluoromethyl)pyridazin-3-methanol

At room temperature, methyl 6-(trifluoromethyl)pyridazin-3-carboxylate (200 mg, 0.9703 mmol) was dissolved in a mixed solution of THF and methanol (6 mL, 2:1), and sodium borohydride (45 mg, 1.164 mmol) was added. The mixture was stirred at 25°C for 16 h, then poured over water (30 mL) and extracted with EA (50 mL × 2). The organic phases were combined, dried over anhydrous sodium sulfate and filtered. The filtrate was concentrated under reduced pressure to give a crude product, which was purified by column chromatography (0-10% EA/PE) to give 6-(trifluoromethyl)pyridazin-3-methanol (0.1 g, yield: 58%).

LCMS (ESI) m/z: 179.10 [M+H]⁺

### Step II: (6-(trifluoromethyl)pyridazin-3-yl)methyl 4-methylbenzenesulfonate

At room temperature, 6-(trifluoromethyl)pyridazin-3-methanol (400 mg, 2.246 mmol) and p-toluenesulfonyl chloride (655 mg, 3.369 mmol) were dissolved in THF (10 mL), and triethylamine (348 mg, 3.369 mmol) was added. The mixture was stirred at 25°C for 2 h. Upon completion of the reaction as indicated by LCMS, water (10 mL) was added and the mixture was extracted with EA (20 mL × 2). The organic phases were combined, dried over anhydrous sodium sulfate and filtered. The filtrate was concentrated under reduced pressure and purified by column chromatography (0-10% EA/PE) to give (6-(trifluoromethyl)pyridazin-3-yl)methyl 4-methylbenzenesulfonate (500 mg, yield: 67%).

LCMS (ESI) m/z: 333.1 [M+H]⁺

### Step III: N-(6-(trifluoromethyl)pyridazin-3-ylmethyl)cyclopropanamine

At room temperature, (6-(trifluoromethyl)pyridazin-3-yl)methyl 4-methylbenzenesulfonate (500 mg, 1.505 mmol) was dissolved in DMF (10 mL), and cyclopropanamine (438 mg, 7.523 mmol) and cesium carbonate (1.25 g, 3.762 mmol) were added. The mixture was stirred at room temperature for 2 h. Upon completion of the reaction as indicated by LCMS, water (10 mL) was added, and the mixture was extracted with EA (20 mL × 2). The organic phases were combined, dried over anhydrous sodium sulfate and filtered. The filtrate was concentrated under reduced pressure and purified by column chromatography (0-10% EA/PE) to give *N*-(6-(trifluoromethyl)pyridazin-3-ylmethyl)cyclopropanamine (120 mg, yield: 36.7%).

LCMS (ESI) m/z: 218.1 [M+H]⁺

The intermediate amines in the following table may be prepared using the synthesis steps described for Intermediate 6 with replacement of the corresponding starting materials only:

| Intermediate No. | Chemical Structure | Chemical Name | m/z(ESI): (M+H)⁺ |
|---|---|---|---|
| 7 | 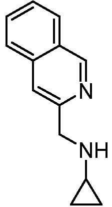 | *N*-(isoquinolin-3-ylmethyl)cyclopropanamine | 199.12 |
| 8 | 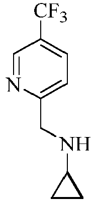 | *N*-((5-(trifluoromethyl)pyridin-2-yl)methyl)cyclopropanamine | 217.09 |
| 9 | 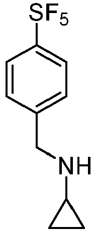 | *N*-((5-(trifluoromethyl)pyridin-2-yl)methyl)bicyclo[1.1.1]pentan-1-amine | 274.1 |
| 10 | 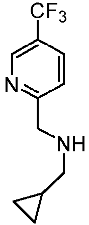 | 1-cyclopropyl-*N*-((5-(trifluoromethyl)pyridin-2-yl)methyl)methanamine | 231.1 |
| 11 | 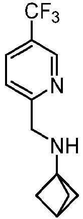 | *N*-((5-(trifluoromethyl)pyridin-2-yl)methyl)bicyclo[1.1.1]pentan-1-amine | 243.1 |
| 12 | 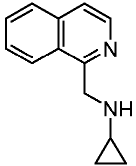 | *N*-(isoquinolin-1-ylmethyl)cyclopropanamine | 199.2 |
| 13 | 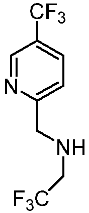 | 2,2,2-trifluoro-*N*-((5-(trifluoromethyl)pyridin-2-yl)methyl)ethan-1-amine | 259.1 |
| 14 | 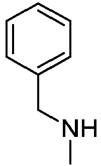 | *N*-methyl-1-benzylamine | 122.1 |
| 15 | 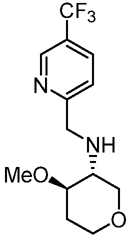 | (3*R*,4*R*)-4-methoxy-*N*-(5-trifluoromethyl)pyridin-2-ylmethyl)tetrahydropyran-3-amine | 291.2 |
| 1B | 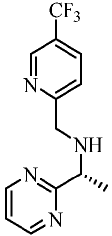 | (*R*)-1-(pyrimidin-2-yl)-*N*-(5-(trifluoromethyl)pyridin-2-yl)methyl)ethyl-1-amine | 283 |
| 64 | 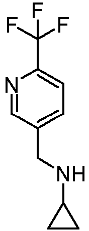 | *N*-(6-(trifluoromethyl)pyridin-3-ylmethyl)cyclopropanamine | 217.1 |
| 65 | 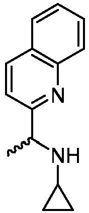 | *N*-(1-(quinolin-2-yl)ethyl)cyclopropanamine | 213.20 |
| 66 | 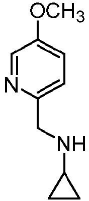 | *N*-((5-methoxypyridin-2-yl)methyl)cyclopropanamine | 179.2 |
| 67 | 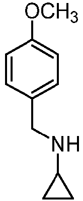 | *N*-(4-methoxybenzyl)cyclopropanamine | 178.1 |
| 68 | 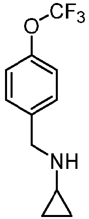 | *N*-(4-(trifluoromethoxy)benzyl)cyclopropanamine | 231.1 |

### Intermediate 16 N-methyl-1-(6-(trifluoromethyl) pyridazin-3-yl) ethan-1-amine

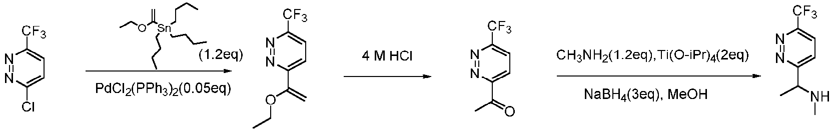

Step I: A reaction system of 3-chloro-6-(trifluoromethyl)pyridazine (1000 mg, 5.48 mmol), tributyl(1-ethoxyvinyl)stannane (2370 mg, 6.57 mmol), bis-triphenylphosphine palladium dichloride (192.27 mg, 0.27 mmol) and 15 mL of dioxane was stirred at 100°C for 8 h. Then the solvent was removed by rotary evaporation followed by extraction with EA (60 mL) for 3 times. The organic phases were combined, washed with a saturated brine solution and dried over anhydrous sodium sulfate. The combined organic phases were concentrated under reduced pressure to give 3-(1-ethoxyvinyl)-6-(trifluoromethyl)pyridazine (800 mg, 3.65 mmol, 66.93%) as a colorless oil.

LCMS (ESI): 219 [M+H]⁺

Step II: 3-(1-ethoxyvinyl)-6-(trifluoromethyl)pyridazine (800 mg, 3.67 mmol) was added into a 4 M dioxane hydrochloride solution (15 mL) and stirred for 2 h. Then the resulting mixture was concentrated under reduced pressure and purified by column chromatography (PE:EA=5:1) to give 1-(6-(trifluoromethyl)pyridazin-3-yl)ethan-1-one (400 mg, 2.09 mmol, yield: 57.38%) as a white solid.

LCMS (ESI): 191 [M+H]⁺

Step III: A solution of 1-(6-(trifluoromethyl)pyridazin-3-yl)ethan-1-one (400 mg, 2.1 mmol), methanamine (78 mg, 2.52 mmol), tetraisopropyl titanate (1200 mg, 4.21 mmol) and 1,2-dichloroethane (8 mL) was stirred at 80°C for 2 h. Upon complete conversion as indicated by LCMS, the temperature was lowered to room temperature, and a solution of sodium borohydride (239 mg, 6.31 mmol) in methanol (10 mL) was added dropwise under ice-bath stirring, followed by further stirring for 2 h. Then the solvent was removed by rotary evaporation followed by extraction with EA (60 mL) for 3 times. The organic phases were combined, washed with a saturated brine solution and dried over anhydrous sodium sulfate.

The combined organic phases were concentrated under reduced pressure and underwent column chromatography (PE:EA=1:2) to give N-methyl-1-(6-(trifluoromethyl)pyridazin-3-yl)ethan-1-amine (60 mg, 0.229 mmol, yield: 15.44%) as a white solid.

LCMS (ESI): 206 [M+H]⁺

The intermediate amines in the following table may be prepared using the synthesis steps described for Intermediate 15 with replacement of the corresponding starting materials only:

| Intermediat e No. | Chemical Structure | Chemical Name | m/z(ESI) : (M+H)⁺ |
|---|---|---|---|
| 17 | 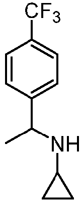 | *N-*(1-(4-(trifluoromethyl)phenyl)ethyl)cyclopropanamin e | 230.11 |
| 18 | 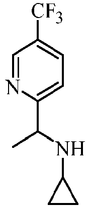 | cyclopropyl{[5-(trifluoromethyl)(2-pyridinyl)]ethyl}amine | 231 |
| 19 | 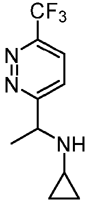 | cyclopropyl{[6-(trifluoromethyl)pyridazin-3-yl]ethyl}amine | 232.10 |
| 20 | 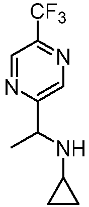 | *N*-(1-(5-trifluoromethyl)pyrazin-2-ethyl)ethyl)cyclopropanamine | 232.10 |
| 21 | 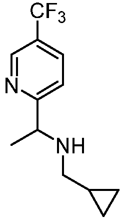 | *N*-(cyclopropylmethyl)-1-(5-(trifluoromethyl)pyridin-2-yl)ethan-1-amine | 245.12 |
| 22 | 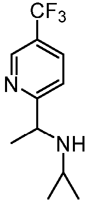 | *N*-(1-(5-trifluoromethyl)pyridin-2-ethyl)propan-2-amine | 233.2 |
| 23 | 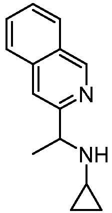 | *N*-(1-(isoquinolin-3-yl)ethyl)cyclopropanamine | 213.20 |
| 24 | 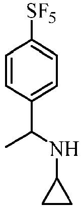 | *N*-(1-(4-(pentafluoro-*λ*⁶-sulfonamido)phenyl)ethyl)cyclopropanamine | 288.08 |
| 25 | 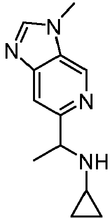 | *N*-(1-(3-methyl-3*H*-imidazo[4,5-*c*]pyridin-6-ethyl)ethyl)cyclopropanamine | 217.1 |
| 26 | 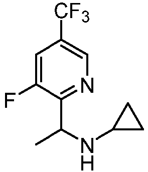 | *N*-(1-(3-fluoro-5-(trifluoromethyl)pyridin-2-yl)ethyl)cyclopropanamine | 249.09 |
| 27 | 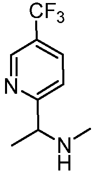 | *N*-methyl-1-(5-(trifluoromethyl)pyridin-2-yl)ethan-1-amine | 205.09 |
| 28 | 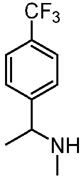 | *N*-methyl-1-(4-(trifluoromethyl)phenyl)ethan-1-amine | 204.09 |
| 69 | 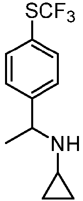 | cyclopropyl{[4-(trifluoromethylthio)phenyl]ethyl}amine | 261.2 |
| 70 | 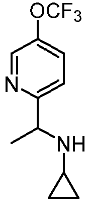 | *N*-(1-(5-(trifluoromethoxy)pyridin-2-yl)ethyl)cyclopropanamine | 247.1 |

### Intermediate 29 (R)-1-(4-cyclopropylphenyl)-N-methylethanol-1-amine

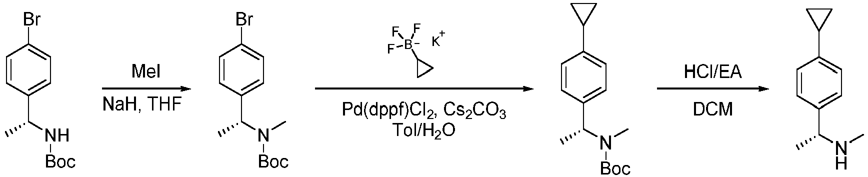

### Step I: Synthesis of t-butyl (R)-(1-(4-bromophenyl)ethyl)(methyl)carbamate

Under an atmosphere of N₂, NaH (2.66 g, 66.6 mmol, purity: 60%) was added into a THF solution of t-butyl (*R*)-(1-(4-bromophenyl)ethyl)carbamate **(**CAS: 578729-21-2**,** 10.0 g, 33.3 mmol). The reaction solution was stirred at room temperature for 30 min. Iodomethane (7.24 g, 50.0 mmol) was added into the reaction solution, which was then stirred at room temperature for 4 h. Then the reaction solution was poured over water (150 mL) and extracted with EA (100 mL × 3). The organic phases were combined, dried over anhydrous sodium sulfate and filtered. The filtrate was concentrated under reduced pressure and purified by silica gel column chromatography (PE:EA=10:1) to give t-butyl (R)-(1-(4-bromophenyl)ethyl)(methyl)carbamate (8.0 g, yield: 76%) as a yellow oily compound.

LCMS (ESI) m/z: 315.2 [M+H]⁺

### Step II: Synthesis of t-butyl (R)-(1-(4-cyclopropylphenyl)ethyl)(methyl)carbamate

Under an atmosphere of N₂, Pd(dppf)Cl₂ (325 mg, 0.477 mmol) was added into mixed solutions of t-butyl (*R*)-(1-(4-bromophenyl)ethyl)(methyl)carbamate (1.5 g, 4.77 mmol), potassium cyclopropanetrifluoroborate salt (1.77 g, 11.7 mmol) and cesium carbonate (4.76 g, 14.3 mmol) in toluene (30 mL) and water (5 mL). Then the reaction mixture was stirred at 100°C for 16 h, the system was poured over water (80 mL) and extracted with EA (50 mL × 3), with organic phases combined, dried over anhydrous sodium sulfate and filtered. The filtrate was concentrated under reduced pressure to give a crude product, which was purified by silica gel column chromatography (PE:EA=10:1) to give (*R*)-(1-(4-cyclopropylphenyl)ethyl)(methyl)carbamate (600 mg, yield: 46%) as a white solid.

LCMS (ESI) m/z: 276.3 [M+H]⁺

### Step III: Synthesis of (R)-1-(4-cyclopropylphenyl)-N-methylethanol-1-amine

At room temperature, hydrochloric acid/EA (2.7 mL, 4 M, 10.9 mmol) was added into a solution of t-butyl (*R*)-(1-(4-cyclopropylphenyl)ethyl)(methyl)carbamate (500 mg, 1.82 mmol) in DCM (10 mL). Then the reaction mixture was stirred at room temperature for 5 h, the solution was directly concentrated under reduced pressure to give a crude product, (*R*)-1-(4-cyclopropylphenyl)-*N*-methylethanol-1-amine hydrochloride (350 mg, crude product), which was directly used in the next step without purification.

LCMS (ESI) m/z: 176.20 [M+H]⁺

### Intermediate 30 (R)-N-methyl-1-(4-(trifluoromethyl)phenyl)ethan-1-amine

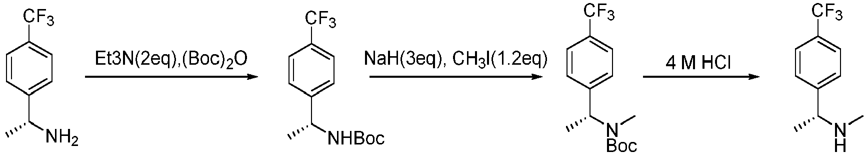

Step I: (*R*)-1-(4-(trifluoromethyl)phenyl)ethan-1-amine **(**CAS: 578027-35-7**,** 2000 mg, 10.57 mmol), triethylamine (4271 mg, 42.29 mmol), and Boc anhydride (2768 mg, 12.69 mmol) were dissolved in DCM (50 mL) and stirred at room temperature for 2 h. Then the extraction was performed with EA (30 mL × 3). The organic phases were combined, washed with a saturated brine solution and dried over anhydrous sodium sulfate. The combined organic phases were concentrated under reduced pressure to give (*R*)-(1-(4-(trifluoromethyl)phenyl)ethyl)t-butylcarbamate (2000 mg, yield: 58.85%) as a white solid. LCMS (ESI): 290 [M+H]⁺

Step II: (*R*)-(1-(4-(trifluoromethyl)phenyl)ethyl)t-butylcarbamate (2000 mg, 6.91 mmol) was dissolved in THF (15 mL), sodium hydrogen (497.71 mg, 20.74 mmol) was slowly added and stirred for 1 h, then iodomethane (1180 mg, 8.30 mmol) was added dropwise, followed by stirring at room temperature for 2 h. Then the extraction was performed with EA (30 mL × 3). The organic phases were combined, washed with a saturated brine solution and dried over anhydrous sodium sulfate. The combined organic phases were concentrated under reduced pressure to give (*R*)-methyl(1-(4-(trifluoromethyl)phenyl)ethyl)t-butylcarbamate (800 mg, yield: 38.09%) as a white solid.

LCMS (ESI): 304[M+H]⁺

Step III: (*R*)-methyl(1-(4-(trifluoromethyl)phenyl)ethyl)t-butylcarbamate (800 mg, 2.64 mmol) was added into a 4 M HCl solution in dioxane (20 mL) and stirred for 2 h. Then the resulting mixture was concentrated under reduced pressure to give (*R*)-*N*-methyl-1-(4-(trifluoromethyl)phenyl)ethan-1-amine (300 mg, yield: 55.98%) as a white solid.

LCMS (ESI): 204[M+H]⁺

The intermediate amines in the following table may be prepared using the synthesis steps described for **Intermediate 30** with replacement of the corresponding starting materials only:

| Intermediate No. | Chemical Structure | Chemical Name | m/z(ESI): (M+H)⁺ |
|---|---|---|---|
| 31 | 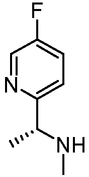 | (*R*)-1-(5-fluoropyridin-2-yl)-*N-*methylethyl-1-amine | 155.15 |
| 32 | 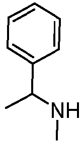 | *N*-methyl-1-phenyl-1-amine | 136.3 |
| 33 | 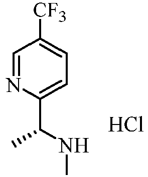 | (*R*)*-N-*methyl-1-(5-(trifluoromethyl)pyridin-2-yl)ethan-1-amine hydrochloride | 205.1 |
| 34 | 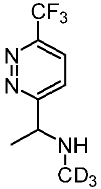 | *N*-(methyl-*d₃*)-1-(6-(trifluoromethyl)pyridazin-3-yl)ethan-1-amine | 209.1 |
| 35 | 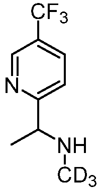 | *N*-methyl-*d₃*-1-(5-trifluoromethyl)pyridin-2-yl)ethan-1-amine | 208.2 |
| 36 | 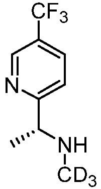 | (*R*)-*N*-(methyl-*d₃*)-1-(5-trifluoromethyl)pyridin-2-yl)ethan-1-amine | 208.2 |

The intermediate amines in the following table may be prepared using step III in the synthesis of Intermediate **15** with replacement of the corresponding starting materials only:

| Intermediate No. | Chemical Structure | Chemical Name | m/z(ESI): (M+H)⁺ |
|---|---|---|---|
| 71 | 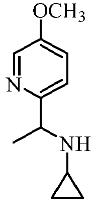 | *N*-(1-(5-methoxypyridin-2-yl)ethyl)cyclopropanamine | 192.0 |
| 72 | 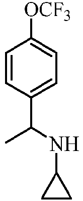 | *N-*(1-(4-(trifluoromethoxy)phenyl)ethyl)cyclopropanamine | 246.2 |
| 73 | 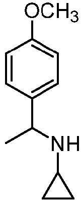 | *N*-(1-(4-methoxyphenyl)ethyl)cyclopropanamine | 191.2 |

### Intermediate 37 methyl 4-((4-methoxybenzyl)amino)imidazo[1,5-a]quinoxaline-8-carboxylate

### Method I:

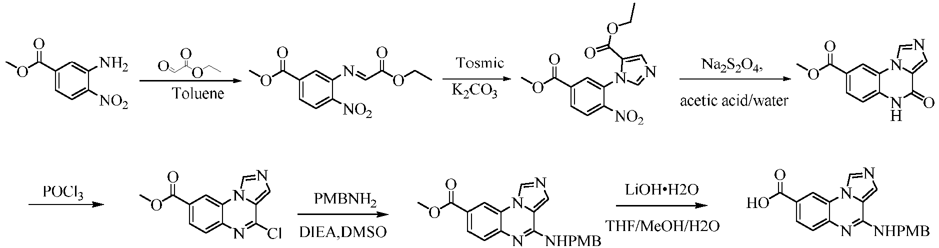

### Step I: Synthesis of methyl 3-(2-ethoxy-2-oxoethylideneamino)-4-nitrobenzoate

At room temperature, methyl 3-amino-4-nitrobenzoate (4.5 g, 22.94 mmol) and ethyl glyoxylate (9.4 g, 45.88 mmol) were first added into a 250 mL round-bottom flask, then toluene (100 mL) was poured into the flask, and anhydrous magnesium sulfate (5.6 g, 45.88 mmol) was added thereto. After the resulting mixture was stirred at 100°C for 16 h, the reaction solution was filtered. The filtrate was concentrated in vacuum and purified by silica gel column chromatography (PE:EA=2:1) to give methyl 3-(2-ethoxy-2-oxoethylideneamino)-4-nitrobenzoate (2.4 g, yield: 37.3%) as a yellow solid.

LCMS (ESI) m/z: 281.2 [M+H]⁺

### Step II: Synthesis of ethyl 1-(5-methoxycarbonyl)-2-nitrophenyl)-1H-imidazole-5-formate

At room temperature, methyl 3-(2-ethoxy-2-oxoethylideneamino)-4-nitrobenzoate (2.4 g, 8.564 mmol) was first dissolved in THF (40 mL), and then Tosmic (2.5 g, 12.85 mmol) and potassium carbonate (3.0 g, 21.41 mmol) were successively added into the system. After the resulting reaction system was stirred at 75°C for 48 h, the reaction solution was poured over water (50 mL) and extracted with EA (50 mL × 3). The organic phases were combined, dried over anhydrous sodium sulfate and filtered. The filtrate was concentrated under reduced pressure to give a crude product, which was purified by silica gel column chromatography (PE:EA=1:1) to give ethyl 1-(5-methoxycarbonyl)-2-nitrophenyl)-1*H-*imidazole-5-formate (1.2 g, yield: 43.9%) as a yellow solid.

LCMS (ESI) m/z: 320.0 [M+H]⁺

### Step III: Synthesis of methyl 4-oxo-4,5-dihydroimidazo[1,5-a]quinoxaline-8-formate

At room temperature, ethyl 1-(5-methoxycarbonyl)-2-nitrophenyl)-1*H*-imidazole-5-formate (400 mg, 1.253 mmol) and sodium bisulfite (881 mg, 5.011 mmol) were first dissolved in a mixed solvent (20 mL) of acetic acid and water (1:1). Then the mixture was heated to 105°C and kept at this temperature for 16 h. Upon completion of the reaction, the system was poured over water (10 mL) and filtered. The resulting filter cake was washed with water and dried to give methyl 4-oxo-4,5-dihydroimidazo[1,5-*a*]quinoxaline-8-formate (280 mg, yield: 91.9%) as a light yellow solid.

LCMS (ESI) m/z: 244.2 [M+H]⁺

### Step IV: Synthesis of methyl 4-chloroimidazo[1,5-a]quinoxaline-8-carboxylate

At room temperature, methyl 4-oxo-4,5-dihydroimidazo[1,5-a]quinoxaline-8-carboxylate (280 mg, 1.19 mmol) was first dissolved in phosphorus oxychloride (5 mL), then the reaction system was heated to 100°C and stirred for 3 hours while maintaining this temperature. Upon completion of the reaction, the reaction solution was concentrated under reduced pressure and diluted with a small amount of acetonitrile before being poured over water (30 mL). Then the pH was adjusted to 8 with a saturated aqueous solution of sodium hydrogen carbonate and extracted with EA (30 mL × 3). The organic phases were combined, dried over anhydrous sodium sulfate and filtered. The resulting filtrate was concentrated in vacuum to give methyl **4-chloroimidazo[1,5-*a*]quinoxaline-8-carboxylate** (130 mg, yield: 41.7%) as a brown solid.

LCMS (ESI) m/z: 262.0 [M+H]⁺

### Step V: Synthesis of methyl 4-((4-methoxybenzyl)amino)imidazo[1,5-a]quinoxaline-8-carboxylate

At room temperature, methyl 4-chloroimidazo[1,5-*a*]quinoxaline-8-carboxylate (130 mg, 0.4968 mmol) was first dissolved in DMSO (3 mL), and then 4-methoxybenzylamine (136 mg, 0.9936 mmol) and DIEA (197 mg, 1.490 mmol) were successively added into the reaction system. After the resulting mixture was stirred at 90°C for 16 h, the reaction solution was poured over water (20 mL) and extracted with EA (30 mL × 3). The organic phases were combined, dried over anhydrous sodium sulfate and filtered. The filtrate was concentrated under reduced pressure to give a crude product, which was purified by silica gel column chromatography (PE: EA (v/v)=1:1) to give methyl 4-(4-methoxybenzyl)amino)imidazo[1,5-*a*]quinoxaline-8-carboxylate (85 mg, yield: 47.21%) as a yellow solid.

LCMS (ESI) m/z: **363.3** [M+H]⁺

### Step VI: Synthesis of 4-((4-methoxybenzyl)amino)imidazo[1,5-a]quinoxaline-8-carboxylic acid

At room temperature, methyl 4-((4-methoxybenzyl)amino)imidazo[1,5-*a*]quinoxaline-8-carboxylate (85 mg, 0.2346 mmol) was first dissolved in a mixed system of THF and water (3.6 mL, v/v=5:1), and then lithium hydroxide monohydrate (20.0 mg, 0.4691 mmol) was added. After the resulting mixture was stirred at 50°C for 5 h, the pH of the reaction solution was adjusted to 6 with 4M hydrochloric acid and concentrated under reduced pressure to obtain a crude product, 4-((4-methoxybenzyl)amino)imidazo[1,5-*a*]quinoxaline-8-carboxylic acid (85 mg), which was directly used in the reaction of the next step without purification.

LCMS (ESI) m/z: **349.2** [M+H]⁺

### Method II:

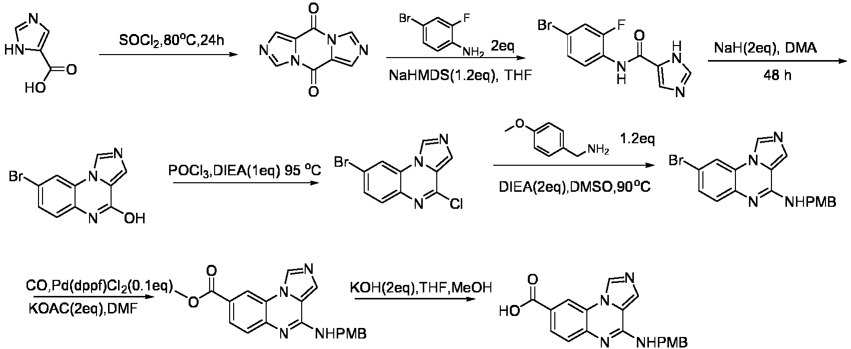

### Step I: Synthesis of imidazo[1,5-a]imidazo[1,5-d]1,4-diazapiperazine-5,10-dione

Imidazole-5-carboxylic acid (100 g, 892.14 mmol) was added into thionyl chloride (500 mL), and the mixture was stirred at 80 °C for 12 h and concentrated under vacuum to give imidazo[1,5-*a*]imidazo[1,5-*d*]1,4-diazapiperazine-5,10-dione (70 g, 0.37 mol, yield: 42%) as a yellow solid.

LCMS (ESI) m/z: 189 [M+H]⁺

### Step II: Synthesis of N-(4-bromo-2-fluorophenyl)imidazol-5-ylformamide

Sodium bistrimethylsilylamide (318.91 mL, 637.82 mmol, 2 mol/L) was slowly added dropwise into a THF (600 mL) mixture of 4-bromo-2-fluorophenylamine (60.60 g, 318.91 mmol) at 0°C within 1h, and then imidazo[1,5-*a*]imidazo[1,5-*d*]1,4-diazapiperazine-5, 10-dione (60 g, 318.91 mmol) was added. The mixture was stirred at room temperature for 12 h, and then poured over water and filtered. The crude product was purified by silica gel column chromatography (PE:EA=40%) to give N-(4-bromo-2-fluorophenyl)imidazol-5-ylformamide (60 g, 0.21 mol, yield: 66%) as a white solid.

LCMS (ESI) m/z: 284 [M+H]⁺

### Step III: Synthesis of 8-bromo-10-hydroimidazo[1,5-a]quinoxalin-4-ol

*N*-(4-bromo-2-fluorophenyl)imidazol-5-ylformamide (60 g, 211.20 mmol) and sodium hydride (16.88 g, 422.40 mmol, content: 60%) were added in dimethylacetamide (600 mL), then the mixture was stirred at 140°C for 12 h. Then the mixture was poured over water and filtered to give 8-bromo-10-hydroimidazo[1,5-*a*]quinoxalin-4-ol (50 g, yield: 90%) as a yellow solid.

LCMS (ESI) m/z: **264** [M+H]⁺

### Step IV: Synthesis of 8-bromo-4-chloro-10-hydroimidazo[1,5-a]quinoxaline

Phosphorus oxychloride (500 mL) was added into a mixture of 8-bromo-10-hydroimidazo[1,5-*a*]quinoxalin-4-ol (50 g, 189.34 mmol) and N,N-diisopropylethylamine (48.85 g, 378.67 mmol). The reaction system was stirred at 90°C for 2 h, and then concentrated. After the residue was dissolved with acetonitrile, it was slowly dropped over ice water for solid precipitation, followed by filtration to give 8-bromo-4-chloro-10-hydroimidazo[1,5-*a*]quinoxaline (50 g, yield: 93%).

LCMS (ESI) m/z: **282** [M+H]⁺

### Step V: Synthesis of (8-bromo(10-hydroimidazo[1,5-a]quinoxalin-4-yl))[(4-methoxyphenyl)methyl]amine

8-Bromo-4-chloro-10-hydroimidazo[1,5-*a*]quinoxaline (50 g, 176.98 mmol) and 4-methoxybenzylamine (29.13 g, 212.38 mmol) were added into DMSO (500 mL), and then *N,N*-diisopropylethylamine (45.66 g, 353.96 mmol) was added. The mixture was stirred at 80°C for 2 h, and then poured over water and filtered to give (8-bromo(10-hydroimidazo[1,5-*a*]quinoxalin-4-yl))[(4-methoxylphenyl)methyl]amine (50 g, yield: 74%) as a yellow oil.

LCMS (ESI) m/z: **384** [M+H]⁺

### Step VI: Synthesis of methyl 4-{[(4-methoxylphenyl)methyl]amino}-10-hydroimidazo[1,5-a]quinoxaline-8-carboxylate

(8-Bromo(10-hydroimidazo[1,5-*a*]quinoxalin-4-yl))[(4-methoxylphenyl)methyl]amine (50 g, 130.47 mmol), [1,1'-bis(diphenylphosphino)ferrocene]palladium dichloride (10.83 g, 13.05 mmol) and potassium acetate (25.57 g, 260.93 mmol) were added into a solution of dimethylformamide (50 mL) and methanol (250 mL). The system was stirred at 100°C for 12 h under an atmosphere of carbon monoxide (4 MPa), then poured over water and filtered, and purified by silica gel column chromatography (PE:EA=80%) to give methyl 4-{[(4-methoxylphenyl)methyl]amino}-10-hydroimidazo[1,5-*a*]quinoxaline-8-carboxylate (30 g, yield: 63%) as a yellow solid.

LCMS (ESI) m/z: 363 [M+H]⁺

### Step VII: Synthesis of methyl 4-{[(4-methoxylphenyl)methyl]amino}-10-hydroimidazo[1,5-a]quinoxaline-8-carboxylic acid

Methyl 4-{[(4-methoxyphenyl)methyl]amino}-10-hydroimidazo[1,5-*a*]quinoxaline-8-carboxylate (30 g, 82.87 mmol) was added into a mixed solution of methanol, water and THF (1:1:1, 150 mL), and potassium hydroxide (92.40 g, 165.0 mmol) was added. The mixture was stirred at 60 °C for 12 h and concentrated in vacuum to remove the organic solvent, and then poured over water, extracted with EA (100 mL × 3) and concentrated in vacuum to give 4-{[(4-methoxylphenyl)methyl]amino}-10-hydroimidazo[1,5-*a*]quinoxaline-8-carboxylic acid (25 g, yield: 86%).

LCMS (ESI) m/z: 349 [M+H]⁺

The intermediate carboxylic acids in the following table may be prepared using the synthesis steps in Method II described for Intermediate 37 with replacement of the corresponding starting materials only:

| Intermediat e No. | Chemical Structure | Chemical Name | m/z(ESI) : (M+H)⁺ |
|---|---|---|---|
| 38 | 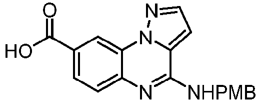 | 4-((4-methoxybenzyl)amino)imidazo[1,2 -*a*]quinoxaline-8-carboxylic acid | 349.12 |
| 39 | 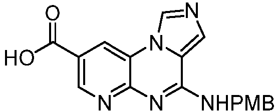 | 4-((4-methoxybenzyl)amino)imidazo[1,5 -*a*]pyrido[2,3-*e*]pyrazine-8-carboxylic acid | 350.2 |
| 40 | 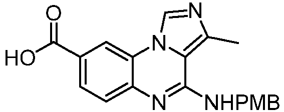 | 4-((4-methoxybenzyl)amino)-3-methylimidazo[1,5-*a*]quinoxaline-8-carboxylic acid | 363.1 |
| 41 | 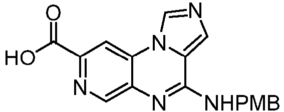 | 4-((4-methoxybenzyl)amino)imidazo[1,5 -*a*]pyrido[3,4-*e*]pyrazine-8-carboxylic acid | 350.1 |
| 42 | 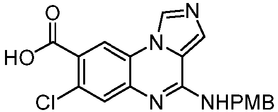 | 7-chloro-4-((4-methoxybenzyl)amino)imidazo[1,5 -*a*]quinoxaline-8-carboxylic acid | 383.1 |
| 43 | 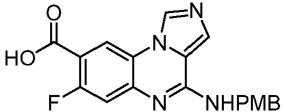 | 7-fluoro-4-((4-methoxybenzyl)amino)imidazo[1,5 -*a*]quinoxaline-8-carboxylic acid | 367.1 |
| 44 | 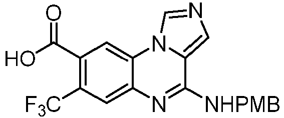 | 4-((4-methoxybenzyl)amino)-7-trifluoromethylimidazo[1,5-*a*]quinoxaline-8-carboxylic acid | 417.1 |

### Intermediate 45 4-((4-methoxybenzyl)amino)pyrrolo[1,2-a]quinoxaline-8-carboxylic acid

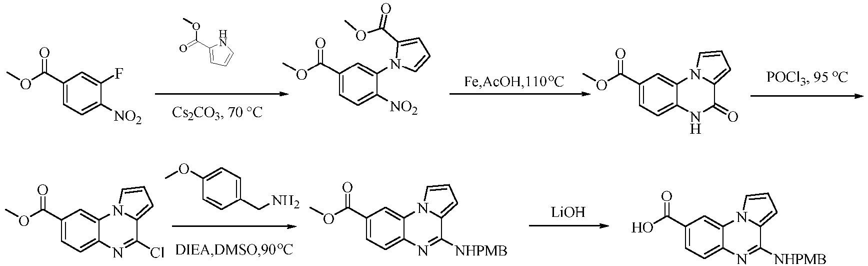

### Step I: Synthesis of methyl 1-(5-(methoxycarbonyl)-2-nitrophenyl)-1H-pyrrole-2-carboxylate

At room temperature, methyl 3-amino-4-nitrobenzoate (5 g, 25.12 mmol) and methyl 1*H-*pyrrole-2-carboxylate (4.711 g, 37.68 mmol) were first added into a 250 mL round-bottom flask, then *N,N*-dimethylformamide (100 mL) was poured into the flask, and cesium carbonate (24.5 g, 75.37 mmol) was added. After the resulting mixture was stirred at 70°C for 6 h, the reaction solution was filtered. The filtrate was concentrated in vacuum and purified by silica gel column chromatography (PE:EA=2:1) to give methyl 1-(5-(methoxylcarbonyl)-2-nitrophenyl)-1*H*-pyrrole-2-carboxylate (3.0 g, yield: 39.2%) as a yellow solid.

LCMS (ESI) m/z: 305.2[M+H]⁺

### Step II: Synthesis of methyl 4-oxo-4,5-dihydropyrrolo[1,2-a]quinoxaline-8-carboxylate

At room temperature, methyl 1-(5-(methoxycarbonyl)-2-nitrophenyl)-1*H*-pyrrole-2-carboxylate (3.0 g, 22.94 mmol) was dissolved in acetic acid (60 mL), and then iron powder (7.7 g, 137.64 mmol) was successively added into the system. After the resulting reaction system was stirred at 110°C for 4 h, the reaction solution was poured over water (100 mL) and filtered. The resulting filter cake was washed with water and dried to give methyl 4-oxo-4,5-dihydropyrrolo[1,2-*a*]quinoxaline-8-carboxylate (1.8 g, yield: 32.4%) as a grey solid.

LCMS (ESI) m/z: 243.1 [M+H]⁺

### Step III: Synthesis of methyl 4-chloropyrrolo[1,2-a]quinoxaline-8-carboxylate

At room temperature, methyl 4-oxo-4,5-dihydropyrrolo[1,2-*a*]quinoxaline-8-carboxylate (1.8 g, 7.43 mmol) was first dissolved in phosphorus oxychloride (20 mL), then the mixture was heated to 90°C and stirred for 3 h while maintaining this temperature. Upon completion of the reaction, the system was poured over water (50 mL) and filtered. The resulting filter cake was washed with water and dried to give methyl 4-chloropyrrolo[1,2-*a*]quinoxaline-8-carboxylate (1.3 g, yield: 67.2%) as a brown solid.

LCMS (ESI) m/z: 261.2[M+H]⁺

### Step IV: Synthesis of methyl 4-((4-methoxybenzyl)amino)pyrrolo[1,2-a]quinoxaline-8-carboxylate

At room temperature, methyl 4-chloropyrrolo[1,2-*a*]quinoxaline-8-carboxylate (1.3 g, 5.000 mmol) was first dissolved in DMSO (25 mL), and then 4-methoxybenzylamine (1.027 g, 7.500 mmol) and DIEA (1.935 g, 15.00 mmol) were successively added into the reaction system. After the resulting mixture was stirred at 90°C for 8 h, the reaction solution was poured over water (50 mL) and extracted with EA (50 mL × 3). The organic phases were combined, dried over anhydrous sodium sulfate and filtered. The filtrate was concentrated under reduced pressure to give a crude product, which was then purified by silica gel column chromatography (PE: EA (v/v)=1:1) to give methyl 4-((4-methoxybenzyl)amino)imidazo[1,5-*a*]quinoxaline-8-carboxylate (1.4 g, yield: 77.7%) as a yellow solid.

LCMS (ESI) m/z: 361.1 [M+H]⁺

### Step V: Synthesis of 4-((4-methoxybenzyl)amino)pyrrolo[1,2-a]quinoxaline-8-carboxylic acid

At room temperature, methyl 4-((4-methoxybenzyl)amino)pyrrolo[1,2-*a*]quinoxaline-8-carboxylate (130 mg, 0.3601 mmol) was first dissolved in a mixed system of THF, methanol and water (4 mL, v/v=2:2:1), and then lithium hydroxide monohydrate (25.9 mg, 1.080 mmol) was added. After the resulting mixture was stirred at 25°C for 16 h, the pH of the reaction solution was adjusted to 6 with 4M hydrochloric acid,and concentrated under reduced pressure to give a crude product, 4-((4-methoxybenzyl)amino)pyrrolo[1,2-*a*]quinoxaline-8-carboxylic acid (100 mg, crude product), which was directly used in the reaction of the next step without purification.

LCMS (ESI) m/z: 348.1 [M+H]⁺

The intermediate carboxylic acids in the following table may be prepared using the synthesis steps described for Intermediate 45 with replacement of the corresponding starting materials only:

| Intermediat e No. | Chemical Structure | Chemical Name | m/z(ESI) : (M+H)⁺ |
|---|---|---|---|
| 46 | 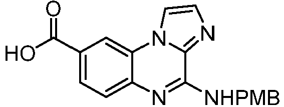 | 4-((4-methoxybenzyl)amino)imidazo[1,2 -*a*]quinoxaline-8-carboxylic acid | 349.1 |
| 47 | 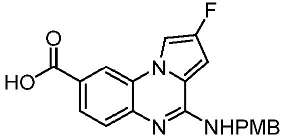 | 2-fluoro-4-((4-methoxybenzyl)amino)pyrrolo[1,2-*a*]quinoxaline-8-carboxylic acid | 366.1 |
| 48 | 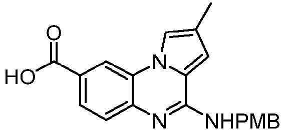 | 4-((4-methoxybenzyl)amino)-2-methylpyrrolo[1,2-*a*]quinoxaline-8-carboxylic acid | 362.1 |
| | | | |
| 49 | 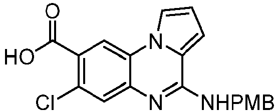 | 7-chloro-4-((4-methoxybenzyl)amino)pyrrolo[1,2-*a*]quinoxaline-8-carboxylic acid | 382.1 |
| 50 | 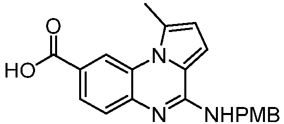 | 4-((4-methoxybenzyl)amino)-1-methylpyrrolo[1,2-*a*]quinoxaline-8-carboxylic acid | 362.1 |
| 51 | 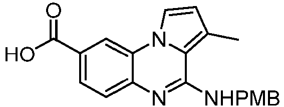 | 4-((4-methoxybenzyl)amino)-3-methylpyrrolo[1,2-*a*]quinoxaline-8-carboxylic acid | 362.1 |

### Intermediate 52 4-((4-methoxybenzyl)amino)-1-methylimidazo[1,5-a]quinoxaline-8-carboxylic acid

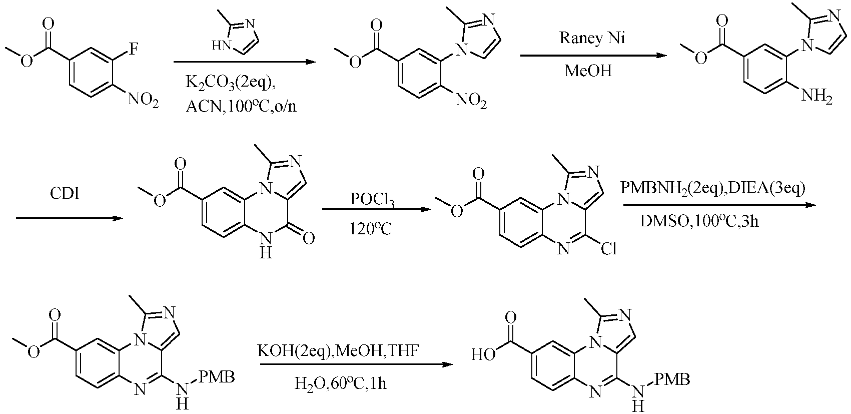

### Step I: Synthesis of methyl 3-(2-methyl-1H-imidazol-1-yl)-4-nitrobenzoate

Methyl 3-fluoro-4-nitrobenzoate (20.0 g, 100 mmol) was dissolved in acetonitrile (100 mL), and then 2-methyl-1*H*-imidazole (8.2 g, 100 mmol) and potassium carbonate (27.6 g, 200 mmol) were added. The mixture was stirred at 100°C for 12 h. The reaction was monitored by LCMS. The reaction solution was poured over water (300 mL), and EA (200 mL × 3) was added for extraction. The organic phases were combined and dried over anhydrous sodium sulfate. The organic phases were concentrated and purified by silica gel column chromatography (PE:EA=10%) to give methyl 3-(2-methyl-1*H*-imidazol-1-yl)-4-nitrobenzoate (25.0 g, yield: 95%) as a yellow solid.

LCMS (ESI) m/z: 262 [M+H]⁺

### Step II: Synthesis of methyl 4-amino-3-(2-methyl-1H-imidazol-1-yl)benzoate

At room temperature, methyl 3-(2-methyl-1*H*-imidazol-1-yl)-4-nitrobenzoate (25 g, 95.8 mmol) was dissolved in methanol (300 mL), and then Raney Nickel (2 g) was added. The mixture was stirred under a hydrogen atmosphere at room temperature for 12 h. The reaction was monitored by LCMS. The residue was filtered to give a filtrate, and the filtrate was concentrated and purified by silica gel column chromatography (PE:EA=20%) to give methyl 4-amino-3-(2-methyl-1*H*-imidazol-1-yl)benzoate (20.5 g, 93%) as a yellow solid.

LCMS (ESI) m/z: 232 [M+H]⁺

### Step III: Synthesis of methyl 1-methyl-4-oxo-4,5-dihydroimidazo[1,5-a]quinoxaline-8-carboxylate

At room temperature, methyl 4-amino-3-(2-methyl-1*H*-imidazol-1-yl)benzoate (2.0 g, 8.7 mmol) was dissolved in o-dichlorobenzene (40 mL), and then carbonyldiimidazole (2.8 g, 17.4 mmol) was added. The mixture was stirred at 180°C for 12 h. The reaction was monitored by LC-MS. The mixture was filtered to give a filter cake, and the filter cake was slurried in EA (5 mL) to give methyl 1-methyl-4-oxo-4,5-dihydroimidazo[1,5-*a*]quinoxaline-8-carboxylate (920.0 mg, yield: 41%) as a black solid.

LCMS (ESI) m/z: 258 [M+H]⁺

### Step IV: Synthesis of methyl 4-chloro-1-methylimidazo[1,5-a]quinoxaline-8-carboxylate

At room temperature, methyl 1-methyl-4-oxo-4,5-dihydroimidazo[1,5-*a*]quinoxaline-8-carboxylate (100.0 mg, 0.39 mmol) was dissolved in phosphorus oxychloride (5 mL). The mixture was stirred at 120°C for 4 h. The reaction was monitored by LC-MS. The reaction solution was concentrated under reduced pressure, diluted with a small amount of acetonitrile, and then poured over water (5 mL) and filtered. The filter cake was washed with water and vacuum dried to give methyl 4-chloro-1-methylimidazo[1,5-*a*]quinoxaline-8-carboxylate (60.0 mg, yield: 56%) as a black solid.

LCMS (ESI) m/z: 276 [M+H]⁺

### Step V: Synthesis of methyl 4-((4-methoxybenzyl)amino)-1-methylimidazo[1,5-a]quinoxaline-8-carboxylate

At room temperature, methyl 4-chloro-1-methylimidazo[1,5-*a*]quinoxaline-8-carboxylate (60 mg, 0.22 mmol) was dissolved in DMSO (2 mL), and then (4-methoxyphenyl)methanamine (60 mg, 0.44 mmol) and *N,N*-diisopropylethylamine (67 mg, 0.52 mmol) were added. The mixture was stirred at 100°C for 1 h. The reaction was monitored by LC-MS. The reaction solution was poured over water (10 mL), and EA (10 mL × 3) was added for extraction. The organic phases were combined and dried over anhydrous sodium sulfate. The organic phases were concentrated and purified by silica gel column chromatography (PE:EA=40%) to give methyl 4-((4-methoxybenzyl)amino)-1-methylimidazo[1,5-*a*]quinoxaline-8-carboxylate (60 mg, yield: 73%) as a black solid.

LCMS (ESI) m/z: 377 [M+H]⁺

### Step VI: Synthesis of 4-((4-methoxybenzyl)amino)-1-methylimidazo[1,5-a]quinoxaline-8-carboxylic acid

At room temperature, methyl 4-((4-methoxybenzyl)amino)-1-methylimidazo[1,5-*a*]quinoxaline-8-carboxylate (60 mg, 0.16 mmol) was dissolved in methanol (1 mL), THF (1 mL) and water (1 mL), and then potassium hydroxide (26 mg, 0.44 mmol) was added. The mixture was stirred at room temperature for 1 h. The reaction was monitored by LC-MS. The reaction solution was poured over water (10 mL) and adjusted to be acidic by addition of formic acid, and EA (10 mL × 3)) was added for extraction. The organic phases were combined and dried over anhydrous sodium sulfate. The organic phases were concentrated to give 4-((4-methoxybenzyl)amino)-1-methylimidazo[1,5-*a*]quinoxaline-8-carboxylic acid (50 mg, yield: 86%) as a white solid.

LCMS (ESI) m/z: 363 [M+H]⁺

The intermediate carboxylic acid in the following table may be prepared using the synthesis steps described for Intermediate 52 with replacement of the corresponding starting material only:

| Intermediate No. | Chemical Structure | Chemical Name | m/z(ESI): (M+H)⁺ |
|---|---|---|---|
| 53 | 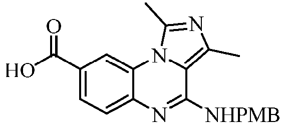 | 4-((4-methoxybenzyl)amino)-1,3-dimethylimidazo[1,5-*a*]quinoxaline-8-carboxylic acid | 377.1 |

### Intermediate 54: 4-amino-7-cyanoimidazo[1,5-a]quinoxaline-8-carboxylic acid

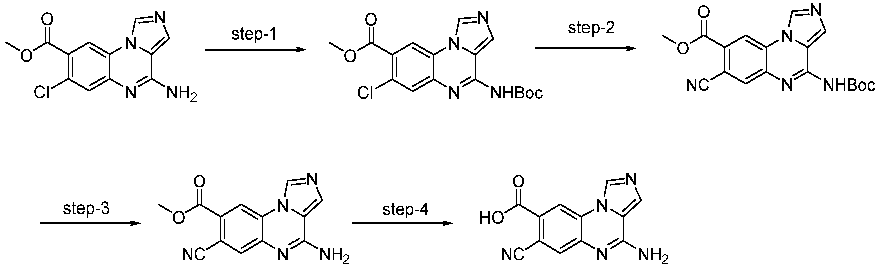

### Step I: Synthesis of methyl 4-((t-butoxycarbonyl)amino)-7-chloroimidazo[1,5-a]quinoxaline-8-carboxylate

A solution of methyl 4-amino-7-chloroimidazo[1,5-*a*]quinoxaline-8-carboxylate (1.20 g, 4.34 mmol), di-t-butyl dicarbonate (1.89 g, 8.67 mmol) and triethylamine (1.30 g, 13.01 mmol) in DCM (10 mL) was stirred at room temperature for 2 h. Upon completion of the reaction, the reaction solution was poured over water (5 mL) and extracted with EA (5 mL). The organic phases were dried over anhydrous sodium sulfate and filtered, then concentrated and purified by column chromatography (PE:EA=70:30) to give methyl 4-((t-butoxycarbonyl)amino)-7-chloroimidazo[1,5-*a*]quinoxaline-8-carboxylate (1.00 g, yield: 61% as a white solid.

LCMS (ESI): 376 [M+H]⁺

### Step II: Synthesis of methyl 4-((t-butoxycarbonyl)amino)-7-cyanoimidazo[1,5-a]quinoxaline-8-carboxylate

Under an atmosphere of N₂, a mixture of methyl 4-((t-butoxycarbonyl)amino)-7-chloroimidazo[1,5-*a*]quinoxaline-8-carboxylate)carbonylamino]-7-chloro-10-hydroimidazo[1,5-*a*]quinoxaline-8-carboxylate (0.20 g, 0.53 mmol), potassium ferricyanide (0.05 g, 0.16 mmol), 2-di-t-butylphosphino-2',4',6'-triisopropylbiphenyl (0.09 g, 0.21 mmol), methanesulfonato(2-di-t-butylphosphino-2',4',6'-triisopropyl-1,1'-biphenyl)(2'-amino-1,1'-biphenyl-2-yl)palladium(II) (0.17 g, 0.21 mmol) and potassium acetate (0.01 g, 0.07 mmol) in dioxane/water (4.00 mL) were stirred at 100°C for 2 h, and then the mixture was concentrated and purified by column chromatography (PE:EA=70:30) to give methyl 4-((t-butoxycarbonyl)amino)-7-cyanoimidazo[1,5-*a*]quinoxaline-8-carboxylate (0.10 g, 51%) as a white solid.

LCMS (ESI): 368[M+H]⁺

### Step III: Synthesis of methyl 4-amino-7-cyanoimidazo[1,5-a]quinoxaline-8-carboxylate

Trifluoroacetic acid (0.10 mL) was added into a solution of methyl 4-((t-butoxycarbonyl)amino)-7-cyanoimidazo[1,5-*a*]quinoxaline-8-carboxylate (0.20 g, 0.54 mmol) in DCM (0.50 mL). The reaction solution was stirred at room temperature for 2 h and then concentrated to give methyl 4-amino-7-cyanoimidazo[1,5-*a*]quinoxaline-8-carboxylate (0.05 g, crude product).

LCMS (ESI): 268[M+H]⁺

### Step IV: Synthesis of 4-amino-7-cyanoimidazo[1,5-a]quinoxaline-8-carboxylic acid

A mixture of methyl 4-amino-7-cyanoimidazo[1,5-*a*]quinoxaline-8-carboxylate (0.20 g, 0.75 mmol), potassium hydroxide (0.07 g, 1.50 mmol) and THF/methanol/water (1 mL/1 mL/1 mL) was stirred at room temperature for 2 h. Upon completion of the reaction, the system was concentrated to give a crude product, 4-amino-7-cyanoimidazo[1, 5-*a*]quinoxaline-8-carboxylic acid crude (210 mg, crude product). The crude product was directly used in the reaction of the next step without purification.

LCMS (ESI): 254[M+H]⁺

### Intermediate 55 4-amino-7-methylimidazo[1,5-a]quinoxaline-8-carboxylic acid

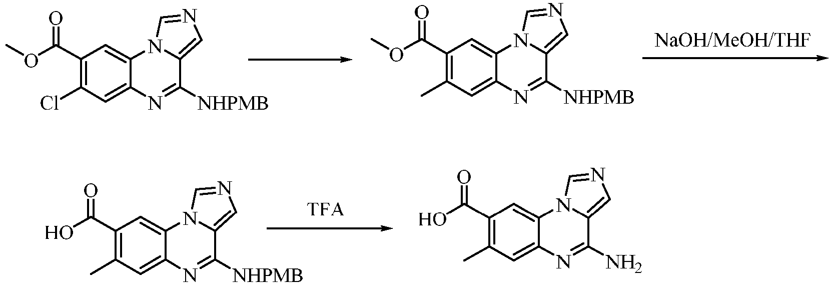

### Step I: Synthesis of methyl 4-{[(4-methoxyphenyl)methyl]amino}-7-methyl-10-hydroimidazo[1,5-a]quinoxaline-8-carboxylate

A mixture of methyl 7-chloro-4-{[(4-methoxylphenyl)methyl]amino}-10-hydroimidazo[1,5-*a*]quinoxaline-8-carboxylate (200 mg, 0.5 mmol), potassium carbonate (300 mg, 2 mmol), dichloro[1,1'-bis(t-butylphosphino)ferrocene palladium (II) (0.08 g, 0.1 mmol) and trimethylboronoxane (0.01 g, 0.10 mmol) was stirred in dioxane (2.00 mL) at 100°C for 12 h, then poured over water, extracted with EA (5 mL), dried over anhydrous sodium sulfate, filtered and purified by column chromatography (PE:EA=30%) to give methyl 4-{[(4-methoxyphenyl)methyl]amino}-7-methyl-10-hydroimidazo[1,5-*a*]quinoxaline-8-carboxylate (100 mg, yield: 53%).

LCMS (ESI):268 [M+H]⁺

### Step II: Synthesis of 4-((4-methoxybenzyl)amino)-7-methylimidazo[1,5-a]quinoxaline-8-carboxylic acid

Methyl 4-{[(4-methoxyphenyl)methyl]amino}-7-methyl-10-hydroimidazo[1,5-*a*]quinoxaline-8-carboxylate (500 mg, 1.86 mmol) was dissolved in an aqueous solution of methanol: THF: saturated potassium hydroxide (1 mL: 1 mL: 1 mL). The reaction solution was stirred at 60 °C for 12 h, then concentrated in vacuum, the pH was adjusted to 3 with formic acid, and then the reaction solution was filtered to give 4-((4-methoxybenzyl)amino)-7-methylimidazo[1,5-*a*]quinoxaline-8-carboxylic acid (52 mg, yield: 12%).

LCMS (ESI):363 [M+H]⁺

### Step III: Synthesis of 4-amino-7-methylimidazo[1,5-a]quinoxaline-8-carboxylic acid

4-((4-Methoxybenzyl)amino)-7-methylimidazo[1,5-*a*]quinoxaline-8-carboxylic acid (500 mg, 1.86 mmol) was dissolved in a TFA solution (5 mL). The reaction solution was stirred at 100°C for 12 h, then concentrated in vacuum to give 4-amino-7-methylimidazo[1,5-*a*]quinoxaline-8-carboxylic acid (52 mg, 0.23 mmol).

LCMS (ESI):363 [M+H]⁺

The following intermediate acids were prepared by the method used in the third step for

### Intermediate 55:

| Intermediate No. | Chemical Structure | Chemical Name | m/z(ESI): (M+H)⁺ |
|---|---|---|---|
| 56 | 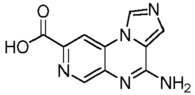 | 4-aminoimidazo[1,5-*a*]pyrido[3,4-*e*]pyrazine-8-carboxylic acid | 230.1 |
| 57 | 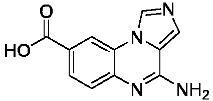 | 4-amino-10-hydroimidazo[1,5-*a*]quinoxaline-8-carboxylic acid | 229.1 |
| 58 | 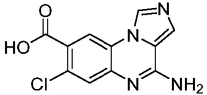 | 4-amino-10-hydroimidazo[1,5-*a*]quinoxaline-8-carboxylic acid | 263.1 |
| 59 | 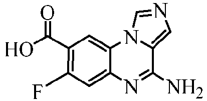 | 4-amino-7-fluoroimidazo[1,5-*a*]quinoxaline-8-carboxylic acid | 247.1 |
| 60 | 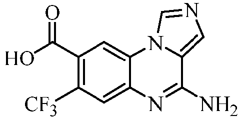 | 4-amino-7-(trifluoromethyl)imidazo[1,5-*a*]quinoxaline-8-carboxylic acid | 297.1 |
| 61 | 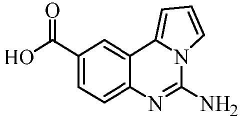 | 5-aminopyrrolo[1,2-*c*]quinoxaline-9-carboxylic acid | 228.1 |

### Intermediate 62 4-amino-[1,2,4]triazolo[4,3-a]quinoxaline-8-formic acid

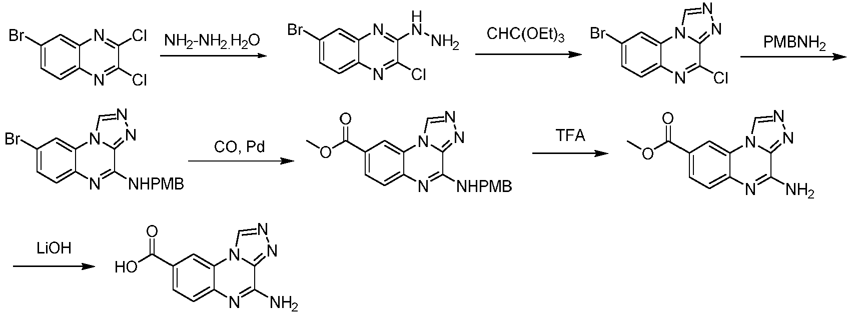

### Step I: Synthesis of 6-bromo-2-chloro-3-hydrazinoquinoxaline

At room temperature, 6-bromo-2,3-dichloroquinoxaline (278.0 mg, 1.0 mmol) was first added into a 25 mL single-neck flask, and hydrazine hydrate (156 mg, 2.5 mmol, 80 wt%) was added. After the addition, the resulting reaction mixture was allowed to sit at room temperature overnight. Upon completion of the reaction, filtration was carried out, and the filter cake was washed with water (10 mL × 2) and EA (5 mL × 2) to give 6-bromo-2-chloro-3-hydrazinoquinoxaline (150 mg).

LCMS (ESI) m/z:273.0/275.0 [M+H]⁺

### Step II: Synthesis of 8-bromo-4-chloro-[1,2,4]triazolo[4,3-a]quinoxaline

At room temperature, 6-bromo-2-chloro-3-hydrazinoquinoxaline (116.0 mg, 0.5 mmol) was first added into a 25 mL single-neck flask, and triethyl orthoformate (4.0 mL) was added thereto. After the addition, the reaction system was heated to 100°C and stirred at this temperature for 1 h. Upon completion of the reaction as indicated by LCMS, the reaction system was cooled to room temperature and filtered. The filter cake was washed with methanol (3.0 mL × 3) and dried to give 8-bromo-4-chloro-[1,2,4]triazolo[4,3-*a*]quinoxaline (110 mg).

¹H NMR (400 MHz, CDCl₃) δ 10.21(s, 1H), 8.84(d, 1H), 7.98(d, 1H), 7.90(dd, 1H).

### Step III: Synthesis of 8-bromo-N-(4-methoxybenzyl)-[1,2,4]triazolo[4,3-a]quinoxaline-4-amine

8-Bromo-4-chloro-[1,2,4]triazolo[4,3-*a*]quinoxaline (1.70 g, 6.00 mmol) was dissolved in DMSO (15.0 mL), and p-methoxybenzylamine (1.23 g, 9.00 mmol) and DIEA (2.32 g, 18.00 mmol) were added into the reaction system, which was stirred at 90°C for 4 h. Upon completion of the reaction, water (60 mL) was added, and the mixture was extracted with EA (40 mL × 3). The organic phases were dried over sodium sulfate, filtered and dried by evaporation to give a crude product, 8-bromo-*N*-(4-methoxybenzyl)-[1,2,4]triazolo[4,3-*a*]quinoxaline-4-amine (2.20 g).

LCMS (ESI) m/z: 384.1/386.1 [M+H]⁺

### Step IV: Synthesis of methyl 4-((4-methoxybenzyl)amino)-[1,2,4]triazolo[4,3-a]quinoxaline-8-carboxylate

At room temperature, potassium acetate (1.7 g, 17.2 mmol) and Pd(dppf)Cl₂ (420 mg, 0.57 mmol) were added into solutions of 8-bromo-*N*-(4-methoxybenzyl)-[1,2,4]triazolo[4,3-*a*]quinoxaline-4-amine (2.2 g, 5.74 mmol) in MeOH (30 mL) and DMF (30 mL). Under a CO atmosphere, the mixture was stirred at 100°C for 12 h. After removal of the methanol by rotary evaporation, 100 mL of water was added and the mixture was extracted with EA (50 mL × 3). The organic phases were combined, dried over anhydrous Na₂SO₄ and concentrated in vacuum to give a crude product, which was slurried in EA (20 mL) to give methyl 4-((4-methoxybenzyl)amino)-[1,2,4]triazolo[4,3-*a*]quinoxaline-8-carboxylate (1.1 g, yield: 53%).

LCMS (ESI) m/z: 364.2 [M+H]+

### Step V: Synthesis of methyl 4-amino-[1,2,4]triazolo[4,3-a]quinoxaline-8-carboxylate.

The compound methyl 4-((4-methoxybenzyl)amino)-[1,2,4]triazolo[4,3-*a*]quinoxaline-8-carboxylate (300.0 mg, 0.78 mmol) was placed in a 25 mL single-neck flask, and TFA (5.0 mL) was added thereto. The reaction system was heated to 80°C, and stirred at this temperature for 16 h. Upon completion of the reaction, the reaction solution was concentrated in vacuum to give a crude methyl 4-amino-[1,2,4]triazolo[4,3-*a*]quinoxaline-8-carboxylate (190 mg).

LCMS (ESI) m/z: 244.3 [M+H]⁺

### Step VI: Synthesis of 4-amino-[1,2,4]triazolo[4,3-a]quinoxaline-8-carboxylic acid.

THF (5.0 mL) and methanol (5.0 mL) were added into the crude methyl 4-amino-[1,2,4]triazolo[4,3-*a*]quinoxaline-8-carboxylate (190 mg, 0.78 mmol) of the last step, the pH was adjusted to 7 with a 3 M aqueous solution of potassium hydroxide, and then lithium hydroxide (65.0 mg, 1.56 mmol) was added. The reaction mixture was stirred at 50°C for 16 h. Upon completion of the reaction, methanol and THF in the system were removed by rotary evaporation, and the pH was adjusted to 6.5 with a 1 M dilute hydrochloric acid solution. After filtration, the filter cake was washed with water (5.0 mL × 3), and then water was removed to give a crude 4-amino-[1,2,4]triazolo[4,3-*a*]quinoxaline-8-carboxylic acid (120 mg).

LCMS (ESI) m/z: 230.1 [M+H]⁺

### Intermediate 63 5-aminoimidazo[1,5-c]quinazoline-9-carboxylic acid

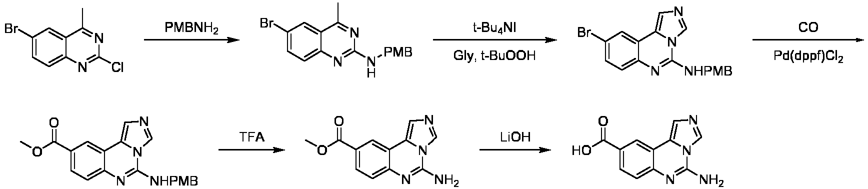

### Step I: Synthesis of 6-bromo-N-(4-methoxybenzyl)-4-methylquinazoline-2-amine

At room temperature, 6-bromo-2-chloro-4-methylquinazoline (0.5 g, 1.942 mmol) was dissolved in DMSO (15 mL), and DIEA (768 mg, 5.825 mmol) and 4-methoxybenzylamine (600 mg, 3.883 mmol) were added. The reaction mixture was stirred at room temperature for 16 h, and then water (50 mL) was added, and extracted with EA (20 mL × 3). The organic phases were combined, dried over anhydrous sodium sulfate and filtered. The filtrate was concentrated under reduced pressure and purified by column chromatography (0-10% methanol/DCM) to give 6-bromo-*N*-(4-methoxybenzyl)-4-methylquinazoline-2-amine (500 mg, yield: 72%).

LCMS (ESI) m/z: 359.2 [M+H]⁺

### Step II: Synthesis of 9-bromo-N-(4-methoxybenzyl)imidazo[1,5-c]quinazoline-5-amine

At room temperature, 6-bromo-*N*-(4-methoxybenzyl)-4-methylquinazoline-2-amine (0.5 g, 1.396 mmol) was dissolved in DMSO (20 mL) , and glycine (211 mg, 2.792 mmol), t-butyl hydroperoxide (719 mg, 5.583 mmol), tetrabutylammonium iodide (104 mg, 0.2792 mmol) and acetic acid (251 mg, 4.187 mmol) were added. The reaction mixture was stirred at 90°C for 16 h under an atmosphere of N₂, and then water (60 mL) was added and extracted with EA (30 mL × 3). The organic phases were combined, dried over anhydrous sodium sulfate and filtered. The filtrate was concentrated under reduced pressure and purified by column chromatography (0-10% methanol/DCM) to give 9-bromo-*N*-(4-methoxybenzyl)imidazo[1,5-c]quinazoline-5-amine (300 mg, yield: 56%).

LCMS (ESI) m/z: 384.2 [M+H]⁺

### Step III: Synthesis of methyl 5-((4-methoxybenzyl)amino)imidazo[1,5-c]quinazoline-9-carboxylate

At room temperature, potassium acetate (230 mg gel column chromatography, 2.36 mmol) and Pd(dppf)Cl₂ (58 mg, 0.0785 mmol) were added into solutions of 9-bromo-N-(4-methoxybenzyl)imidazo[1,5-c]quinazoline-5-amine (300 mg, 0.785 mmol) in MeOH (10 mL) and DMF (10 mL). Under a CO atmosphere, the mixture was stirred at 100°C for 12 h. After removal of the methanol by rotary evaporation, water (60 mL) was added and extracted with EA (30 mL × 3). The organic phases were combined, dried over anhydrous sodium sulfate and filtered. The filtrate was concentrated under reduced pressure and purified by column chromatography (0-10% methanol/DCM) to give methyl 5-(4-methoxybenzyl)amino)imidazo[1,5-c]quinazoline-9-carboxylate (200 mg, yield: 56%).

LCMS (ESI) m/z: 363.2 [M+H]⁺

### Step IV: Synthesis of methyl 5-aminoimidazo[1,5-c]quinazoline-9-carboxylate

At room temperature, methyl 5-(4-methoxybenzyl)amino)imidazo[1,5-c]quinazoline-9-carboxylate (200 mg, 0.5519 mmol) was placed in a 20 mL round-bottom flask, and TFA (2 mL) was added into the mixture, which was stirred at 78°C for 3 h. After removal of the TFA by rotary evaporation, water (5 mL) was added, followed by filtration, and the filter cake was washed with water to give methyl 5-aminoimidazo[1, 5-c]quinazoline-9-carboxylate (200 mg, crude).

LCMS (ESI) m/z: 243.2 [M+H]⁺

### Step V: Synthesis of 5-aminoimidazo[1,5-c]quinazoline-9-carboxylic acid

At room temperature, methyl 5-((4-methoxybenzyl)amino)imidazo[1,5-c]quinazoline-9-carboxylate (200 mg, 0.8257 mmol) was dissolved in a mixed solvent of methanol/THF/water (9 mL, 4:4:1), and lithium hydroxide (40 mg, 1.651 mmol) was added. The mixture was stirred at 78°C for 3 h. After removal of the TFA by rotary evaporation, water (5 mL) was added and filtrated. Then the filter cake was washed with water to give 5-aminoimidazo[1,5-c]quinazoline-9-carboxylic acid (200 mg, crude).

LCMS (ESI) m/z: 243.2 [M+H]⁺

### Example 1

### 4-amino-N-cyclopropyl-N-(5-trifluoromethylpyridin-2-yl)methyl)imidazo[1,5-a]quinoxaline-8-formamide

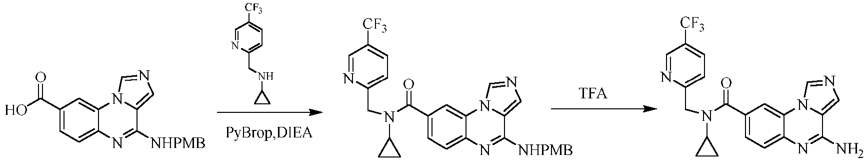

### Step I: Synthesis of N-cyclopropyl-4-(4-methoxybenzyl)amino)-N-(5-trifluoromethyl)pyridin-2-yl)methyl)imidazo[1,5-a]quinoxaline-8-formamide

At room temperature, 4-((4-methoxybenzyl)amino)imidazo[1,5-*a*]quinoxaline-8-carboxylic acid (85 mg, 0.24 mmol) was dissolved in *N,N*-dimethylacetamide (3 mL), and then cyclopropyl{[5-(trifluoromethyl)(2-pyridinyl)]methyl}amine (20.0 mg, 0.469 mmol), bromo-tris-pyrrolidino-phosphonium hexafluorophosphate (PyBrOP) (230 mg, 0.488 mmol), and *N,N-*diisopropylethylamine (DIEA) (96 mg, 0.7320 mmol) were added into the system successively. The resulting mixture was stirred at 50°C for 3 h. After the reaction completed, the reaction solution was poured over water (15 mL), and the mixture was extracted with ethyl acetate (EA) (10 mL × 3). The combined organic phases were dried over anhydrous sodium sulfate and filtered. The resulting filtrate was concentrated under reduced pressure to give a crude product, which was then purified by silica gel column chromatography (petroleum ether (PE):EA = 1:1, v/v) to give *N*-cyclopropyl-4-(4-methoxybenzyl)amino)-*N-*(5-trifluoromethyl)pyridin-2-yl)methyl)imidazo[1,5-*a*]quinoxaline-8-formamide (100 mg, yield: 75%) as a yellow solid.

LCMS (ESI) m/z: 559.3 [M+H]⁺

### Step II: Synthesis of 4-amino-N-cyclopropyl-N-(5-trifluoromethylpyridin-2-yl)methyl)imidazo[1,5-a]quinoxaline-8-formamide

At room temperature, *N*-cyclopropyl-4-(4-methoxybenzyl)amino)-*N*-(5-trifluoromethyl)pyridin-2-yl)methyl)imidazo[1,5-*a*]quinoxaline-8-formamide (100 mg, 0.183 mmol) was dissolved in trifluoroacetic acid (2 mL). The resulting mixture was stirred at 50°C for 3 hours. Then the reaction solution was concentrated under reduced pressure, poured over water (10 mL), then adjusted to pH= 8 with saturated sodium bicarbonate solution, and extracted with EA (10 mL×3). The combined organic phases were dried over anhydrous sodium sulfate and filtered. The resulting filtrate was concentrated under reduced pressure to give a crude product, which was then purified by reversed-phase column chromatography (water to acetonitrile (v/v) = 5:95) to give 4-amino-*N*-cyclopropyl-*N*-(5-trifluoromethylpyridin-2-yl)methyl)imidazo[1,5-*a*]quinoxaline-8-formamide (4.98 mg, yield: 3.96%) as a white solid.

LCMS (ESI) m/z: 427.3 [M+H]⁺

¹H NMR (400 MHz, CDCl₃) δ 8.91 - 8.84 (m, 1H), 8.63 (s, 1H), 8.10 (d, *J =* 1.8 Hz, 1H), 7.93 (dd, *J =* 8.2, 2.3 Hz, 1H), 7.76 - 7.67 (m, 2H), 7.60 (d, *J* = 8.4 Hz, 1H), 7.49 (d, *J =* 8.2 Hz, 1H), 5.39 (s, 2H), 4.97 (s, 2H), 1.10 (s, 1H), 0.67 (d, *J* = 6.8 Hz, 2H), 0.59 (s, 2H).

The examples in the following table may be prepared using the synthetic step described in Example 1, only replacing the corresponding starting materials:

| Exa mple No. | Chemical Structure | Chemical Name | ¹HNMR&¹⁹F NMR& LCMS |
|---|---|---|---|
| 2 | 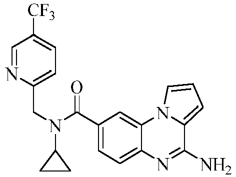 | 4-amino-*N-*cyclopropyl-*N*-(5-trifluoromethylpyridi n-2-yl)methyl)pyrrolo[1,5 -*a*]quinoxaline-8-formamide | ¹H NMR (400 MHz, DMSO-*d₆*) δ 8.98 (s, 1H), 8.31 (s, 1H), 8.24 - 8.18 (m, 2H), 7.64 (d, *J* = 8.4 Hz, 1H), 7.54 - 7.47 (m, 1H), 7.41 (d, *J* = 8.4 Hz, 1H), 7.11 (s, 2H), 7.10 - 7.06 (m, 1H), 6.77 -6.73 (m, 1H), 4.88 (s, 2H), 3.12 (s, 1H), 0.54 (d, *J* = 7.6 Hz, 4H). |
| | | | LCMS (ESI) m/z: 426.1 [M+H]⁺ |
| 3 | 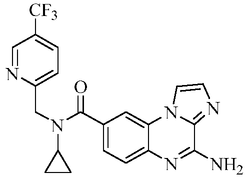 | 4-amino-*N-*cyclopropyl-*N*-(5-trifluoromethylpyridi n-2-yl)methyl)imidazo[1, 2-*a*]quinoxaline-8-formamide | ¹H NMR (400 MHz, CDCl₃) δ 8.88(s, 1H), 8.02(d, 2H), 7.93(dd, 1H), 7.76-7.72(m, 1H), 7.68(d, 1H), 7.65(d, 1H), 7.50(d, 1H), 5.87(s, 2H), 4.98(s, 2H), 3.03(s, 1H), 0.66(d, 2H), 0.58(s, 2H). |
| | | | LCMS (ESI) m/z: 427.3 [M+H]⁺ |
| 4 | 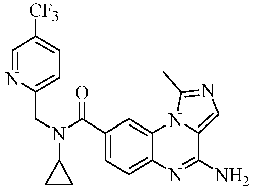 | 4-amino-*N-*cyclopropyl-1-methyl-*N*-((5-(trifluoromethyl)pyrid in-2-yl)methyl)imidazo[1, 5-*a*]quinoxaline-8-formamide | ¹H NMR (400 MHz, DMSO-*d*₆) δ 8.98 (d, *J =* 2.3 Hz, 1H), 8.29 (s, 1H), 8.22 (dd, *J* = 8.4, 2.4 Hz, 1H), 7.84 (s, 1H), 7.68 - 7.57 (m, 2H), 7.47 (d, *J* = 8.3 Hz, 1H), 7.36 (s, 2H), 4.91 (s, 2H), 3.04 (s, 1H), 2.96 (s, 3H), 0.64 - 0.50 (m, 4H). |
| | | | LCMS (ESI) m/z: 441 [M+H]⁺ |
| 5 | 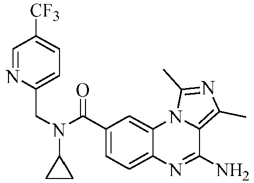 | 4-amino-*N-*cyclopropyl-1,3-dimethyl-*N*-((5-(trifluoromethyl)pyrid in-2-yl)methyl)imidazo[1, 5-*a*]quinoxaline-8-formamide | ¹H NMR (400 MHz, DMSO-*d*₆) δ 8.98 (s, 1H), 8.22 (d, *J* = 10.5 Hz, 2H), 7.64 (t, *J* = 8.9 Hz, 2H), 7.49 (d, *J* = 8.3 Hz, 1H), 7.38 (s, 2H), 4.91 (s, 2H), 3.03 (s, 1H), 2.93 (s, 3H), 2.63 (s, 3H), 0.61 - 0.53 (m, 4H). |
| | | | LCMS (ESI) m/z: 455[M+H]⁺ |
| 6 | 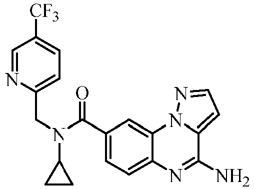 | (4-amino(10-hydropyrazolo[1,5-*a*]quinoxaline-8-yl))-*N*-cyclopropyl-*N*-{[5-(trifluoromethyl)(2-pyridinyl)]methyl}for mamide | ¹H NMR (400 MHz, DMSO-*d₆*): δ *ppm* 8.95 (s, 1H), 8.06 (s, 1H), 8.50-8.40 (m, 1H), 8.20-8.10 (m, 2H), 7.80-7.52 (m, 3H), 7.20 (s, 1H), 4.90 (s, 2H), 3.12-3.09 (m, 1H),0.56-0.51 (m, 4H). |
| | | | LCMS (ESI) *m*/*z:* 427.35 [M+H]⁺ |
| 7 | 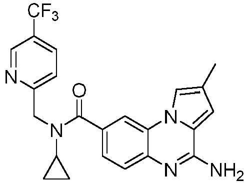 | 4-amino-*N-*cyclopropyl-2-methyl-*N*-((5-(trifluoromethyl)pyrid in-2-yl) methyl)pyrrolo[1,2-*a*]quinoxaline-8-formamide | ¹H NMR (400 MHz, DMSO-*d₆*): δ *ppm* 8.98 (s, 1H), 8.23-8.20 (m, 1H), 8.11 (d, *J* = 16.4 Hz, 2H), 7.64 (d, *J* = 8.4 Hz, 1H), 7.47-7.45 (m, 1H), 7.40-7.38 (m, 1H), 7.01 (s, 2H), 6.88 (s, 1H), 4.88 (s, 2H), 3.14 (br, 1H), 2.28 (s, 3H), 0.53 (s, 4H). ¹⁹F NMR (376 MHz, DMSO) δ-60.67 (s, 3F). |
| | | | LCMS (ESI)m/z: 439.85 [M+H]⁺. |
| 8 | 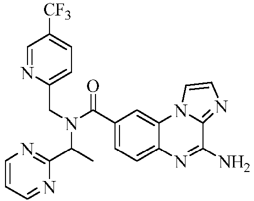 | 4-amino-*N-*cyclopropyl-*N*-(5-trifluoromethylpyridi n-2-yl)methyl)imidazo[1, 5-*a*]quinoxaline-8-formamide | ¹H NMR (400 MHz, CDCl₃) δ 8.71 (d, 1H), 8.22 (s, 1H), 8.02 (d, 1H), 7.84 (d, 1H), 7.75 (m, *2H*), 7.64 (s, 1H), 7.56 - 7.48 (m, 1H), 7.19 (t, 1H), 5.75 (s, 2H), 5.60 (s, 1H), 5.04 (d, 1H), 4.75 (d, 1H), 1.69 (d, 3H). |
| | | | LCMS (ESI) m/z: **493.2** [M+H]⁺ |
| 9 | 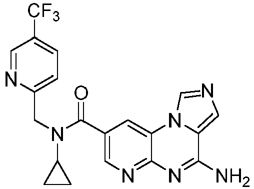 | 4-amino-*N-*cyclopropyl-*N-*((5-(trifluoromethyl)pyrid in-2-yl)methyl)imidazo[1, 5-*a*]pyrido[2,3-*e*]pyrazine-8-formamide | ¹H NMR (400 MHz, DMSO-*d₆*) δ 9.24(s, 1H), 8.99(s, 1H), 8.76-8.72(m, 2H), 8.22-8.20(m, 1H), 7.96(s, 1H), 7.89(s, 2H), 7.66(d, 1H), 4.91(s, 1H), 3.22(m, 1H), 0.60-0.58(m, 4H). |
| | | | LCMS (ESI) m/z: 427.3 [M+H]+ |
| 10 | 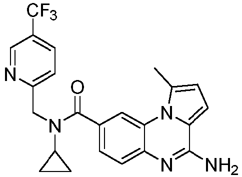 | 4-amino-*N-*cyclopropyl-1-methyl-*N*-((5-(trifluoromethyl)pyridin-2-yl)methyl)pyrrolo[1,2 -*a*]quinoxaline-8-formamide | ¹H NMR (400 MHz, CDCl₃) δ 8.94-8.87 (m, 1H), 8.49 (s, 1H), 7.95 (dd, *J =* 8.1, 2.3 Hz, 1H), 7.64 (d, *J =* 2.3 Hz, 2H), 7.52 (d, *J =* 8.2 Hz, 1H), 6.74 (d, *J* = 4.0 Hz, 1H), 6.54 (d, *J* = 4.0 Hz, 1H), 5.10 (s, 2H), 5.01 (s, 2H), 3.05 (s, 0H), 2.96 (s, 3H), 0.69 (d, *J =* 6.7 Hz, 2H), 0.61 - 0.45 (m, 2H). |
| | | | LCMS (ESI) m/z: **440.2** [M+H]⁺ |
| 11 | 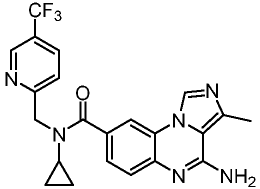 | 4-amino-*N-*cyclopropyl-3-methyl-*N*-((5-(trifluoromethyl)pyrid in-2-yl)methyl)imidazo[1, 5-*a*]quinoxaline-8-formamide | ¹H NMR (400 MHz, DMSO-*d*₆) δ 9.07 (s, 1H), 9.01 - 8.96 (m, 1H), 8.32 (d, *J* = 1.8 Hz, 1H), 8.22 (dd, *J =* 8.3, 2.4 Hz, 1H), 7.65 (d, *J* = 8.3 Hz, 1H), 7.56 (d, *J* = 8.4 Hz, 1H), 7.41 (d, *J =* 8.3 Hz, 1H), 6.90 (s, 2H), 4.89 (s, 2H), 3.14 (s, 1H), 2.65 (s, 3H), 0.56 (t, *J* = 7.2 Hz, 4H). ¹⁹F NMR (376 MHz, DMSO-*d₆*) δ -60.67. |
| | | | LCMS (ESI) m/z: 441 [M+H]⁺ |
| 12 | 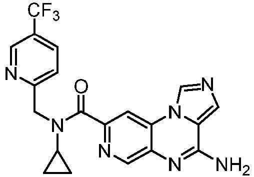 | 4-amino-*N-*cyclopropyl-*N*-((5-(trifluoromethyl)pyrid in-2-yl)methyl)imidazo[1, 5-*a*]pyrido[3,4-*e*]pyrazine-8-formamide | ¹H NMR (400 MHz, DMSO-*d₆*) δ 9.28 (s, 1H), 8.98 (s, 1H), 8.69 (s, 1H), 8.41 (s, 1H), 8.26 (d, *J* = 8.2 Hz, 1H), 7.99 (s, 1H), 7.70 (s, 2H), 7.65 (d, *J*= 8.2 Hz, 1H), 4.92 (s, 2H), 3.15 (s, 1H), 0.49 (s, 4H). ¹⁹F NMR (376 MHz, DMSO-*d₆*) δ -60.65. |
| | | | LCMS (ESI) m/z: 428 [M+H]⁺ |
| 13 | 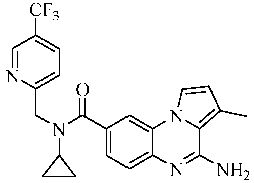 | 4-amino-*N-*cyclopropyl-3-methyl-*N*-((5-(trifluoromethyl)pyrid in-2-yl)methyl)pyrrolo[1,2 -*a*]quinoxaline-8-formamide | ¹H NMR (400 MHz, DMSO-*d*₆) δ 8.99 (d, *J =* 2.3 Hz, 1H), 8.22 (q, *J =* 3.2 Hz, 2H), 8.19 - 8.14 (m, 1H), 7.65 (d, *J =* 8.3 Hz, 1H), 7.47 (d, *J =* 8.3 Hz, 1H), 7.37 (d, *J* = 8.3 Hz, 1H), 6.61 - 6.54 (m, 3H), 4.89 (s, 2H), 3.12 (s, 1H), 2.56 (s, 3H), 0.55 (d, *J* = 7.8 Hz, 4H). |
| | | | ¹⁹F NMR (376 MHz, DMSO-*d*₆) δ -60.67. |
| | | | LCMS (ESI)*m*/*z*: 439.85 [M+H]⁺. |
| 14 | 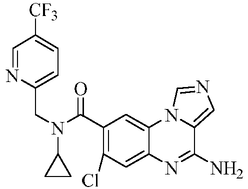 | 4-amino-7-chloro-*N-*cyclopropyl-*N*-((5-(trifluoromethyl)pyrid in-2-yl)methyl)imidazo[1, 5-*a*]quinoxaline-8-formamide | ¹H NMR (400 MHz, DMSO-*d*₆) δ 9.16 (s, 1H), 9.01 (d, *J* = 2.4 Hz, 1H), 8.33 (s, 1H), 8.27 (dd, *J =* 8.3, 2.4 Hz, 1H), 7.95 (s, 1H), 7.69 (d, *J =* 8.3 Hz, 1H), 7.59 (s, 2H), 7.50 (s, 1H), 4.91 (s, 2H), 2.96 - 2.85 (m, 1H), 0.69 - 0.58 (m, 2H), 0.47 (dt, *J* = 6.9, 3.1 Hz, 2H). |
| | | | LCMS (ESI) m/z: **461** [M+H]⁺ |
| 15 | 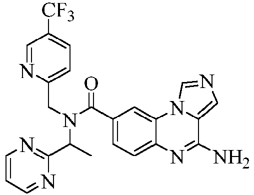 | 4-amino-*N*-(1-(pyrimidin-2-yl)ethyl)-*N*-((5-(trifluoromethyl)pyrid in-2-yl)methyl)imidazo[1, 5-*a*]quinoxaline-8-formamide | ¹H NMR (400 MHz, DMSO-*d*₆) δ 9.15 (s, 1H), 8.85 (s, 1H), 8.79 (d, *J* = 4.9 Hz, 2H), 8.39 (s, 1H), 8.11 (d*, J =* 8.6 Hz, 1H), 7.92 (s, 1H), 7.58 (t, *J* = 9.3 Hz, 2H), 7.52 - 7.34 (m, 4H), 5.44 (d, *J* = 7.6 Hz, 1H), 4.93 (d, *J =* 17.0 Hz, 1H), 4.57 (d, *J* = 16.9 Hz, 1H), 1.63 (d, *J* = 7.0 Hz, 3H). |
| | | | LCMS (ESI) m/z: 493 [M+H]⁺ |
| 16 | 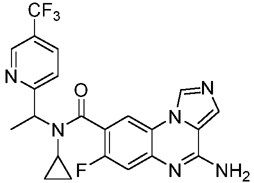 | 4-amino-*N-*cyclopropyl-7-fluoro-*N-*(1-(5-trifluoromethyl)pyridi n-2-yl)ethyl)imidazo[1,5-*a*]quinoxaline-8-formamide | ¹H NMR (400 MHz, DMSO-*d*₆) δ 9.14 (s, 1H), 9.01 - 8.89 (m, 1H), 8.29 (d, *J* = 6.6 Hz, 1H), 8.21 (dd, *J* = 8.4, 2.4 Hz, 1H), 7.93 (s, 1H), 7.76 - 7.45 (m, 3H), 7.21 (d, *J* = 11.2 Hz, 1H), 5.50 (d, *J* = 7.0 Hz, 1H), 2.87 (s, 1H), 1.84 (d, *J* = 7.1 Hz, 3H), 0.66 - 0.11 (m, 4H). |
| | | | LCMS (ESI)m/z: **459.4** [M+H]⁺ |
| 17 | 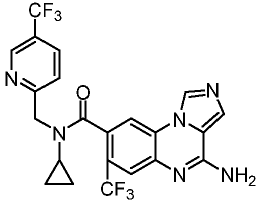 | 4-amino-*N-*cyclopropyl-7-(trifluoromethyl)-*N-*((5-(trifluoromethyl)pyrid in-2-yl)methyl)imidazo[1, 5-*a*]quinoxaline-8-formamide | ¹H NMR (400 MHz, DMSO-*d₆*) δ 9.26 (s, 1H), 9.06 - 8.95 (m, 1H), 8.44 (s, 1H), 8.30 - 8.22 (m, 1H),7.98 (s, 1H), 7.71 (s, 1H), 7.70 - 7.57 (m, 3H), 4.89 (s, 2H), 3.08 - 2.77 (m, 1H), 0.95 - 0.22 (m, 4H). |
| | | | LCMS (ESI) m/z: 495.4 [M+H]⁺ |

### Example 18

### 4-amino-N-cyclopropyl-N-(1-(4-(trifluoromethyl)phenyl)ethyl)imidazol[1,5-a]pyrido[3,4-e]pyrazine-8-formamide

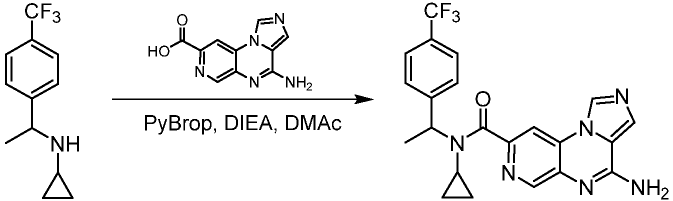

At room temperature, 4-aminoimidazo[1,5-*a*]pyrido[3,4-*e*]pyrazine-8-carboxylic acid (240 mg, 1.047 mmol) was dissolved in *N,N*-dimethylacetamide (5 mL), and then *N-*(1-(4-(trifluoromethyl)phenyl)ethyl)cyclopropanamine (200 mg, 0.8724 mmol), PyBrOP (493 mg, 1.047 mmol), and DIEA (345 mg, 2.617 mmol) were added. The mixture was stirred at 25°C for 2 h. The reaction solution was poured over water (50 mL), and the mixture was extracted with EA (20 mL × 3). The combined organic phases were dried over anhydrous sodium sulfate, and concentrated under reduced pressure to give a crude product, which was then purified by column chromatography (dichloromethane (DCM): methanol = 10:1) to give 4-amino-*N*-cyclopropyl-*N*-(1-(4-(trifluoromethyl)phenyl)ethyl)imidazo[1,5-*a*]pyrido[3,4-*e*]pyrazine-8-formamide (50 mg, yield: 13.01%).

¹H NMR (400 MHz, DMSO-*d₆*) δ 9.28 (s, 1H), 8.66 (s, 1H), 8.42 (s, 1H), 7.99 (s, 1H), 7.74 (d, *J =* 8.2 Hz, 2H), 7.68 (s, 2H), 7.63 (d, *J =* 8.2 Hz, 2H), 5.57 (d, *J* = 7.3 Hz, 1H), 3.17 (d, *J =* 5.2 Hz, 1H), 2.89 (s, 1H), 1.82 (d, *J =* 7.2 Hz, 3H), 0.36 (d, *J =* 42.1 Hz, 3H), 0.12 (dd, *J* = 10.2, 5.8 Hz, 1H).

¹⁹F NMR (376 MHz, DMSO-*d₆*) δ -60.77.

LCMS (ESI) m/z: 441.5 [M+H]⁺

The examples in the following table may be prepared using the synthetic step described in Example 18, only replacing the corresponding starting materials:

| Exa mple No. | Chemical Structure | Chemical Name | ¹HNMR&¹⁹F NMR& LCMS |
|---|---|---|---|
| 19 | 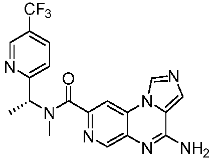 | (*R*)-4-amino-*N*-methyl-*N-*(1-(5-(trifluoromethyl)pyridin-2-yl)ethyl)imidazo[1,5-*a*]pyrido[3,4-*e*]pyrazine-8-formamide | ¹H NMR (400 MHz, DMSO-*d₆*) δ 9.30 (d, *J =* 5.7 Hz, 1H), 9.09 - 8.88 (m, 1H), 8.64 (d, *J* = 19.5 Hz, 1H), 8.47 (s, 1H), 8.25 (ddd, *J =* 15.5, 8.3, 2.4 Hz, 1H), 7.99 (s, 1H), 7.82 - 7.48 (m, 3H), 6.14 - 5.46 (m, 1H), 2.88 (d, *J* = 53.7 Hz, 3H), 1.66 (dd, *J =* 9.0, 6.9 Hz, 3H). |
| | | | LCMS (ESI) m/z: 416.1 [M+H]⁺ |
| 20 | 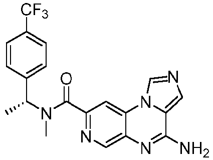 | (*R*)-4-amino-*N*-methyl-*N-*(1-4-(trifluoromethyl)phenyl)eth yl)imidazo[1,5-*a*]pyrido[3,4-*e*]pyrazine-8-formamide | ¹H NMR (400 MHz, DMSO-*d₆*) δ 9.30-9.28 (m,1H), 8.65-8.64(m, 1H), 8.53-8.48 (m, 1H), 7.98 (s, 1H), 7.79 - 7.75 (m, 2H), 7.72-7.71 (m, 2H), 7.68-7.60(m,2H),5.99-5.46 (m, 1H), 2.77-2.70 (m, 3H), 1.66 - 1.58 (m, 3H). |
| | | | LCMS (ESI) *m*/*z*: 415 .20[M+H]⁺ |
| 21 | 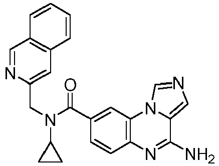 | 4-amino-*N*-cyclopropyl-*N-*(isoquinolin-3-ylmethyl)imidazo[1,5-*a*]quinoxaline-8-formamide | ¹HNMR (400 MHz, DMSO-d*₆*) δ 9.34(s, 1H), 9.19(s, 1H), 8.40(d, 1H), 8.14(d, 1H), 8.02(d, 1H), 7.91(s, 1H), 7.81-7.72(m, 2H), 7.69-7.59(m, 2H), 7.44-7.42(m, 3H), 4.94(s, 2H), 3.09-3.07(m, 1H), 0.58(s, 4H). |
| | | | LCMS (ESI) m/z: 409.2 [M+H]⁺ |
| 22 | 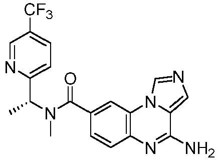 | (*R*)-4-amino-*N*-methyl-*N-*(1-(5-(trifluoromethyl)pyridin-2-yl)ethyl)imidazo[1,5-*a*]quinoxaline-8-formamide | ¹H NMR (400 MHz, DMSO-*d*₆) δ 9.17(s, 1H), 9.06-8.96(m, 1H), 8.31(t, 1H), 8.23(dd, 1H), 7.91(s, 1H), 7.66-7.64(m, 1H), 7.45-7.40(m, 4H), 588-5.86(m, 0.5H), 5.22-5.20(m, 0.5H), 2.90-2.82(m, 3H), 1.67(d, 3H). |
| | | | LCMS (ESI) m/z: 415.2 [M+H]⁺ |
| 23 | 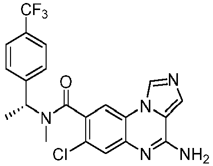 | *N*-{(1*R*)-1-[4-(trifluoromethyl)phenyl]eth yl}(4-amino-7-chloro(10-hydroimidazo[1,5-*a*]quinoxaline-8-yl))-*N-*methylformamide | ¹H NMR (400 MHz, DMSO-*d₆*) δ 9.21-9.10 (m, 1H), 8.43-8.30 (m, 1H), 7.94-7.91 (m, 1H), 7.80-7.71 (m, 2H), 7.70-7.60 (m, 3H), 7.57 - 7.54 (m, 1H), 7.50-7.48 (m, 1H), 6.04-4.83 (m, 1H), 2.56 (s, 3H), 1.65-1.61 (m, 3H). |
| | | | LCMS (ESI):448.10 [M+H]⁺. |
| 24 | 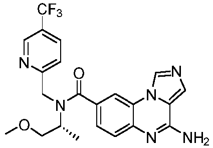 | (*R*)-4-amino-*N*-(1-methoxypropan-2-yl)-*N-*((5-(trifluoromethyl)pyridin-2-yl)methyl)imidazo[1,5-*a*]quinoxaline-8-formamide | ¹H NMR (400 MHz, DMSO-*d*₆) δ 9.18 (s, 1H), 8.93 (s, 1H), 8.28 (s, 1H), 8.19-8.17 (m, 1H), 7.92 (s, 1H), 7.73-7.71 (m, 1H), 7.46-7.43 (m, 4H), 4.84-4.69 (m, 2H), 4.24 (s, 1H), 3.43-3.40(m,1H), 3.26-3.24(m,1H), 3.10 (s, 3H), 1.15-1.13 (m, 3H). |
| | | | LCMS (ESI): 459.30 [M+H]⁺ |
| 25 | 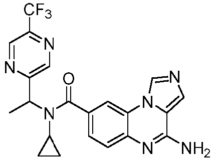 | 4-amino-*N*-cyclopropyl-*N-*(1-(5-(trifluoromethyl)pyrazin-2-ethyl)imidazo[1,5-*a*]quinoxaline-8-formamide | ¹HNMR (400 MHz, DMSO-*d₆*) δ 9.28 - 9.08 (m, 2H), 8.93 (d, *J* = 1.4 Hz, 1H), 8.36 (d, *J* = 1.8 Hz,1H), 7.92 (s, 1H), 7.57 (dd, *J* = 8.4, 1.8 Hz, 1H), 7.44 (t, *J =* 9.5 Hz, 2H), 5.41 (q, *J =* 7.0 Hz, 1H), 3.25-3.15 (m, 1H), 1.89 (d, *J* = 7.1 Hz, 3H), 0.89-0.58 (m, 2H), 0.57-0.38 (m, 3H). |
| | | | ¹⁹F NMR (376 MHz, DMSO-*d₆*) δ -66.02, -69.20, -71.09. |
| | | | LCMS (ESI)m/z: 442.2 [M+H]⁺ |
| 26 | 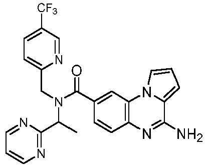 | 4-amino-*N-*(1-(pyrimidin-2-yl)ethyl)-*N*-((5-(trifluoromethyl)pyridin-2-yl)methyl)pyrrolo[1,2-*a*]quinoxaline-8-formamide | ¹H NMR (400 MHz, DMSO-*d*₆) δ 8.97 - 8.90 (m, 1H), 8.85-8.84 (m,2H),8.31-8.01 (m, 3H), 7.66-7.64 (m,1H), 7.52 - 7.44 (m, 3H), 7.17-7.14 (m, 3H), 6.82 (s,1H), 5.75-5.53 (m, 1H), 5.00-4.95 (m, 1H), 4.67-4.63(m,1H), 1.68-1.67 (m, 3H). |
| | | | LCMS (ESI): 492.40 [M+H]⁺ |
| 27 | 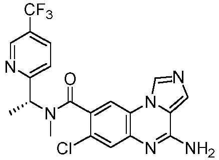 | (*R*)-4-amino-7-chloro-*N-*methyl-*N*-(1-(5-(trifluoromethyl)pyridin-2-yl)ethyl)imidazo[1,5-*a*]quinoxaline-8-formamide | ¹H NMR (400 MHz, DMSO-*d*₆) δ 9.07(s, 1H), 8.95(s, 1H), 8.27-8.18(m, 2H), 7.91(s, 1H), 7.71-7.69(m, 1H), 7.52-7.50(m, 1H), 7.40-7.28(m, 2H), 5.99-4.92(m, 1H), 2.90-2.72(m, 3H), 1.67(d, 3H). |
| | | | LCMS (ESI) m/z:449.2[M+H]⁺ |
| 28 | 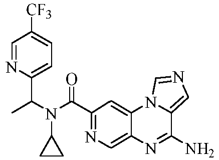 | 4-amino-*N*-cyclopropyl-*N-*(1-(5-(trifluoromethyl)pyridine-2-ethyl)imidazo[1,5-*a]*pyrido[3,4-*e*]pyrazine-8-formamide | ¹H NMR (400 MHz, DMSO-*d₆*) δ 9.31-9.25 (m, 1H), 8.96 (dd, *J* = 2.4, 1.2 Hz, 1H), 8.67 (s, 1H), 8.40 (s, 1H), 8.20 (dd, *J* = 8.6, 2.4 Hz, 1H), 7.99 (d, *J =* 0.7 Hz, 1H), 7.72-7.63 (m, 3H), 5.46 (q, *J =* 7.0 Hz, 1H), 3.06 (s, 1H), 1.85 (d, *J =* 7.1 Hz, 3H), 0.44 (d, *J =* 46.9 Hz, 4H). |
| | | | ¹⁹F NMR (376 MHz, DMSO-*d₆*) δ -60.65. |
| | | | LCMS (ESI) m/z: 442.4 [M+H]⁺ |
| 29 | 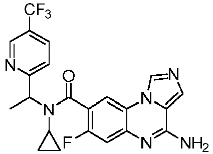 | 4-amino-*N*-cyclopropyl-7-fluoro-*N*-(1-(5-trifluoromethyl)pyridin-2-yl)ethyl)imidazo[1,5-*a*]quinoxaline-8-formamide | ¹H NMR (400 MHz, DMSO-*d₆*) δ 9.13 (s, 1H), 9.04 - 8.93 (m, 1H), 8.29 (d, *J =* 6.6 Hz, 1H), 8.20 (dd, *J =* 8.5, 2.4 Hz, 1H), 7.93 (s, 1H), 7.63 (d, *J =* 8.4 Hz, 1H), 7.58 (s, 2H), 7.22 (d, *J =* 11.2 Hz, 1H), 5.50 (d, *J =* 6.8 Hz, 1H), 2.88 (s, 1H), 1.84 (d, *J* = 7.2 Hz, 3H), 0.55 - 0.32 (m, 4H). |
| | | | ¹⁹F NMR (376 MHz, DMSO-*d₆*) δ -60.65. -118.44. |
| 30 | 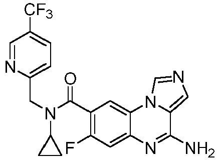 | 4-amino-*N*-cyclopropyl-7-fluoro-*N*-(5-(trifluoromethyl)pyridin-2-yl)methyl)imidazo[1,5-*a*]quinoxaline-8-formamide | ¹H NMR (400 MHz, DMSO-*d₆*) δ 9.14 (s, 1H), 9.00 (s, 1H), 8.29 (dd, *J* = 18.4, 7.4 Hz, 2H), 7.94 (s, 1H), 7.62 (d, *J* = 9.4 Hz, 3H), 7.25 (d, *J* = 11.2 Hz, 1H), 4.91 (s, 2H), 2.95 (s, 1H), 0.54 (dd, *J* = 31.4, 5.4 Hz, 4H). |
| | | | LCMS (ESI) m/z: 445.2 [M+H]⁺ |
| 31 | 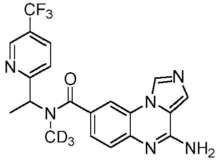 | 4-amino-*N*-methyl-d*₃*-*N*-(1-(5-trifluoromethyl)pyridin-2-yl)ethyl)imidazo[1,5-*a*]quinoxaline-8-formamide | ¹H NMR (400 MHz, DMSO-*d₆*) δ 9.19 (s, 1H), 9.00 (s, 1H), 8.32 (s, 1H), 8.24-8.22 (d, J = 8.4, 2.5 Hz, 1H), 7.94 (s, 1H), 7.66 (s, 1H), 7.52-7.45 (m,4H), 5.88-5.18 (m, 1H), 1.68-1.66 (d, J = 7.0 Hz, 3H). |
| | | | LCMS (ESI): 418.10 [M+H]⁺ |
| 32 | 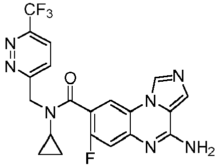 | 4-amino-*N*-cyclopropyl-7-fluoro-*N*-(6-(trifluoromethyl)pyridazine -3-methyl)imidazo[1,5-*a*]quinoxaline-8-formamide | ¹H NMR (400 MHz, DMSO-*d₆*) δ 9.13 (s, 1H), 8.32 (dd, *J* = 7.8, 4.4 Hz, 2H), 8.06 - 7.93 (m, 2H), 7.74 (s, 2H), 7.25 (d, *J =* 11.1 Hz, 1H),5.13 (s, 2H), 3.00 (s, 1H), 0.68 - 0.46 (m, 4H). |
| | | | LCMS (ESI) m/z: 446.2 [M+H]⁺ |
| 33 | 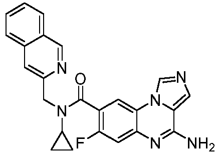 | 4-amino-*N*-cyclopropyl-7-fluoro-*N*-(isoquinolin-3-ylmethyl)imidazo[1,5-*a*]quinoxaline-8-carboxylamide | ¹H NMR (400 MHz, DMSO-*d*₆) δ 9.35 (s, 1H), 9.14 (s, 1H), 8.34 (d, *J* = 6.6 Hz, 1H), 8.15 (d, *J* = 8.2 Hz, 1H), 7.99 (d, *J =* 8.2 Hz, 1H), 7.94 (s, 1H), 7.84 - 7.76 (m, 2H), 7.68 (t, *J =* 7.5 Hz, 1H), 7.58 (s, 2H), 7.25 (d, *J* = 11.3 Hz, 1H), 4.97 (s, 2H), 2.93 (d, *J* = 10.8 Hz, 1H), 0.75 - 0.44 (m, 4H). |
| | | | LCMS (ESI) m/z:427.3[M+H]⁺ |
| 34 | 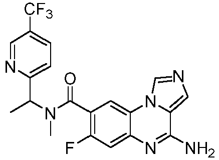 | 4-amino-7-fluoro-N-methyl-*N*-(1-(5-(trifluoromethyl)pyridin-2-yl)ethyl)imidazo[1,5-*a*]quinoxaline-8-formamide | ¹H NMR (400 MHz, DMSO-*d₆*) δ 9.24 - 8.68 (m, 2H), 8.55 - 8.14 (m, 2H), 7.92 (d, 1H), 7.58 (td, 3H), 7.23 (d, 1H), 6.07 - 4.96 (m, 1H), 2.96 - 2.70 (m, 3H), 1.65 (dd, 3H). |
| | | | LCMS (ESI) m/z: 433.20[M+H]⁺ |
| 35 | 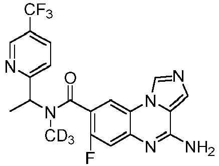 | 4-amino-7-fluoro-*N-*methyl-*d₃*-*N*-(1-(5-trifluoromethyl)pyridin-2-yl)ethyl)imidazo[1,5-*a*]quinoxaline-8-formamide | ¹H NMR (400 MHz, DMSO-*d₆*) δ 9.24 - 8.89 (m, 2H), 8.39 - 8.10 (m, 2H), 7.92 (d, *J* = 3.9 Hz, 1H), 7.59 (td, *J* = 32.7, 31.3, 8.3 Hz, 3H), 7.24 (d, *J* = 11.1 Hz, 1H), 5.50 (m,1 H), 1.65 (dd, *J* = 10.4, 7.0 Hz, 3H). |
| | | | LCMS (ESI) m/z: 436.4 [M+H]⁺ |
| 36 | 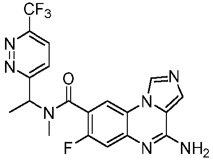 | 4-amino-7-fluoro-*N-*methyl-*N*-(1-(6-(trifluoromethyl)pyridazin-3-yl)ethyl)imidazo[1,5-*a*]quinoxaline-8-formamide | ¹H NMR (400 MHz, DMSO-*d₆*) δ 9.14 (s, 1H), 8.32 (dd, *J =* 11.0, 7.7 Hz, 2H), 8.11 - 7.78 (m, 2H),7.60 (d, *J =* 7.5 Hz, 2H), 7.23 (s, 1H), 5.70 (dd, *J* = 298.6, 7.1 Hz, 1H), 2.90 (s, 3H), 1.76 (dd, *J* = 18.8,7.0 Hz, 3H). |
| | | | LCMS (ESI) m/z:434.2[M+H]⁺ |
| 37 | 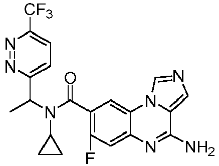 | 4-amino-*N*-cyclopropyl-7-fluoro-*N*-(1-(6-trifluoromethyl)pyridazin-3-yl)ethyl)imidazo[1,5-*a*]quinoxaline-8-formamide | ¹H NMR (400 MHz, DMSO-*d₆*) δ 9.12 (d, *J =* 1.8 Hz, 1H), 8.34 - 8.19 (m, 2H), 8.08 - 7.84 (m, 2H), 7.59 (s, 2H), 7.22 (dd, *J* = 11.2, 1.8 Hz, 1H), 5.59 (d, *J =* 7.4 Hz, 1H), 2.98 (s, 1H), 1.94 (d, *J= 6.9* Hz, 3H), 0.69 - 0.36 (m, 4H). |
| | | | LCMS (ESI)m/z: 460.4 [M+H]⁺ |
| 38 | 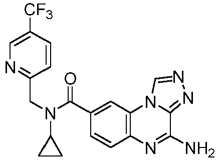 | 4-amino-N-cyclopropyl-N-((5-(trifluoromethyl)pyridin-2-yl)methyl)-[1,2,4]triazolo[4,3-*a*]quinoxaline-8-formamide | ¹H NMR (400 MHz, DMSO-*d₆*) δ 10.06 (s, 1H), 8.99 (t, *J* = 1.6 Hz, 1H), 8.48 - 8.32 (m, 1H), 8.22(dd, *J* = 8.2, 2.4 Hz, 1H), 7.86 (s, 2H), 7.67 (dd, *J* = 24.9, 8.3 Hz, 2H), 7.57 (d, *J* = 8.3 Hz, 1H), 4.90 (s, 2H), 3.20 - 3.03 (m, 1H), 0.55 (s, 4H). |
| | | | LCMS (ESI) m/z: 428.30 [M+H]⁺ |
| 39 | 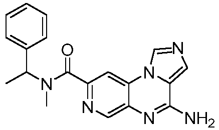 | 4-amino-*N*-methyl-*N*-(1-phenylethyl)imidazo[3,4-e]pyrazine-8-formamide | ¹H NMR (400 MHz, DMSO-*d₆*) δ 9.30 (d, *J= 7.5* Hz, 1H), 8.66 (d, *J* = 5.6 Hz, 1H), 8.50 (d, *J* = 21.6 Hz, 1H), 8.00 (s, 1H), 7.79 - 7.66 (m, 2H), 7.56 - 7.53 (m, 2H), 7.45 - 7.39 (m, 3H), 6.01 - 5.28 (m, 1H), 2.70 (d, *J=* 15.9 Hz, 3H), 1.63 - 1.57 (m, 3H). LCMS (ESI) m/z: 347.4 [M+H]⁺ |
| 40 | 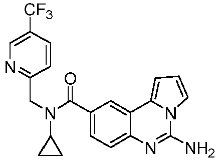 | 5-amino-N-cyclopropyl-N-((5-(trifluoromethyl)pyridin-2-yl)methyl)pyrroloindole[1,2 -c]quinoxaline-8-formamide | ¹H NMR (400 MHz, DMSO-*d₆*) δ 9.01 - 8.94 (m, 1H), 8.21 (dd, *J* = 8.4, 2.4 Hz, 1H), 8.15 (s, 1H), 7.75 (dd, *J* = 3.2, 1.2 Hz, 1H), 7.63 (d, *J* = 8.4 Hz, 1H), 7.55 (d, *J=* 8.4 Hz, 1H), 7.47 (s, 2H), 7.35 (d, *J* = 8.4 Hz, 1H), 7.08 (d, *J* = 3.6 Hz, 1H), 6.79 (m, 1H), 4.88 (s, 2H), 3.08 (s, 1H), 0.72 - 0.35 (m, 4H) |
| | | | LCMS (ESI) m/z: 426.2 [M+H]⁺ |
| 41 | 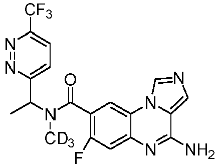 | 4-amino-7-fluoro-N-(methyl-d₃)-*N*-(1-(6-(trifluoromethyl)pyridazin-3-yl)ethyl)imidazo[1,5-*a*]quinoxaline-8-carboxylamide | ¹H NMR (400 MHz, DMSO-*d₆*) δ 9.14 (s, 1H), 8.31 (dd*, J =* 10.9, 7.7 Hz, 2H), 8.08 - 7.83 (m, 2H),7.59 (d, *J* = 7.6 Hz, 2H), 7.24 (d, *J=* 11.1 Hz, 1H), 6.16 - 5.20 (m, 1H), 1.75 (dd, *J=* 18.9, 7.0 Hz, 3H). |
| | | | LCMS (ESI) m/z: 437.3 [M+H]⁺ |
| 42 | 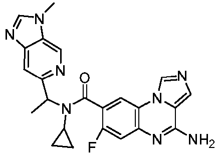 | 4-amino-N-cyclopropyl-7-fluoro-N-(1-(3-methyl-3H-imidazo[4,5-c]pyridin-6-yl)ethyl)imidazo[1,5-*a*]quinoxaline-8-formamide | ¹H NMR (400 MHz, DMSO-*d₆*) δ 9.17 (s, 1H), 8.95 (s, 1H), 8.53 -8.26 (m, 2H), 7.93 (s, 1H), 7.61 (d, *J= 43.4* Hz, 3H), 7.21 (d, *J=* 11.2 Hz, 1H), 5.72 (d, *J* = 8.2 Hz, 1H), 3.96 (s, 3H), 2.74 (s, 1H), 1.87 (d, *J* = 7.1 Hz, 3H), 0.41 (s, 2H), 0.30 (s, 2H). |
| | | | LCMS (ESI) m/z: 445.2 [M+H]⁺ |
| 43 | 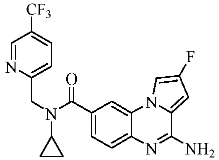 | 4-amino-2-fluoro(10-hydropyrrolo[1,2-*a*]quinoxaline-8-yl)-*N-*cyclopropyl-*N*-{[5-trifluoromethyl]methyl} for mamide | ¹H NMR (400 MHz, DMSO-*d₆*): δ *ppm* 8.98 (s, 1H), 8.14 (s, 1H), 8.23-8.17 (m, 2H), 7.64 (d, *J =* 7.6 Hz, 1H), 7.52 (d, *J* = 7.2 Hz, 1H), 7.42 (d, *J* = 8.0 Hz, 1H), 7.15 (s, 2H), 6.95 (s, 1H), 4.88 (s, 2H), 3.15 (br, 1H), 0.53 (s, 4H). |
| | | | LCMS (ESI) m/z: 443.75[M+H]⁺ |
| 44 | 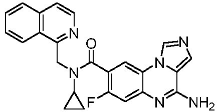 | 4-amino-N-cyclopropyl-7-fluoro-N-(isoquinolin-1-ylmethyl)imidazo[1,5-*a*]quinoxaline-8-carboxylamide | ¹H NMR (400 MHz, DMSO-*d₆*) δ 9.14 (s, 1H), 8.56 (d, *J= 5.7* Hz, 1H), 8.33 (dd, *J=* 23.2, 7.4 Hz, 2H), 8.04 (d, *J= 8.2* Hz, 1H), 7.94 (s, 1H), 7.87 - 7.69 (m, 3H), 7.59 (s, 2H), 7.21 (d, *J* = 11.1 Hz, 1H),5.38 (s, 2H), 2.84 (dt, *J* = 7.3, 3.4 Hz, 1H), 0.91 - 0.34 (m, 4H). |
| | | | LCMS (ESI) m/z: 427.3 [M+H]⁺ |
| 45 | 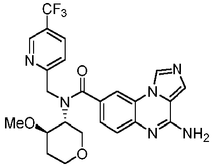 | 4-amino-7-fluoro-*N-*(3*R,*4*R*)-4-methoxytetrahydro-2*H-*pyran-3-yl)-*N*-(5-trifluoromethyl)pyridin-2-ylmethyl)imidazo[1,5-*a*]quinoxaline-8-formamide | ¹H NMR (400 MHz, DMSO-*d₆*) δ 9.18 (s, 1H), 8.99 - 8.78 (m, 1H), 8.29 - 8.19 (m, 1H), 8.05 - 7.80 (m, 2H), 7.76 - 7.46 (m, 3H), 7.31 - 7.04 (m, 1H), 5.00 - 4.71 (m, 1H), 4.07 - 3.67 (m, 1H), 3.65 - 3.41 (m, 2H), 3.22 (d, *J* = 52.5 Hz, 4H), 2.14 (dd, *J* = 56.3, 12.5 Hz, 1H), 1.31 - 1.21 (m, 3H), 1.10 (s, 1H). |
| | | | LCMS (ESI) m/z: 518.5 [M+H]⁺ |
| 46 | 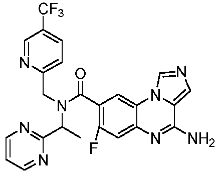 | 4-amino-7-fluoro-*N*-(1-(pyrimidin-2-yl)ethyl)-N-(5-(trifluoromethyl)pyridin-2-yl)methyl)imidazo[1,5-*a*]quinoxaline-8-formamide | ¹H NMR (400 MHz, DMSO-*d₆*) δ 9.13 (s, 1H), 8.95 - 8.86 (m, 1H), 8.78 (dd, *J* = 14.6, 4.9 Hz, 2H), 8.36 - 8.18 (m, 1H), 8.08 - 7.81 (m, 2H), 7.56 (dd, *J* = 22.0, 13.6 Hz, 3H), 7.40 (q, *J* = 5.2, 4.3 Hz, 1H), 7.31 - 7.03 (m, 1H), 5.98 - 5.22 (m, 1H), 5.13 - 4.58 (m, 2H), 1.63 (dd, *J* = 13.8, 7.1 Hz, 3H). |
| | | | LCMS (ESI) m/z: 511.2 [M+H]⁺ |
| 98 | 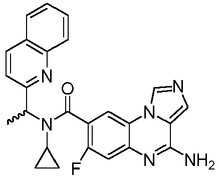 | 4-amino-*N*-cyclopropyl-7-fluoro-*N*-(1-(quinolin-2-yl)ethyl)imidazo[1,5-*a*]quinoxaline-8-carboxylamide | ¹H NMR (400 MHz, DMSO-*d₆*) δ 9.15 (s, 1H), 8.47 - 8.17 (m, 2H), 7.99 (dd, *J* = 12.4, 8.2 Hz, 2H),7.92 (s, 1H), 7.77 (ddd, *J =* 8.4, 6.8, 1.5 Hz, 1H), 7.72 - 7.51 (m, 4H), 7.23 (d, *J* = 11.2 Hz, 1H), 5.75(d, *J* = 7.6 Hz, 1H), 2.84 (s, 1H), 1.91 (d, *J =* 7.1 Hz, 3H), 0.58 - 0.20 (m, 4H). |
| | | | LCMS (ESI) m/z: 441.30 [M+H]+ |

### Example 47 (R)-4-amino-7-fluoro-N-(1-(5-fluoropyridin-2-yl)ethyl)-N-methylimidazo[1,5-a]quinoxaline-8-formamide

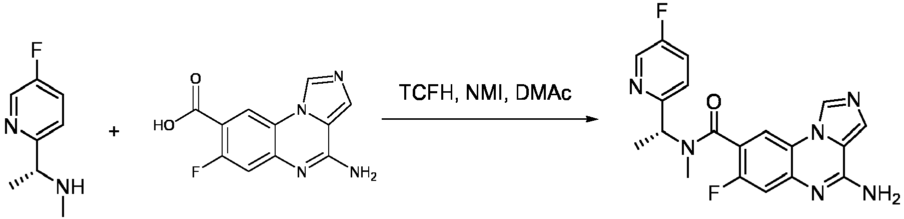

4-amino-7-fluoroimidazo[1,5-*a*]quinoxaline-8-carboxylic acid (287.41 mg, 1.17 mmol), TCFH (491.32 mg, 1.75 mmol), NMI (191.71 mg, 2.33 mmol) were dissolved in dimethylacetamide (3 mL), and then (*R*)-1-(5-fluoropyridin-2-yl)-*N*-methylethyl-1-amine (180 mg, 1.17 mmol) was added. The mixture was stirred at room temperature for 2 h. After the reaction completed, the system was cooled to room temperature and extracted with EA for 3 times and 10 mL for each time. The combined organic phases were washed with saturated brine solution (10 mL × 3), dried over anhydrous sodium sulfate, and concentrated under reduced pressure. The crude product was purified by HPLC (column: Kinetex 5 m EVO C18, 30 mm × 150 mm; mobile phase A: water (10 mmol/L ammonium bicarbonate), mobile phase B: acetonitrile (ACN); flow rate: 60 mL/min; gradient: 20% B to 47% B in 10 min; wavelength: 254/220 nm; retention time (min): 9.35). (*R*)-4-amino-7-fluoro-*N*-(1-(5-fluoropyridin-2-yl)ethyl)-*N*-methylimidazo[1,5-*a*]quinoxaline-8-formamide (17.9 mg, 1.17 mmol, yield: 3.84%) was obtained as a white solid.

¹H NMR (400 MHz, DMSO-*d₆*) δ 9.35 (s, 1H), 9.13 (s, 1H), 8.34-8.32 (m, 1H), 8.15-8.13 (m, 1H), 8.05-8.04(m,1H),7.85 - 7.65 (m, 2H),7.63 - 7.45 (m, 2H), 7.34 (s,2H),7.23-7.22 (m 1H), 4.96 (S,2H),2.92 - 2.90(m,1H), 0.60-0.49 (M, 4H).

LCMS (ESI): 426.85 [M+H]⁺

The examples in the following table may be prepared using the synthetic step described in **Example 47,** only replacing the corresponding starting materials:

| Example No. | Chemical Structure | Chemical Name | ¹HNMR&¹⁹F NMR& LCMS |
|---|---|---|---|
| 48 | 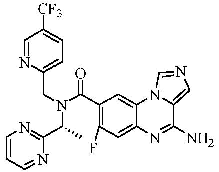 | (*R*)-4-amino-7-fluoro-*N*-(1-(pyrimidin-2-yl)ethyl)-*N*-((5-(trifluoromethyl)pyrid in-2-yl)methyl)imidazo[1, 5-*a*]quinoxaline-8-formamide | ¹H NMR (400 MHz, DMSO-*d₆*) δ 9.12-9.11 (m, 1H), 8.93-8.96 (m, 1H), 8.82-8.75 (m, 2H), 8.31-8.17 (m, 2H), 7.92 (s, 1H), 7.59 - 7.49 (m, 3H), 7.39 - 7.25 (m, 2H), 5.22 (q, *J* = 6.9 Hz, 1H), 5.05 (d, *J* = 17.0 Hz, 1H), 4.80-4.67 (m, 1H), 1.65-1.60 (m, 3H). |
| | | | LCMS (ESI) m/z: 511.10 [M+H]⁺ |
| 49 | 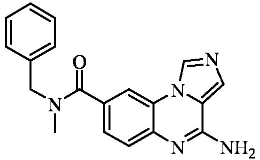 | 4-amino-*N*-benzyl-*N-*methylimidazo[1,5-*a*]quinoxaline-8-formamide | ¹H NMR (400 MHz, DMSO-*d₆*) δ 9.16 (s, 1H), 8.26 (s, 1H), 7.90 (s, 1H), 7.43-7.42 (m, 3H), 7.38-7.36(m, 1H),3.01(s, 6H). |
| | | | LCMS (ESI): 332.30 [M+H]⁺ |
| 50 | 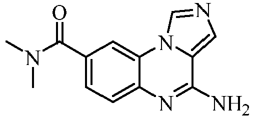 | 4-amino*-N,N-*dimethylimidazo[1,5-*a*]quinoxaline-8-formamide | ¹H NMR (400 MHz, DMSO-*d₆*) δ 9.16 (s, 1H), 8.26 (s, 1H), 7.90 (s, 1H), 7.45-7.43 (d, 3H), 7.39-7.36 (m, 1H), 3.01 (s, 6H). |
| | | | LCMS (ESI): 256.30 [M+H]⁺ |
| 51 | 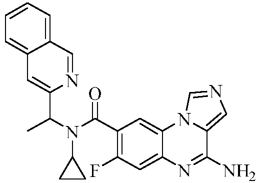 | 4-amino-*N-*cyclopropyl-7-fluoro-*N*-(1-(isoquinolin-3-yl)ethyl)imidazo[1,5-*a*]quinoxaline-8-carboxylamide | ¹H NMR (400 MHz, DMSO-*d₆*) δ 9.34 (s, 1H), 9.15 (s, 1H), 8.30 (d, *J* = 6.5 Hz, 1H), 8.13 (d, *J* = 8.2 Hz, 1H), 8.00 (d, *J* = 8.3 Hz, 1H), 7.92 (s, 1H), 7.85 - 7.74 (m, 2H), 7.68 (t, *J* = 7.5 Hz, 1H), 7.55 (s, 2H), 7.21 (d, *J* = 11.2 Hz, 1H), 5.70 (s, 1H), 2.80 (s, 1H), 1.90 (d, *J* = 7.2 Hz, 3H), 0.41 (d, *J* = 18.5 Hz, 4H). |
| | | | LCMS (ESI) m/z: 441.20 [M+H]⁺ |
| 52 | 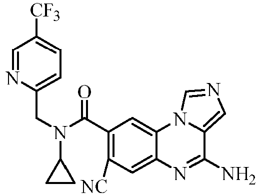 | 4-amino-7-cyano-*N-*cyclopropyl-*N*-(5-trifluoromethylpyridi n-2-yl)methyl)imidazo[1, 5-*a*]quinoxaline-8-formamide | ¹H NMR (400 MHz, DMSO-*d₆*) δ 9.33 (s, 1H), 9.06 (s, 1H), 8.56 (s, 1H), 8.30 - 8.29 (m, 1H), 8.05 (s, 1H), 7.91 (s, 1H), 7.82- 7.78 (m, 2H), 7.76- 7.66 (m, 1H), 5.01 (s, 2H), 3.23 (s, 1H) 0.87 -0.65 (m, 4H). |
| | | | LCMS (ESI) m/z: 452.15[M+H]⁺ |
| 53 | 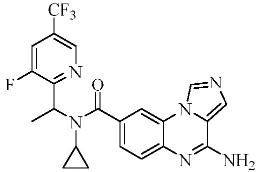 | 4-amino-*N-*cyclopropyl-*N*-(1-(3-fluoro-5-(trifluoromethyl)pyrid in-2-yl)ethyl)imidazo[1,5-*a*]quinoxaline-8-formamide | ¹H NMR (400 MHz, Methanol-*d₄*) δ 8.96 (s, 1H), 8.61 (s, 1H), 8.15-8.14(m, 1H), 7.89 - 7.86 (m, 1H), 7.83 (s, 1H), 7.52 - 7.49 (m, 1H), 7.44 - 7.42 (m, 1H), 5.72 - 5.57 (m, 1H), 4.55 (s, 2H),2.91 (s, 1H), 1.80 - 1.77 (m, 3H), 0.88 - 0.83 (m, 1H), 0.61 - 0.55 (m, 1H), 0.51 - 0.46 (m, 2H). |
| | | | LCMS (ESI) m/z: 459.35[M+H]⁺ |
| 54 | 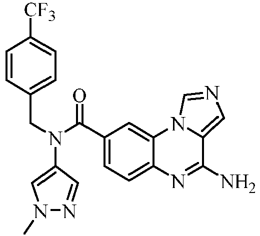 | 4-amino-*N*-(1-methyl-1*H*-pyrazol-4-yl)-*N-*(4-(trifluoromethyl)benz yl)imidazo[1,5-*a*]quinoxaline-8-formamide | ¹H NMR (400 MHz, DMSO-*d₆*) δ 9.09 (s, 1H), 8.27 (s, 1H), 7.89 (d, *J* = 0.7 Hz, 1H), 7.72 (d, *J* = 8.1 Hz, 2H), 7.56-7.54 (m, 3H), 7.45 (s, 2H), 7.27 (s, 2H), 7.19-7.07 (m, 1H), 5.07 (s, 2H), 3.62 (s, 3H). |
| | | | LCMS (ESI): 466.10 [M+H]⁺ |
| 55 | 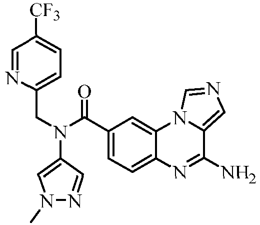 | 4-amino-N-(1-methyl-1H-pyrazol-4-yl)-N-((5- (trifluoromethyl) pyridin-2-yl) methyl)imidazo[1,5-*a*]quinoxaline-8-formamide | ¹H NMR (400 MHz, DMSO-*d₆*) δ 9.09 (s, 1H), 8.96 (d, *J= 2.0* Hz, 1H), 8.28 (s, 1H), 8.20 (d, *J* = 8.2 Hz, 1H), 7.89 (s, 1H), 7.67 (s, 2H), 7.45 (s, 2H), 7.28 (s, 3H), 5.15 (s, 2H), 3.63 (s, 3H). |
| | | | LCMS (ESI): 467 [M+H]⁺ |
| 56 | 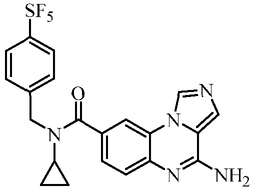 | 4-amino-*N-*cyclopropyl-*N*-(4-(pentafluoro-16-sulfonamido)benzyl)i midazo[1,5-*a*]quinoxaline-8-formamide | ¹H NMR (400 MHz, DMSO-*d₆*) δ 9.20 (s, 1H), 8.38 (d, J = 1.8 Hz, 1H), 7.96 - 7.87 (m, 3H), 7.58 (dd, J = 15.7, 7.9 Hz, 3H), 7.48 - 7.38 (m, 3H), 4.80 (s, 2H), 3.03 (s, 1H), 0.59 - 0.44 (m, 4H). |
| | | | LCMS (ESI): 483 [M+H]⁺ |
| 57 | 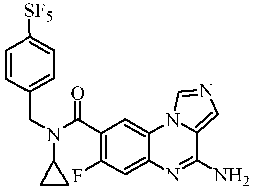 | 4-amino-*N-*cyclopropyl-7-fluoro-*N*-(4-(pentafluoro-16-sulfonamido)benzyl)i midazo[1,5-*a*]quinoxaline-8-formamide | ¹H NMR (400 MHz, DMSO-*d₆*) δ 9.12 (s, 1H), 8.31 (d, *J =* 6.6 Hz, 1H), 8.00 - 7.79 (m, 3H), 7.60 (d, *J* = 10.0 Hz, 4H), 7.23 (d, *J* = 11.3 Hz, 1H), 4.83 (s, 2H), 2.76 (s, 1H), 0.59 - 0.41 (m, 4H). |
| | | | LCMS (ESI): 501 [M+H]⁺ |
| 58 | 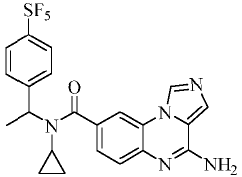 | 4-amino-*N-*cyclopropyl-*N*-(1-(4-(pentafluoro-16-sulfonamido)phenyl)e thyl)imidazo[1,5-*a*]quinoxaline-8-formamide | ¹H NMR (400 MHz, DMSO-*d₆*) δ 9.20 (d, *J* = 0.7 Hz, 1H), 8.37 (d, *J* = 1.8 Hz, 1H), 7.95 - 7.82 (m, 3H), 7.65 - 7.52 (m, 2H), 7.56-7.54 (m, 1H), 7.44-7.42(m,3H), 5.51-5.47 (m, 1H), 2.98-2.76(m, 1H), 1.84-1.82 (m, 3H), 0.53 - 0.35 (m, 2H), 0.34 - 0.27 (m, 1H), 0.17 - 0.12 (m, 1H). |
| | | | LCMS (ESI): 497 [M+H]⁺ |
| 59 | 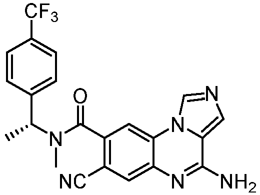 | (*R*)-4-amino-7-cyano-*N*-methyl-*N*-(1-(4-(trifluoromethyl)phen yl)ethyl)imidazo[1,5-*a*]quinoxaline-8-formamide | ¹H NMR (400 MHz, DMSO-*d₆*, *ppm*) δ 9.24-9.21 (m, 1H), 8.50-8.42 (m, 1H),7.96 (s, 1H), 7.89 (s, 1H), 7.78-7.67 (m, 5H), 7.58-7.54 (m, 1H), 6.02-6.01 (m, 1H), 2.82-2.63 (m, 3H), 1.67 - 1.65 (m, 3H) |
| | | | LCMS (ESI) m/z: 438.80[M+H]⁺ |
| 60 | 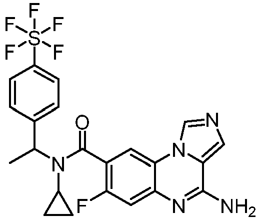 | 4-amino-*N-*cyclopropyl-7-fluoro-*N*-(1-(4-(pentafluoro-16-sulfonamido)phenyl)e thyl)imidazo[1,5-*a*]quinoxaline-8-formamide | ¹H NMR (400 MHz, DMSO-*d₆*) δ 9.18 (s, 1H), 8.36-8.34 (m, 1H), 8.09 - 7.81 (m, 5H), 7.60 (d, *J* = 8.4 Hz, 2H), 7.27-7.22(m, 1H), 2.94 (s, 1H), 2.78 (s, 1H), 1.81-1.79 (m, 3H), 0.46-0.19 (m, 4H). |
| | | | LCMS (ESI): 515.75 [M+H]⁺ |
| 100 | 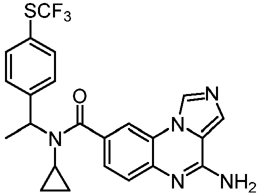 | (4-amino(10-hydroimidazo[1,5-*a*]quinoxalin-8-yl)-*N-*cyclopropyl-*N*-{[4-(trifluoromethylthio)p henyl]ethyl } formamid e | ¹H NMR (400 MHz, DMSO-*d₆*) δ 9.20 (s, 1H), 8.37 (d, *J* = 1.8 Hz, 1H), 7.91 (s, 1H), 7.71 (d, *J* = 8.2 Hz, 2H), 7.56 (d, *J* = 8.2 Hz, 3H), 7.45 - 7.28 (m, 3H), 5.54-5.51 (m, 1H), 2.94 (s, 1H), 1.83 (d, *J* = 7.2 Hz, 3H), 0.50 - 0.33 (m, 2H), 0.27 (s, 1H), 0.12-0.08 (m, 1H). |
| | | | LCMS (ESI): 471.80 [M+H]⁺ |
| 101 | 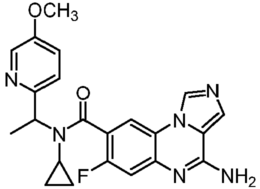 | 4-amino-*N-*cyclopropyl-7-fluoro-*N-*(1-(5-methoxypyridin-2-yl)ethyl)imidazo[1,5-*a*]quinoxaline-8-formamide | ¹H NMR (400 MHz, DMSO-*d₆*) δ 9.13 (s, 1H), 8.34 - 8.15 (m, 2H), 7.91 (s, 1H), 7.55 (s, 2H), 7.38-7.36 (m, 2H), 7.20 (d, *J* = 11.2 Hz, 1H), 5.57 (s, 1H), 3.84 (s, 3H), 2.77 - 2.62 (m, 1H), 1.76 (d, *J =* 7.1 Hz, 3H), 0.43 - 0.03 (m, 4H). |
| | | | LCMS (ESI): 420.80 [M+H]⁺ |
| 102 | 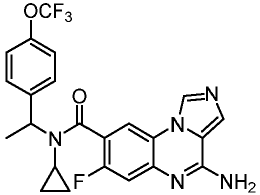 | 4-amino-*N-*cyclopropyl-7-fluoro-*N*-(1-(4-(trifluoromethoxy)phe nyl)ethyl)imidazo[1,5 -*a*]quinoxaline-8-formamide | ¹H NMR (400 MHz, DMSO-*d₆*) δ 9.13 (s, 1H), 8.32 (d, *J* = 6.8 Hz, 1H), 7.92 (s, 1H), 7.56 (s, 2H), 7.51-7.49 (m, 2H), 7.38-7.36 (m, 2H), 7.20 (d, *J* = 11.2 Hz, 1H), 5.62 (s, 1H), 1.78 (d, *J* = 7.2 Hz, 3H), 0.41-0.14 (s, 4H). |
| | | | LCMS (ESI): 474.45 [M+H]⁺ |
| 103 | 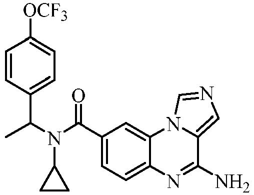 | 4-amino-*N-*cyclopropyl-*N*-(1-(4-(trifluoromethoxy)phe nyl)ethyl)imidazo[1,5 -*a*]quinoxaline-8-formamide | ¹H NMR (400 MHz, DMSO-*d₆*) δ 9.20 (s, 1H), 8.36 (d, *J* = 1.8 Hz, 1H), 8.05 - 8.01 (m, 1H), 7.84-7.82 (m, 1H), 7.58 - 7.50 (m, 3H), 7.46 - 7.39 (m, 2H), 7.38 - 7.32 (m, 2H), 5.58-5.51(m,1H), 2.89 (s, 1H), 1.81 (d, *J* = 7.2 Hz, 3H), 0.49 - 0.33 (m, 2H), 0.30-0.22 (s, 1H), 0.13 - 0.06 (m, 1H). |
| | | | LCMS (ESI): 456.45 [M+H]⁺ |
| 104 | 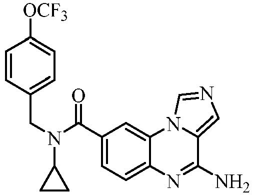 | 4-amino-*N-*cyclopropyl-*N*-(4-(trifluoromethoxy)ben zyl)imidazo[1,5-*a*]quinoxaline-8-formamide | ¹H NMR (400 MHz, DMSO-*d₆*) δ 8.44 (s, 1H), 7.66 (d, *J* = 1.8 Hz, 1H), 7.30 (s, 1H), 7.01 - 6.84 (m, 5H), 6.66-6.60 (m, 3H), 5.01-4.89 (s, 2H), 2.32 (s, 1H), 0.22-0.07 (m,4H). |
| | | | LCMS (ESI): 442.05 [M+H]⁺ |
| 105 | 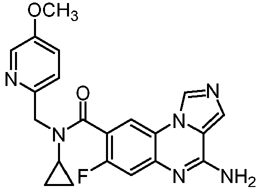 | 4-amino-*N-*cyclopropyl-7-fluoro-*N*-((5-methoxypyridin-2-yl)methyl)imidazo[1, 5-*a*]quinoxaline-8-formamide | ¹H NMR (400 MHz, Methanol-*d₄*) δ 9.00 (s, 1H), 8.26 - 8.14 (m, 2H), 7.93 (s, 1H), 7.45 (s, 2H), 7.25 (d, *J* = 11.2 Hz, 1H), 4.57(s,2H),3.90 (s, 3H), 2.92 (s, 1H), 0.68 - 0.43 (m, 4H). |
| | | | LCMS (ESI): 406 .42[M+H]⁺ |
| 106 | 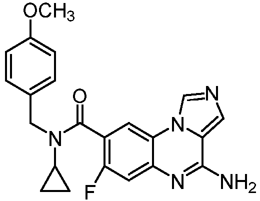 | 4-amino-*N-*cyclopropyl-7-fluoro-*N*-(4-methoxybenzyl)imida zo[1,5-*a*]quinoxaline-8-formamide | ¹H NMR (400 MHz, Methanol-*d₄*) δ 8.92 (s, 1H), 8.07 (d, *J* = 6.3 Hz, 1H), 7.83 (s, 1H), 7.26 (d, *J* = 8.2 Hz, 2H), 7.13 (d, *J* = 11.1 Hz, 1H), 6.84 (d, *J =* 8.2 Hz, 2H), 4.67 (s, 2H), 3.71 (s, 3H), 2.66 (s, 1H), 0.50 - 0.36 (m, 4H). |
| | | | LCMS (ESI): 405.43 [M+H]⁺ |
| 107 | 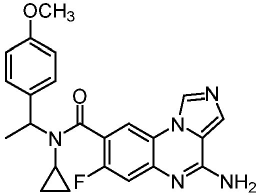 | 4-amino-*N-*cyclopropyl-7-fluoro-*N*-(1-(4-methoxyphenyl)ethyl) imidazo[1,5-*a*]quinoxaline-8-formamide | ¹H NMR (400 MHz, Methanol-*d*₄) δ 9.05 (s, 1H), 8.23 (d, *J* = 6.3 Hz, 1H), 7.95 (s, 1H), 7.40 (d, *J =* 8.1 Hz, 2H), 7.25 (d, *J =* 11.1 Hz, 1H), 6.94 (d, *J* = 8.3 Hz, 2H), 5.82 (s, 1H), 3.82 (s, 3H), 2.68 (s, 1H), 1.83 (d, *J* = 7.1 Hz, 3H), 0.33 (d, *J =* 56.4 Hz, 4H). |
| | | | LCMS (ESI): 419.45[M+H]⁺ |
| | | | |
| 108 | 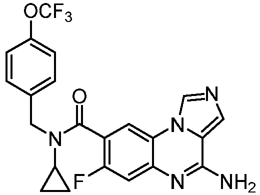 | 4-amino-*N-*cyclopropyl-7-fluoro-*N-*(4-(trifluoromethoxy)ben zyl)imidazo[1,5-*a*]quinoxaline-8-formamide | ¹H NMR (400 MHz, DMSO-*d₆*) δ 9.12 (s, 1H), 8.30 (d, *J* = 6.6 Hz, 1H), 7.92 (s, 1H), 7.57 (s, 2H), 7.50 (d, *J* = 8.3 Hz, 2H), 7.40 (d, *J* = 8.2 Hz, 2H), 7.22 (d, *J* = 11.3 Hz, 1H), 4.77 (s, 2H), 2.72 (s, 1H), 0.49 (d, *J* = 12.9 Hz, 4H). |
| | | | LCMS (ESI): 460.05 [M+H]⁺ |
| 109 | 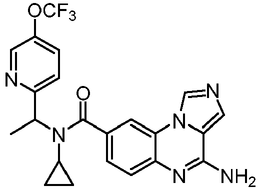 | 4-amino-*N-*cyclopropyl-*N*-(1-(5-(trifluoromethyl)pyrid in-2-yl)ethyl)imidazo[1,5-*a*]quinoxaline-8-formamide | ¹H NMR (400 MHz, Methanol-*d₄*) δ 9.06 (s, 1H), 8.53 (d, *J* = 2.7 Hz, 1H), 8.32 (d, *J* = 1.8 Hz, 1H), 7.94 (s, 1H), 7.75 (dd, *J* = 8.7, 2.8 Hz, 1H), 7.67 - 7.59 (m, 2H), 7.54 (d, *J* = 8.4 Hz, 1H), 5.52 (q, *J* = 7.1 Hz, 1H), 3.03 (p, *J* = 5.4 Hz, 1H), 1.92 (d, *J* = 7.2 Hz, 3H), 0.65 - 0.40 (m, 4H). |
| | | | LCMS (ESI): 457.05 [M+H]⁺. |
| 110 | 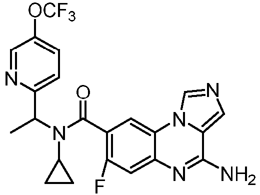 | 4-amino-*N-*cyclopropyl-7-fluoro-*N*-(1-(5-(trifluoromethoxy)pyr idin-2-yl)ethyl)imidazo[1,5-*a*]quinoxaline-8-formamide | ¹H NMR (400 MHz, DMSO-*d₆*) δ 9.13 (s, 1H), 8.66 (d, *J* = 2.8 Hz, 1H), 8.29 (d, *J* = 6.6 Hz, 1H), 7.91-7.89 (m, 2H), 7.57 - 7.53 (m, 3H), 7.212 (d, *J =* 11.2 Hz, 1H), 5.52-5.50 (m, 1H), 2.90-2.82 (s, 1H), 1.85 (d, *J =* 7.2 Hz, 3H), 0.48 - 0.14 (s, 4H). LCMS (ESI): 475.10 [M+H]⁺. |

### Example 6 Method II: (4-amino(10-hydropyrazolo[1,5-a]quinoxaline-8-yl))-N-cyclopropyl-N-{[5-(trifluoromethyl)(2-pyridinyl)]methyl}formamide

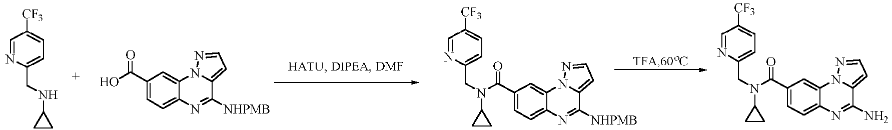

Step I: *N*-cyclopropyl(4-{[(4-methoxyphenyl)methyl]amino}(10-hydropyrazolo[1,5-*a*]quinoxaline-8-yl))-*N*-{[5-(trifluoromethyl)(2-pyridinyl)]methyl}formamide 4-{[(4-methoxyphenyl)methyl]amino}-10-hydropyrazolo[1,5-*a*]quinoxaline-8-carboxylic acid (200.0 mg, 0.57 mmol) and cyclopropyl{[5-(trifluoromethyl)(2-pyridinyl)]methyl}amine (124.1 mg, 0.57 mmol) were dissolved in 3-mL solution of *N,N-*dimethylformamide (DMF), and then HATU (327.25 mg, 0.86 mmol) and DIEA (222.2 mg, 1.72 mmol) were added. After completion of the reaction as monitored by TLC and LCMS, the reaction solution was slowly added into 10 mL of water, and the mixture was extracted with EA (10 mL × 3). The combined organic phases were dried over anhydrous Na₂SO₄, filtered, and concentrated to give *N*-cyclopropyl(4-{[(4-methoxyphenyl)methyl]amino}(10-hydropyrazolo[1,5-*a*]quinoxaline-8-yl))-*N*-{[5-(trifluoromethyl)(2-pyridinyl)]methyl}formamide (200.0 mg, yield: 48%).

LCMS (ESI) *m*/*z*: 509[M+H]⁺

### Step II: Synthesis of (4-amino(10-hydropyrazolo[1,5-a]quinoxaline-8-yl))-N-cyclopropyl-N-{[5-(trifluoromethyl)(2-pyridinyl)]methyl}formamide

The TFA (2 mL) solution of *N*-cyclopropyl(4-{[(4-methoxyphenyl)methyl]amino}(10-hydropyrazolo[1,5-*a*]quinoxaline-8-yl))-*N*-{[5-(trifluoromethyl)(2-pyridinyl)]methyl}formamide (100.00 mg, 0.18 mmol) was stirred overnight at 60°C. After the reaction completed, the solution was diluted with EA and washed continuously with saturated sodium bicarbonate. Organic layers were collected, dried over Na₂SO₄, concentrated, and then purified by PREP_HPLC (column: XBridge Shield RP18 OBD column, 30 × 150 mm, 5 µm; mobile phase A: water (10 mmol/L NH₄HCO₃), mobile phase B: ACN; flow rate: 60 mL/min; gradient: 20% B to 55% B in 10 min; wavelength: 220 nm; RT1 (min): 8.48) to give (4-amino(10-hydropyrazolo[1,5-*a*]quinoxaline-8-yl))-*N-*cyclopropyl-N-{[5-(trifluoromethyl)(2-pyridinyl)]methyl}formamide (21.30 mg, yield: 27%) as a white solid.

¹H NMR (400 MHz, DMSO-*d₆*): δ *ppm* 8.95 (s, 1H), 8.06 (s, 1H), 8.50-8.40 (m, 1H), 8.20-8.10 (m, 2H), 7.80-7.52 (m, 3H), 7.20 (s, 1H), 4.90 (s, 2H), 3.12-3.09 (m, 1H),0.56-0.51 (m, 4H).

LCMS (ESI) *m*/*z*: 427.35 [M+H]⁺

The example in the following table may be prepared using the synthetic step described in **Method II of Example 6,** only replacing the corresponding starting materials:

| Exa mple No. | Chemical Structure | Chemical Name | ¹HNMR&¹⁹F NMR& LCMS |
|---|---|---|---|
| 61 | 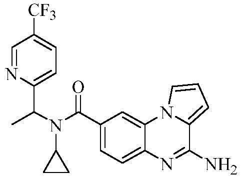 | (4-amino(10-hydropyrrolo[1,2-*a*]quinoxalin-8-yl))-*N*-cyclopropyl-*N*-{[5-(trifluoromethyl)(2-pyridyl)]ethyl}forma mide | ¹H NMR (400 MHz, DMSO-*d₆*) δ 8.95 (s, 1H), 8.32-8.31 (m, 1H), 8.21 (d, *J* = 1.8 Hz, 1H), 8.16-8.15 (m, 1H), 7.65 (d, *J =* 8.3 Hz, 1H), 7.47-7.46 (m, 1H), 7.40 (d, *J* = 8.3 Hz, 1H), 7.18 (s, 3H), 7.10 - 7.04 (m, 1H), 5.44 (q, *J* = 7.0 Hz, 1H), 3.07 (s, 1H), 1.86 (d, *J* = 7.1 Hz, 3H), 0.47 - 0.36 (m, 4H). |
| | | | LCMS (ESI) *m*/*z*: 440.35 [M+H]⁺ |

### Example 62: 4-amino-N-cyclopropyl-N-(1-(5-(trifluoromethyl)pyridin-2-yl)ethyl)imidazo[1,5-a]quinoxaline-8-formamide

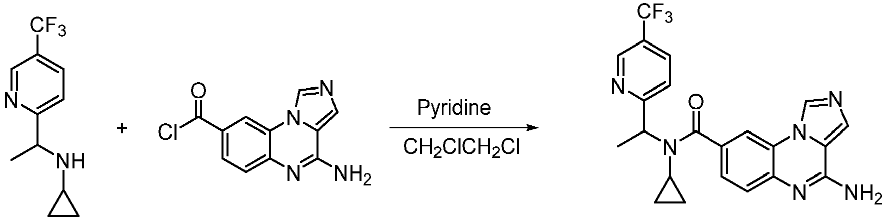

4-{[(4-methoxyphenyl)methyl]amino}-10-hydroimidazo[1,5-*a*]quinoxaline-8-carbonyl chloride (100 mg, 0.41 mmol) was dissolved in 1,2-dichloroethane (2 mL), and then added dropwise into 1,2-dioxane (10 mL) of *N*-(1-(5-(trifluoromethyl)pyridin-2-yl)ethyl)cyclopropanamine (93 mg, 0.41 mmol) and pyridine (97 mg, 1.22 mmol). The mixture was stirred at 60°C for 2 h. After completion of the reaction, the filtrate was concentrated and the crude product was purified by reversed-phase chromatography (ACN:water = 50:50) to give 4-amino-*N*-cyclopropyl-*N*-(1-(5-(trifluoromethyl)pyridin-2-yl)ethyl)imidazo[1,5-*a*]quinoxaline-8-formamide (23 mg, 0.05 mmol, yield: 12.85%) as a white solid.

¹H NMR (400 MHz, DMSO-*d₆*) δ 9.19 (s, 1H), 8.95-8.94 (m, 1H), 8.36-8.35 (m, 1H), 8.18-8.15 (m, 1H), 7.92-7.91 (m, 1H), 7.68-7.61 (m, 1H), 7.57-7.55 (m, 1H), 7.43-7.41 (m, 3H), 5.46-5.41 (m, 1H), 3.10-3.08 (m, 1H), 1.86-1.85 (m, 3H), 0.54-0.34 (m, 4H).

LCMS (ESI): 441.30 [M+H]⁺

The examples in the following table may be prepared using the synthetic step described in Example 62, only replacing the corresponding starting materials:

| Exa mple No. | Chemical Structure | Chemical Name | ¹HNMR&¹⁹F NMR& LCMS |
|---|---|---|---|
| 63 | 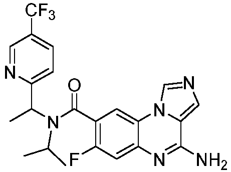 | 4-amino-7-fluoro-*N-*isopropyl-*N*-(1-(5-trifluoromethyl)pyridi n-2-yl)ethyl)imidazo[1,5-*a*]quinoxaline-8-formamide | ¹H NMR (400 MHz, DMSO-*d₆*) δ 9.19 (d, *J =* 4.0 Hz, 1H), 8.96 (s, 1H), 8.18 (s, 2H), 7.92 (d, *J* = 3.7 Hz, 1H), 7.56 (d, *J* = 4.5 Hz, 3H), 7.26 (dd, *J* = 11.2, 4.0 Hz, 1H), 5.08 - 4.75 (m, 1H), 4.17 - 3.86 (m, 1H), 1.92 (s, 2H), 1.62 (d, *J* = 6.9 Hz, 1H), 1.54 - 1.00 (m, 6H). |
| | | | LCMS: (ESI) m/z: 459.1 [M+H]⁺ |
| 64 | 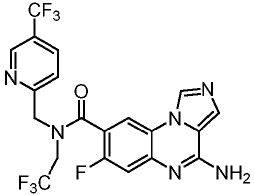 | 4-amino-7-fluoro-*N-*(2,2,2-trifluoroethyl)-*N*-((6-(trifluoromethyl)pyrid in-3-yl)methyl)imidazo[1, 5-*a*]quinoxaline-8-carboxylamide | ¹H NMR (400 MHz, DMSO-*d₆*) δ 9.28 - 8.84 (m, 2H), 8.34 - 8.02 (m, 2H), 7.91 (d, J = 24.2 Hz, 1H),7.74 - 7.54 (m, 3H), 7.31 (dd, J = 29.9, 9.5 Hz, 1H), 7.18 (dd, J = 34.6, 11.6 Hz, 1H), 4.93 (d, J = 63.5Hz, 2H), 4.61 - 4.05 (m, 2H). |
| | | | LCMS (ESI)m/z:487.2[M+H]⁺ |
| 65 | 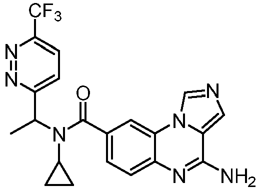 | (4-amino(10-hydroimidazo[1,5-*a*]quinoxalin-8-yl)-*N-*cyclopropyl-*N*-{[6-(trifluoromethyl)pyrid azin-3-yl]ethyl}formamide | ¹H NMR (400 MHz, DMSO-*d₆*) δ 9.19 (s, 1H), 8.37 (s, 1H), 8.23 (d, *J* = 8.8 Hz, 1H), 8.03 (d, *J* = 8.9 Hz, 1H), 7.92 (s, 1H), 7.58 (d, *J* = 8.5 Hz, 1H), 7.44 (d, *J* = 8.7 Hz, 3H), 5.55 (q, *J=* 7.0 Hz, 1H), 1.96 (d, *J=* 7.0 Hz, 3H), 0.57-0.45 (m, 4H). |
| | | | LCMS (ESI): 442.30 [M+H]⁺ |

### Example 66 Method II: 4-amino-7-chloro-N-(1-(pyrimidin-2-yl)ethyl)-N-((5-(trifluoromethyl)pyridin-2-yl)methyl)pyrrolo[1,2-a]quinoxaline-8-formamide

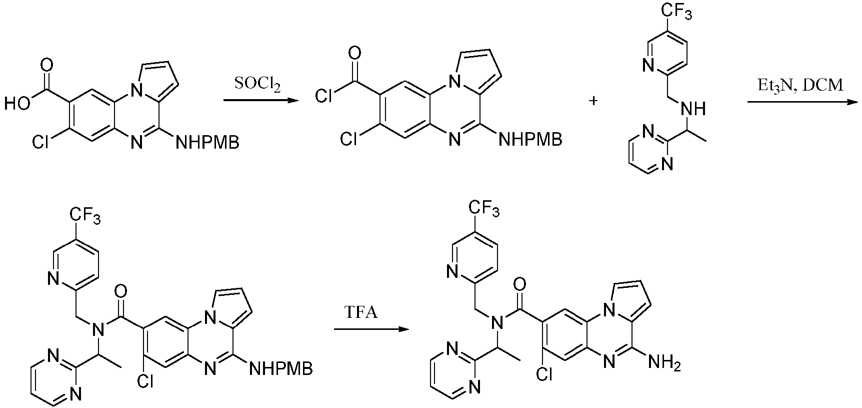

### Step I: Synthesis of 7-chloro-4-((4-methoxybenzyl)amino)-N-(1-(pyrimidin-2-yl)ethyl)-N-((5-(trifluoromethyl)pyridin-2-yl)methyl)pyrrolo[1,2-a]quinoxaline-8-formamide

At room temperature, 7-chloro-4-((4-methoxybenzyl)amino)pyrrolo[1,2-*a*]quinoxaline-8-carboxylic acid (180 mg, 0.472 mmol) was dissolved in DCM (3 mL), and then thionyl chloride (280.76 mg, 2.36 mmol) was added into the system successively. The resulting mixture was stirred at 50°C for 3 h. After the reaction completed, the reaction solution was concentrated in vacuum under reduced pressure and dissolved with DCM (2 mL) after the thionyl chloride was removed. 1-(pyrimidin-2-yl)-*N*-((5-(trifluoromethyl)pyridin-2-yl)methyl)ethan-1-amine (199.65 mg, 0.708 mmol) and triethylamine (TEA) (287.0 mg, 2.838 mmol) were dissolved with DCM (1 mL), and the mixture which had been spun dried and dissolved was added dropwise into the system. The obtained mixture was stirred at 25°C for 3 h. After the reaction completed, the mixture was extracted with EA (10 mL × 3).

The combined organic phases were dried over anhydrous sodium sulfate and filtered. The resulting filtrate was concentrated under reduced pressure to give a crude product, which was then purified by silica gel column chromatography (PE:EA 1:3, v/v) to give 7-chloro-4-((4-methoxybenzyl)amino)-*N*-(1-(pyrimidin-2-yl)ethyl)-*N*-((5-(trifluoromethyl)pyridin-2-yl)methyl)pyrrolo[1,2-*a*]quinoxaline-8-formamide (80.0 mg, yield: 26.2%) as a yellow solid.

LCMS (ESI) m/z: **646.3** [M+H]⁺

### Step II: Synthesis of 4-amino-7-chloro-N-(1-(pyrimidin-2-yl)ethyl)-N-((5-(trifluoromethyl)pyridin-2-yl)methyl)pyrrolo[1,2-a]quinoxaline-8-formamide

At room temperature, 7-chloro-4-((4-methoxybenzyl)amino)-*N*-(1-(pyrimidin-2-yl)ethyl)-*N-*((5-(trifluoromethyl)pyridin-2-yl)methyl)pyrrolo[1,2-*a*]quinoxaline-8-formamide (80 mg, 0.12 mmol) was dissolved in TFA (2 mL), and the obtained mixture was stirred at 75°C for 3 h. After the reaction completed, the reaction solution was concentrated under reduced pressure, poured over water (10 mL), and then adjusted to pH = 8 with saturated sodium bicarbonate solution. The mixture was extracted with EA (10 mL × 3). The combined organic phases were dried over anhydrous sodium sulfate and filtered. The obtained filtrate was concentrated under reduced pressure to give a crude product, which was then purified by reversed-phase column chromatography (water:ACN = 5:95, v/v) to give 4-amino-7-chloro-*N*-(1-(pyrimidin-2-yl)ethyl)-*N*-((5-(trifluoromethyl)pyridin-2-yl)methyl)pyrrolo[1,2-*a*]quinoxaline-8-formamide (11.1 mg, yield: 17.5%) as a white solid.

¹H NMR (400 MHz, DMSO-*d₆*) δ 8.89 - 8.86 (m, 1H), 8.79 (d, *J* = 4.8 Hz, 1H), 8.69 (d, *J* = 4.8 Hz, H), 8.30 - 8.20 (m, 1H), 8.14 - 8.08 (m, 1H), 8.01 (s, 1H), 7.74 (d, *J =* 8.4 Hz, 1H), 7.45 - 7.38 (m, 1H), .32 - 7.26 (m, 2H), 7.22 - 7.13 (m, 1H), 7.12 - 7.00 (m, 1H), 6.77 - 6.65 (m, 1H), 5.18 - 5.03 (m, 2H), .60 (d, *J* = 16.8 Hz, 1H), 1.62 - 1.54 (m, 3H).

LCMS (ESI) m/z: 526.9 [M+H]⁺

### Example 23 Method II: N-{(1R)-1-[4-(trifluoromethyl)phenyl]ethyl}(4-amino-7-chloro(10-hydroimidazo[1,5-a]quinoxaline-8-yl))-N-methylformamide

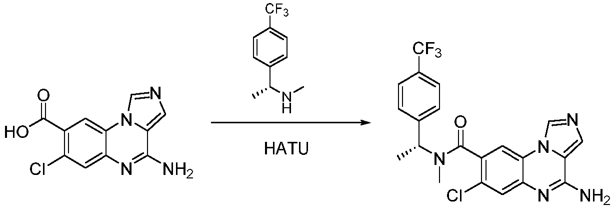

Step I: At room temperature, (*R*)-*N*-methyl-1-(4-(trifluoromethyl)phenyl)ethan-1-amine (100.00 mg, 0.49 mmol) and 4-amino-10-hydroimidazo[1,5-*a*]quinoxaline-8-carboxylic acid (111.22 mg, 0.49 mmol) were dissolved in DMF (2.00 mL), and N,N,N',N'-tetramethyl-O-(7-azabenzotriazol-1-yl)urea hexafluorophosphate (277.80 mg, 0.73 mmol) and DIEA (188.97 mg, 1.46 mmol) were added. The mixture was stirred at 25°C for 16 h. After the reaction completed, the mixture was diluted with EA and washed continuously with saturated sodium bicarbonate. The organic phases were collected, dried over anhydrous sodium sulfate, concentrated, and then purified by PREP_HPLC (column: YMC Triart C18 ExRs 5 m, 30 mm × 150 mm; mobile phase A: water (10 mmol/L NH₄HCO₃), mobile Phase B: ACN; flow rate: 60 mL/min; gradient: 35% B to 57% B in 10 min; wavelength: 254 nm/220 nm; retention time (min): 8.42) to give *N-{(1R)-1-[4-*(trifluoromethyl)phenyl] ethyl}(4-amino-7-chloro(10-hydroimidazo[1,5-*a*]quinoxaline-8-yl))-N-methylformamide (33.84 mg, 0.57 mmol, yield: 13%) as a white solid.

¹H NMR (400 MHz, DMSO-*d₆*) δ 9.21-9.10 (m, 1H), 8.43-8.30 (m, 1H), 7.94-7.91 (m, 1H), 7.80-7.71 (m, 2H), 7.70-7.60 (m, 3H), 7.57 - 7.54 (m, 1H), 7.50-7.48 (m, 1H), 6.04-4.83 (m, 1H), 2.56 (s, 3H), 1.65-1.61 (m, 3H).

LCMS (ESI):448.10 [M+H]⁺.

The examples in the following table may be prepared using the synthetic step described in **Method II of Example 23,** only replacing the corresponding starting materials:

| Exa mple No. | Chemical Structure | Chemical Name | ¹HNMR&¹⁹F NMR& LCMS |
|---|---|---|---|
| 67 | 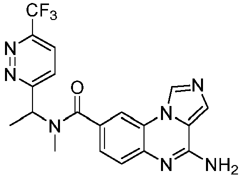 | 4-amino-*N*-methyl-*N-*(1-(6-(trifluoromethyl)pyrid azin-3-yl)ethyl)imidazo[1,5-*a*]quinoxaline-8-formamide | ¹H NMR (400 MHz, DMSO-*d₆*) δ 9.17 (s, 1H), 8.34 - 8.25 (m, 2H), 8.04 (s, 1H), 7.91 (s, 1H), 7.45 (d, *J* = 4.0 Hz, 4H), 5.97-5.76 (m, 1H), 2.94-2.80 (m, 3H), 1.78-1.76 (m, 3H). |
| | | | LCMS (ESI): 416.15 [M+H]⁺ |
| 68 | 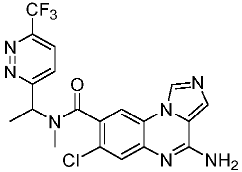 | 4-amino-*N-*cyclopropyl-*N*-(1-(6-(trifluoromethyl)pyrid azin-3-yl)ethyl)imidazo[1,5-*a*]quinoxaline-8-formamide | ¹H NMR (400 MHz, DMSO-*d₆*) δ 9.23-8.99 (m, 1H), 8.46-8.13 (m, 2H), 8.13-7.86 (m, 4H), 7.55-7.47 (m, 1H), 6.15-5.13 (m, 1H), 2.95-2.87 (m, 3H), 1.79-1.65 (m, 3H). |
| | | | LCMS (ESI): 450.10 [M+H]⁺ |
| 69 | 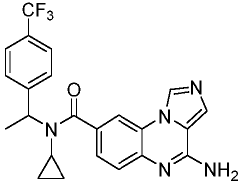 | 4-amino-*N-*cyclopropyl-*N*-(1-(4-(trifluoromethyl)phen yl)ethyl)imidazo[1,5-*a*]quinoxaline-8-formamide | ¹H NMR (400 MHz, DMSO-*d₆*) δ 9.20 (s, 1H), 8.37 (d, *J=* 1.8 Hz, 1H), 7.91 (d, *J* = 0.7 Hz, 1H), 7.73 (d, *J* = 8.2 Hz, 2H), 7.62 (d, *J* = 8.1 Hz, 2H), 7.55 (dd, *J=* 8.4, 1.8 Hz, 1H), 7.43 (d, *J* = 8.6 Hz, 3H), 5.56 (q, *J*= 7.1 Hz, 1H), 2.95 (s, 1H), 1.84(d, *J* = 7.1 Hz, 3H), 0.50 - 0.33 (m, 2H), 0.33 - 0.25 (m, 1H), 0.12 (dt, *J* = 10.2, 5.3 Hz, 1H). |
| | | | ¹⁹F NMR (376 MHz, DMSO-d₆) δ -60.76. |
| | | | LCMS (ESI) m/z: 440.4 [M+H]⁺ |
| 70 | 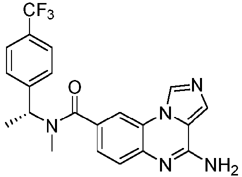 | (*R*)-4-amino*-N-*methyl-*N*-(1-(4-(trifluoromethyl)phen yl)ethyl)imidazo[1,5-*a*]quinoxaline-8-formamide | ¹H NMR (400 MHz, DMSO-*d₆*) δ 9.23-9.17 (m, 1H), 8.31 (s, 1H), 7.92 (s, 1H), 7.76-7.74 (m, 2H), 7.68-7.50 (m, 2H), 7.50-7.42 (m, 4H), 6.04-4.97(m,1H),2.90 (s,3H), 1.68-1.63 (m, 3H). |
| | | | LCMS (ESI): 414.15 [M+H]⁺ |
| 71 | 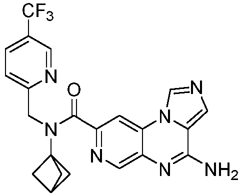 | 4-amino-*N-*(bicyclo[1.1.1]pentan-1-yl)-*N*-((5-(trifluoromethyl)pyrid in-2-yl)methyl)imidazo[1,5 -*a*]pyrido[3,4-*e*]pyrazine-8-formamide | ¹H NMR (400 MHz, DMSO-*d₆*) δ 9.28 (s, 1H), 8.98-8.86 (m, 1H), 8.80- 8.37 (m, 2H), 8.27 (m, 1H), 8.17 (s, 1H), 7.98-7.71 (s, 2H), 7.62 (s, 1H), 4.99 -4.92 (m, 2H), 2.20-2.09 (m, 3H), 1.66 (s, 4H). |
| | | | LCMS (ESI): 454.20 [M+H]⁺ |
| 72 | 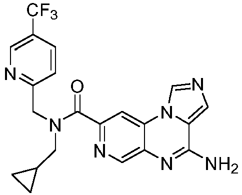 | 4-amino-*N-*(cyclopropylmethyl)-*N*-((5-(trifluoromethyl)pyrid in-2-yl) methyl)imidazo[1,5-*a*]pyrido[3,4-*e*]pyrazine-8-formamide | ¹H NMR (400 MHz, DMSO-*d₆*) δ 9.30-9.27 (m, 1H), 8.95-8.85 (s, 1H), 8.67(s, 1H), 8.49-8.47 (m, 1H), 8.25-8.13(m, 1H), 7.98-7.95(m, 1H), 7.73-7.66(m, 2H), 7.66-7.61(m, 1H), 5.09(s, 1H), 4.99(s, 1H), 3.46-3.35(m, 2H), 1.10-1.07(m, 1H), 0.43-0.42(m, 1H), 0.34-0.32(m, 1H), 0.22-0.21(m, 1H), 0.08-0.07(m, 1H). |
| | | | LCMS (ESI): 442.29 [M+H]⁺ |
| 73 | 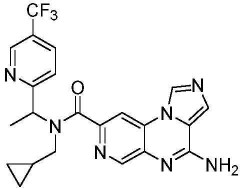 | 4-amino-*N-*(cyclopropylmethyl)-*N*-(1-(5-(trifluoromethyl)pyrid in-2-yl)ethyl)imidazo[1,5-*a*]pyrido[3,4-*e*]pyrazine-8-formamide | ¹H NMR (400 MHz, DMSO-*d₆*) δ 9.45 (s, 1H), 9.11-9.07 (m, 1H), 8.79(s, 1H), 8.63-8.55 (m, 1H), 8.38-8.33 (m,1H), 8.12 (s, 1H), 7.89-7.88 (m, 1H),7.85-7.84 (m, 2H), 5.72-5.67 (m, 1H), 3.21-3.20 (m, 1H), 1.97-1.80(m, 3H),1.13-1.08(m, 1H), 0.54-0.33(m, 3H),0.15-0.01(m, 2H). |
| | | | LCMS (ESI): 456.40 [M+H]⁺ |
| 74 | 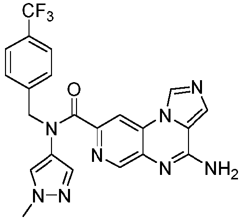 | 4-amino-*N*-(1-methyl-1*H*-pyrazol-4-yl)-*N-*(4-(trifluoromethyl)benz yl)imidazo[1,5-*a*]pyrido[3,4-*e*]pyrazine-8-formamide | ¹H NMR (400 MHz, DMSO-*d₆*) δ 9.25 (s, 1H), 8.57(s, 1H), 8.43 (m, 1H), 8.08-7.96 (m, 1H), 7.74-7.68 (m, 3H), 7.59-7.46 (m, 4H), 7.05 (s, 1H), 5.25-5.09 (m, 2H), 3.79-3.50 (m, 3H). |
| | | | LCMS (ESI): 467.35 [M+H]⁺ |
| 75 | 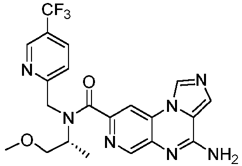 | (*R*)-4-amino-*N*-(1-methoxypropan-2-yl)-*N*-((5-(trifluoromethyl)pyrid in-2-yl)methyl)imidazo[1,5 -*a*]pyrido[3,4-*e*]pyrazine-8-formamide | ¹H NMR (400 MHz, DMSO-*d₆*) δ 9.32 (s, 1H), 8.97(s, 1H), 8.69 (s, 1H), 8.44 (s, 1H), 8.23-8.21 (m, 1H), 7.98 (s, 1H), 7.72 (s, 2H), 7.65-7.63 (m, 1H), 6.53 (s, 1H), 4.89 - 4.85(m, 1H), 4.74 - 4.70 (m,1H), 4.49-4.30 (m, 1H), 3.25-3.24 (m, 1H), 3.05 (s, 3H), 1.23 - 1.17 (m, 3H). |
| | | | LCMS (ESI) m/z: 460.15[M+H]⁺ |
| 76 | 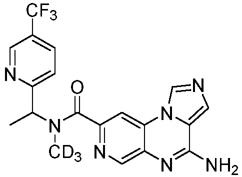 | 4-amino-*N*-(methyl-*d₃)-N-*(1-(5-(trifluoromethyl)pyrid in-2-yl)ethyl)imidazo[1,5-*a*]pyrido[3,4-*e*]pyrazine-8-formamide | ¹H NMR (400 MHz, DMSO-*d₆*) δ 9.29-9.28 (m, 1H), 8.99-8.95 (m, 1H), 8.66-8.61 (m, 1H), 8.50-8.46 (m, 1H), 8.27-8.22 (m, 1H), 7.98 (s, 1H), 7.71-7.66 (m, 3H), 5.95-5.56(m,1H), 1.67-1.63(m, 3H). |
| | | | LCMS (ESI): 419.30[M+H]⁺ |
| 77 | 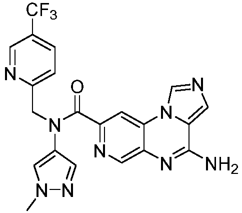 | 4-amino-*N*-(1-methyl-1H-pyrazol-4-yl)-*N-*((5-(trifluoromethyl) pyridin-2-yl)methyl)imidazo[1,5 -*a*]pyrido[3,4- | ¹H NMR (400 MHz, DMSO-*d₆*) δ 9.26 (s, 1H), 8.97-8.51 (m, 1H), 8.56-8.24 (m, 2H), 8.25 - 7.97 (m, 2H), 7.74-7.69 (m,3H), 7.56-7.12 (m, 2H), 5.37 (s, 1H), 5.17 (s, 1H), 3.58 (s, 3H). |
| | | *e*]pyrazine-8-formamide | LCMS (ESI): 468.35 [M+H]⁺ |
| 17 | 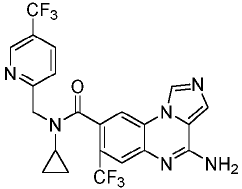 | 4-amino-*N-*cyclopropyl-7-(trifluoromethyl)-*N-*((5-(trifluoromethyl)pyrid in-2-yl)methyl)imidazo[1,5 -*a*]quinoxaline-8-formamide | ¹H NMR (400 MHz, DMSO-*d₆*) δ 9.26 (s, 1H), 9.06 - 8.95 (m, 1H), 8.44 (s, 1H), 8.30 - 8.22 (m, 1H),7.98 (s, 1H), 7.71 (s, 1H), 7.70 - 7.57 (m, 3H), 4.89 (s, 2H), 3.08 - 2.77 (m, 1H), 0.95 - 0.22 (m, 4H). |
| | | | LCMS (ESI) m/z: 495.4 [M+H]+ |
| 78 | 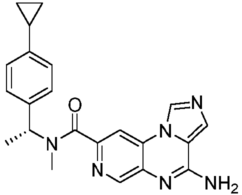 | (*R*)-4-amino-*N*-(1-(4-cyclopropylphenyl)eth yl)N-methylimidazol[1,5-*a*]pyridine[3,4-*e*]pyrazin-8-formamide | ¹H NMR (400 MHz, DMSO-*d₆*) *δ* 9.29 (d, *J* = 7.6 Hz, 1H), 8.65 (d, *J* = 6.4 Hz, 1H), 8.52 - 8.40 (m, 1H), 7.98 (s, 1H), 7.75 - 7.59 (m, 2H), 7.35 - 7.19 (m, 2H), 7.15 - 7.00 (m, 2H), 5.97 - 5.24 (m, 1H), 2.67 (d, *J* = 9.6 Hz, 3H), 1.98 - 1.83 (m, 1H), 1.55 (d, *J* = 6.8 Hz, 3H), 0.99 - 0.89 (m, 2H), 0.70 - 0.60 (m, 2H). |
| | | | LCMS (ESI) m/z: 387.3 [M+H]⁺ |
| 79 | 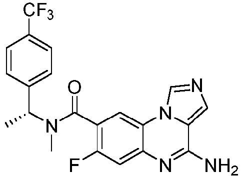 | (*R*)-4-amino-7-fluoro-*N*-methyl-*N*-(1-(4-(trifluoromethyl)phen yl)ethyl)imidazo[1,5-*a*]quinoxaline-8-formamide | ¹H NMR (400 MHz, DMSO-*d₆*) δ9.15-9.11 (m, 1H), 8.40-8.30 (m, 1H), 7.91 (s, 1H), 7.79-7.77 (m, 1H), 7.74-7.72 (m, 1H), 7.63-7.61(m, 1H), 7.60-7.57 (m, 2H), 7.50-7.48(m, 1H), 7.26-7.21(m, 1H), 6.00-5.01(m, 1H), 2.75-2.66(m, 3H), 1.64-1.61 (m, 3H). |
| | | | LCMS (ESI):432.10 [M+H]⁺ |
| 58 | 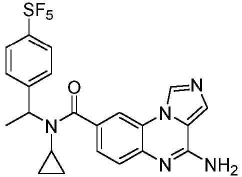 | 4-amino-*N-*cyclopropyl-*N*-(1-(4-(pentafluoro- λ ⁶-sulfonamido)phenyl)et hyl)imidazo[1,5-*a*]quinoxaline-8-formamide | ¹H NMR (400 MHz, DMSO-d₆) δ 9.20 (d, *J* = 0.7 Hz, 1H), 8.37 (d, *J* = 1.8 Hz, 1H), 7.95 - 7.82 (m, 3H), 7.65 - 7.52 (m, 2H), 7.56-7.54 (m, 1H), 7.44-7.42(m,3H), 5.51-5.47 (m, 1H), 2.98-2.76(m,1H),1.84-1.82 (m, 3H), 0.53 - 0.35 (m, 2H), 0.34 - 0.27 (m, 1H), 0.17 - 0.12 (m, 1H). |
| | | | LCMS (ESI): 497 [M+H]⁺ |

### Example 80 6-amino(7-hydroimidazo[1,2-c]quinazolin-2-yl)-N-(pyrimidin-2-ylethyl)-N-{[5-(trifluoromethyl)(2-pyridinyl) methyl } formamide

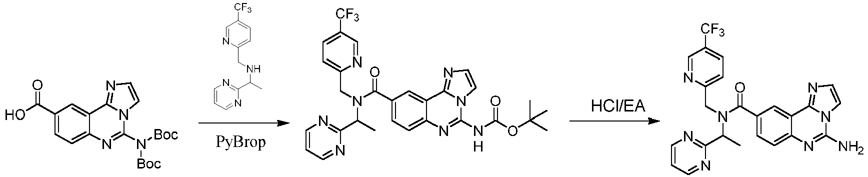

### Step I: {6-[tert-butoxy)carbonylamino](7-hydroimino[1,2-c]quinazolin-2-yl)}-N-(pyrimidin-2-ylethyl)-N-{[5-(trifluoromethyl)(2-pyridinyl)]methyl } formamide

Lithium 6-[(tert-butoxyl)-*N*-[(tert-butyl)oxycarbonyl]carbonylamino]-7-hydroimidazolo[1,2-*c*]quinazoline-2-carboxylate (50 mg, 0.15 mmol) was dissolved in DMF (2 mL), and then (pyrimidin-2-ylethyl)[5-(trifluoromethyl)(2-pyridyl)methyl]amine (52 mg, 0.18 mmol), Triethylamine (47 mg, 0.46 mmol) and PyBrOP(86 mg, 0.18 mmol) was added into the system successively. The mixture was stirred at room temperature for 1 h. After the reaction completed, the system was poured over water (10 mL), and extracted with EA (10 mL × 3). The organic phases were dried over anhydrous sodium sulfate, filtered, concentrated, and then purified by silica gel column chromatography (dichloromethane:methanol = 10:1) to give {6-[tert-butoxyl)carbonylamino](7-hydroimino[1,2-*c*]quinazolin-2-yl)}-*N*-(pyrimidin-2-ylethyl)-*N*-{[5-(trifluoromethyl)(2-pyridyl)]methyl}formamide (60 mg, crude product) as a white solid.

LCMS (ESI) m/z: 593.2[M+H]⁺

### Step II: 6-amino(7-hydroimidazo[1,2-c]quinazolin-2-yl)-N-(pyrimidin-2-ylethyl)-N-{[5-(trifluoromethyl)(2-pyridinyl)]methyl } formamide

At room temperature, {6-[tert-butoxy)carbonylamino](7-hydroimino[1,2-*c*]quinazolin-2-yl)}-*N*-(pyrimidin-2-ylethyl)-*N*-{[5-(trifluoromethyl)(2-pyridyl)methyl}formamide (50 mg, 0.084 mmol) was dissolved in EA(2 mL), and HCl/EA (0.2 mL. 0.84 mmol) was added thereto. After being stirred at room temperature for 2 h, the reaction solution was poured over water (10 mL), adjusted to pH = 8 with saturated sodium carbonate solution, and extracted with EA (10 mL × 3). The organic phases was dried over anhydrous sodium sulfate, filtered, concentrated, and then purified by reversed-phase column chromatography (acetonitrile:water=5:95-70:30) to give 6-amino(7-hydroimidazo[1,2-c]quinazolin-2-yl)-*N-*(pyrimidin-2-ylethyl)-*N*-{[5-(trifluoromethyl)(2-pyridyl)]methyl} formamide (3 mg, 0.006 mmol, yield: 7%) as a white solid.

LCMS (ESI) m/z: 493.3[M+H]⁺

### Example 97 4-amino-N-cyclopropyl-7-fluoro-N-(6-(trifluoromethyl)pyridin-3-ylmethyl)imidazo[1,5-a]quinoxaline-8-formamide

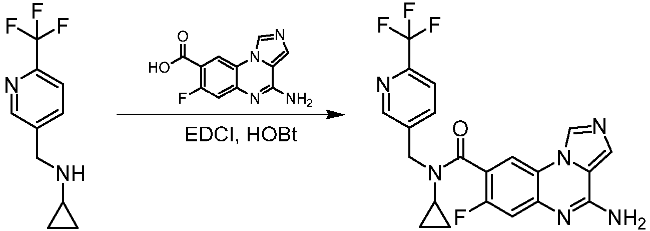

At room temperature, a compound of 4-amino-7-fluoro-10-hydroimidazo[1,5-*a*]quinoxaline-8-carboxylic acid (50 mg, 0.202 mmol) was dissolved in DMF (3 mL), and 1-(3-dimethylaminopropyl)-3-ethylcarbodiimide hydrochloride (46 mg, 0.239 mmol), 1-hydroxybenzotriazole (32 mg, 0.239 mmol) and DIEA (95 mg, 0.736 mmol) were added. The mixture was stirred at room temperature for 0.5 h. *N*-(6-(trifluoromethyl)pyridazin-3-ylmethyl)cyclopropanamine (40 mg, 0.184 mmol) was then added and the mixture was stirred at room temperature for 16 h. When the reaction completed as indicated by LCMS, water (20 mL) was poured over, and the mixture was extracted with EA (30 mL). The organic phases were washed with a saturated brine solution (20 mL), dried over anhydrous sodium sulfate, and filtered. The filtrate was concentrated under reduced pressure to give a crude product, which was then isolated and purified by column chromatography (0-5% methanol/DCM) to give 4-amino-*N*-cyclopropyl-7-fluoro-*N*-(6-(trifluoromethyl)pyridin-3-ylmethyl)imidazo[1,5-*a*]quinoxaline-8-formamide (15 mg, yield: 4.2%).

¹H NMR (400 MHz, DMSO-*d₆*) δ 9.13 (s, 1H), 8.81 (s, 1H), 8.34 (d, *J* = 6.6 Hz, 1H), 8.08 (d, *J* = 8.0 Hz, 1H), 7.96 (d, *J* = 16.4 Hz, 2H), 7.60 (s, 2H), 7.24 (d, *J* = 11.2 Hz, 1H), 4.90 (s, 2H), 2.81 (s, 1H), 0.53 (d, *J =* 12.7 Hz, 4H).

LCMS (ESI) m/z: 445.2 [M+H]⁺

The example in the following table may be prepared using the synthetic step described in **Example 97,** only replacing the corresponding starting materials:

| Exa mple No. | Chemical Structure | Chemical Name | ¹HNMR&¹⁹F NMR& LCMS |
|---|---|---|---|
| 99 | 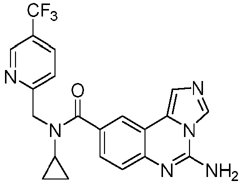 | 5-amino-*N-*cyclopropyl-*N*-((5-trifluoromethyl)pyridi n-2-ylmethyl)imidazo[1,5-*c*]quinazoline-9-formamide | ¹H NMR (400 MHz, DMSO-*d₆*) δ 8.99 (d, *J =* 2.3 Hz, 1H), 8.66 (s, 1H), 8.33 - 8.14 (m, 2H), 7.97 (s, 1H), 7.79 (s, 2H), 7.64 (d, *J* = 8.4 Hz, 2H), 7.41 (d, *J* = 8.4 Hz, 1H), 4.89 (s, 2H), 3.09 (s, 1H), 0.55 (d, *J* = 8.2 Hz, 4H). |
| | | | LCMS (ESI) m/z: 427.2 [M+H]+ |

### Example 81 (R)-4-amino-N-cyclopropyl-N-(1-(5-(trifluoromethyl)pyridin-2-yl)ethyl)imidazo[1,5-a]quinoxaline-8-formamide, and

### Example 82 (S)-4-amino-N-cyclopropyl-N-(1-(5-(trifluoromethyl)pyridin-2-yl)ethyl)imidazo[1,5-a]quinoxaline-8-formamide

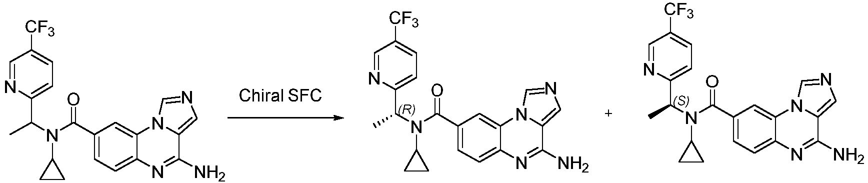

4-amino-*N*-cyclopropyl-*N*-(1-(5-(trifluoromethyl)pyridin-2-yl)ethyl)imidazo[1,5-*a*]quinoxaline-8-formamide (23 mg) was separated by chiral HPLC (CHIRALPAK I-3, 4.6 × 50 mm, 3 µm, mobile phase A: n-Hexane (0.1% Diethylamine), mobile phase B: ethanol, mobile phase A:mobile phase B = 40:60, flow rate: 1.0 mL/min, prepeak retention time: 1.57 min, and postpeak retention time: 2.23 min) to give the prepeak (*R*)-4-amino-*N-*cyclopropyl-*N*-(1-(5-(trifluoromethyl)pyridin-2-yl)ethyl)imidazo[1,5-*a*]quinoxaline-8-formamide (4.79 mg, 0.01 mmol) as a white solid, and the postpeak (*S*)-4-amino-*N-*cyclopropyl-*N*-(1-(5-(trifluoromethyl)pyridin-2-yl)ethyl)imidazo[1, 5-*a*]quinoxaline-8-formamide (3.72 mg, 0.01 mmol) as a white solid.

### Prepeak Example 81 (R)-4-amino-N-cyclopropyl-N-(1-(5-(trifluoromethyl)pyridin-2-yl)ethyl)imidazo[1,5-a]quinoxaline-8-formamide

¹H NMR (400 MHz, DMSO-*d₆*) δ 9.19 (s, 1H), 8.95-8.94 (m, 1H), 8.36-8.35 (m, 1H), 8.18-8.15 (m, 1H), 7.92-7.91 (m, 1H), 7.68-7.61 (m, 1H), 7.57-7.55 (m, 1H), 7.43-7.41 (m, 3H), 5.46-5.41 (m, 1H), 3.10-3.08 (m, 1H), 1.86-1.85 (m, 3H), 0.54-0.34 (m, 4H).

LCMS (ESI): 441.30 [M+H]⁺

### Postpeak Example 82 (S)-4-amino-N-cyclopropyl-N-(1-(5-(trifluoromethyl)pyridin-2-yl)ethyl)imidazo[1,5-a]quinoxaline-8-formamide

¹H NMR (400 MHz, DMSO-*d₆*) δ 9.18 (s, 1H), 8.95-8.93 (m, 1H), 8.36-8.35 (m, 1H), 8.17-8.15 (m, 1H), 7.92-7.90 (m, 1H), 7.68-7.61 (m, 1H), 7.57-7.56 (m, 1H), 7.44-7.41 (m, 3H), 5.46-5.41 (m, 1H), 3.10-3.06 (m, 1H), 1.86-1.85 (m, 3H), 0.57-0.34 (m, 4H).

LCMS (ESI) *m*/*z:* 441.30[M+H]⁺.

The compunds in the following table were obtained using the procedures described in **Examples 81 and 82** and the corresponding conditions for chiral SFC resolution:

| Exa mple No. | Chemical Structure | Chemical Name | SFC Conditions |
|---|---|---|---|
| 83 | 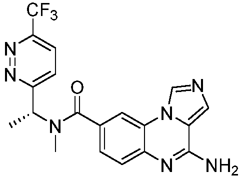 | (*R*)-4-amino-*N-*methyl-*N*-(1-(6-(trifluoromethyl)pyrid azin-3-yl)ethyl)imidazo[1,5-*a*]quinoxaline-8-formamide | Prepeak: SFC (column: ChiralPak IF, AS, (250 × 40 mm I.D., 10 µm), conditions: A for CO₂ and B for MeOH (0.1% NH₃H₂O), 40 %/40%, and flow rate: 120 mL/min) |
| 84 | 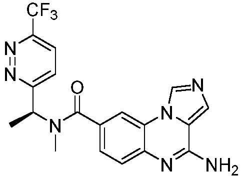 | (*S*)-4-amino-*N-*methyl-*N*-(1-(6-(trifluoromethyl)pyrid azin-3-yl)ethyl)imidazo[1,5-*a*]quinoxaline-8-formamide | Postpeak: SFC (column: ChiralPak IF, AS, (250 × 40 mm I.D., 10 µm), conditions: A for CO₂ and B for MeOH (0.1% NH₃H₂O), 40 %/40%, and flow rate: 120 mL/min) |
| 85 | 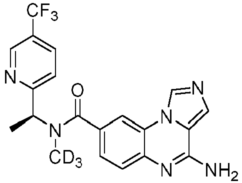 | (*S*)-4-amino-*N-*methyl-*d₃*-*N*-(1-(5-trifluoromethyl)pyridi n-2-yl)ethyl)imidazo[1,5-*a*]quinoxaline-8-formamide | Prepeak: SFC (column: ChiralPak IC HS, (250 mm × 4 mm, 10 µm), conditions: A for CO₂ and B for MeOH (0.1% DEA), 50%/50%, and flow rate: 60 mL/min) |
| 86 | 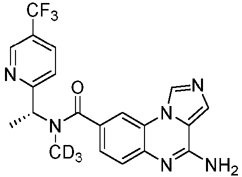 | (*R*)-4-amino-*N-*methyl-*d₃*-*N*-(1-(5-trifluoromethyl)pyridi n-2-yl)ethyl)imidazo[1,5-*a*]quinoxaline-8-formamide | Postpeak: SFC (column: ChiralPak IC HS, (250 mm × 4 mm, 10 µm), conditions: A for CO₂ and B for MeOH (0.1% DEA), 50%/50%, and flow rate: 60 mL/min) |
| 87 | 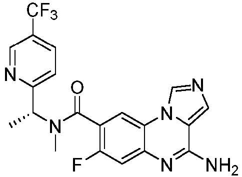 | (*R*)-4-amino-7-fluoro-*N*-methyl-*N*-(1-(4-(trifluoromethyl)phen yl)ethyl)imidazo[1,5-*a*]quinoxaline-8-formamide | Prepeak: SFC (column: ChiralCel OD, (250 mm × 4 mm, 10 µm), conditions: A for CO₂ and B for MeOH (0.1% DEA), 20%/20%, and flow rate: 120 mL/min) |
| 88 | 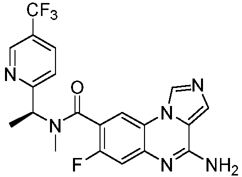 | (*S*)-4-amino-7-fluoro-*N*-methyl-*N*-(1-(4-(trifluoromethyl)phen yl)ethyl)imidazo[1,5-*a*]quinoxaline-8-formamide | Postpeak: SFC (column: ChiralCel OD, (250 mm × 4 mm, 10 µm), conditions: A for CO₂ and B for MeOH (0.1% DEA), 20%/20%, and flow rate: 120 mL/min) |
| 89 | 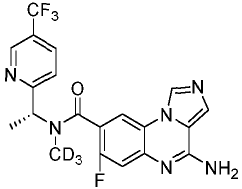 | (*R*)-4-amino-7-fluoro-*N*-methyl-*d₃-N*-(1-(5-trifluoromethyl)pyridi n-2-yl)ethyl)imidazo[1,5-*a*]quinoxaline-8-formamide | Prepeak: SFC (column: ChiralPak IC HS, (250 mm × 40 mm, 10 µm), conditions: A for CO₂ and B for MeOH (0.1% DEA), 50%/50%, and flow rate: 60 mL/min) |
| 90 | 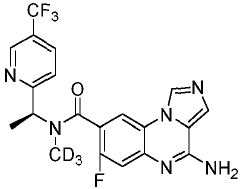 | (*S*)-4-amino-7-fluoro-*N*-methyl-d₃-*N*-(1-(5-trifluoromethyl)pyridi n-2-yl)ethyl)imidazo[1,5-*a*]quinoxaline-8-formamide | Postpeak: SFC (column: ChiralPak IC HS, (250 mm × 40 mm, 10 µm), conditions: A for CO₂ and B for MeOH (0.1% DEA), 50%/50%, and flow rate: 60 mL/min) |
| 91 | 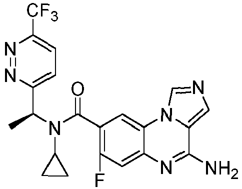 | (*S*)4-amino-*N-*cyclopropyl-7-fluoro-*N*-(1-(6-trifluoromethyl)pyrida zin-3-yl)ethyl)imidazo[1,5-*a*]quinoxaline-8-formamide | Prepeak: SFC (column: ChiralPak IC HS, (250 mm × 40 mm, 10 µm), conditions: A for CO2 and B for MeOH (0.1% NH₃ H₂O), 50%/50%, and flow rate: 60 mL/min) |
| 92 | 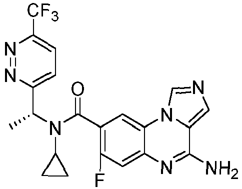 | (*R*)4-amino-N-cyclopropyl-7-fluoro-*N-(*1-(6-trifluoromethyl)pyrida zin-3-yl)ethyl)imidazo[1,5-*a*]quinoxaline-8-formamide | Postpeak: SFC (column: ChiralPak IC HS, (250 mm × 40 mm, 10 µm), conditions: A for CO₂ and B for MeOH (0.1% NH₃ H₂O), 50%/50%, and flow rate: 60 mL/min) |
| 93 | 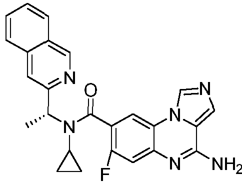 | (*R*)-4-amino-*N-*cyclopropyl-7-fluoro-*N*-(1-(isoquinolin-3-yl)ethyl)imidazo[1,5-*a*]quinoxaline-8-carboxylamide | Prepeak: SFC (column: Daicel ChiralPak AS, (250 × 40 mm I.D., 10 µm), conditions: A for CO₂ and B for MeOH (0.1% NH₃H₂O), 40%/40%, and flow rate: 140 mL/min) |
| 94 | 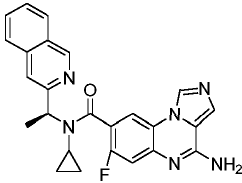 | (*S*)-4-amino-*N-*cyclopropyl-7-fluoro-*N*-(1-(isoquinolin-3-yl)ethyl)imidazo[1,5-*a*]quinoxaline-8-carboxylamide | Postpeak: SFC (column: Daicel ChiralPak AS, (250 × 40 mm I.D., 10 µm), conditions: A for CO₂ and B for MeOH (0.1% NH₃ H₂O), 40%/40%, and flow rate: 140 mL/min) |
| 95 | 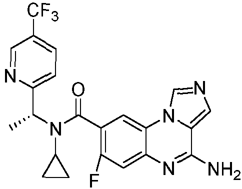 | (*R*)-4-amino-*N-*cyclopropyl-7-fluoro-*N*-(1-(5-trifluoromethyl)pyridi n-2-yl)ethyl)imidazo[1,5-*a*]quinoxaline-8-formamide | Prepeak: SFC (Column: ChiralPak IBN, 40 mm I.D. × 250 mm, 10 µm, conditions: A for CO₂ and B for MeOH (0.1% NH₃.H₂O), 30%/30%, and flow rate:120 mL/min) |
| 96 | 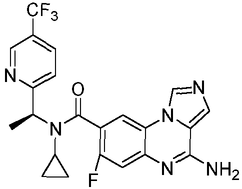 | (*S*)-4-amino-*N-*cyclopropyl-7-fluoro-*N*-(1-(5-trifluoromethyl)pyridi n-2-yl)ethyl)imidazo[1,5-*a*]quinoxaline-8-formamide | Postpeak: SFC (Column: ChiralPak IBN, 40 mm I.D. × 250 mm, 10 µm, conditions: A for CO₂ and B for MeOH (0.1% NH₃.H₂O), 30%/30%, and flow rate:120 mL/min) |
| 111 | 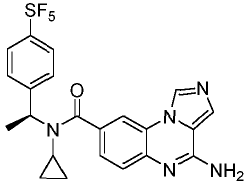 | (*S*)-4-amino-*N-*cyclopropyl-*N*-(1-(4-(pentafluoro- λ ⁶-sulfonamido)phenyl)et hyl)imidazo[1,5-*a*]quinoxaline-8-formamide | Prepeak: SFC (Column: ChiralPak IBN, 40 mm I.D. × 250 mm, 10 µm, conditions: A for CO₂ and B for MeOH (0.1% NH₃.H₂O), 40%/40%, and flow rate:120 mL/min) |
| 112 | 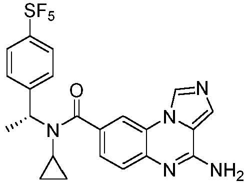 | (*R*)-4-amino-*N-*cyclopropyl-*N*-(1-(4-(pentafluoro-6-sulfonamido)phenyl)et hyl)imidazo[1,5-*a*]quinoxaline-8-formamide | Prepeak: SFC (Column: ChiralPak IBN, 40 mm I.D. × 250 mm, 10 µm, conditions: A for CO₂ and B for MeOH (0.1% NH₃.H₂O), 40%/40%, and flow rate:120 mL/min) |

### Biochemical Evaluation

### I. Inhibitory Activity Test of Compounds on Tumor Cell Proliferation

### Test case 1: Inhibitory activity test of compounds on the proliferation of HCT-116 MTAP(-/- )deleted cells

Materials and cells: HCT-116 MTAP(-/-)deficient cells were purchased from Botron Healthcare (China); RPMI-1640 medium, fetal bovine serum and penicillin-streptomycin were purchased from Thermo Fisher (United States); 384-well plates were purchased from PerkinElmer (United States); and Cell-Titer Glo kits were purchased from Promega (United States).

Cell culture: HCT116 MTAP(-/-)deleted cells were cultured in RPMI 1640 medium containing 10% fetal bovine serum + 1% penicillin-streptomycin at 37°C and 5% CO₂. Cells in the logarithmic growth phase may be used for the test.

Detection of inhibitory activity on cell proliferation: The Cell-Titer Glo kit was used to detect the proliferation inhibitory activity of the compounds on HCT-116 MTAP(-/- )deficient cells. The cell concentration was adjusted, rendering that cells were inoculated into a 384-well plate at 40 µL per well and incubated overnight at 37°C and 5% CO₂. 40 nL of the compound was added into each well to reach a final concentration of 0-10,000 nM (starting concentration: 10,000 nM, 3-fold dilution, 10 points), and the cell plate with 0.1% DMSO content was incubated at 37°C and 5% CO₂ for 8 days. 40 µL of Cell-Titer Glo reagent was added to detect cell activity. The test results are shown in Table 1.

### Test case 2: Inhibitory activity test of compounds on HCT-116 wild-type cell proliferation

Materials and cells: HCT-116 wild-type cells were purchased from Botron Healthcare (China); RPMI-1640 medium, fetal bovine serum and penicillin-streptomycin were purchased from Thermo Fisher (United States); 384-well plates were purchased from PerkinElmer (United States); and Cell-Titer Glo kits were purchased from Promega (United States).

Cell culture: HCT-116 wild-type cells were cultured in RPMI-1640 medium containing 10% fetal bovine serum + 1% penicillin-streptomycin at 37°C and 5% CO₂. Cells in the logarithmic growth phase maybe used for the test.

Detection of cell proliferation activity: The Cell-Titer Glo kit was used to detect the proliferation inhibitory activity of the compounds on HCT-116 wild-type cell proliferation. The cell concentration was adjusted, rendering that cells were inoculated into a 384-well plate at 40 µL per well and incubated overnight at 37°C and 5% CO₂. 40 nL of the compound was added into each well to reach a final concentration of 0-10,000 nM (starting concentration: 10,000 nM, 2-fold dilution, 10 points), and the cell plate with 0.1% DMSO content was incubated at 37°C and 5% CO₂ for 8 days. 40 µL of Cell-Titer Glo reagent was added to detect cell activity. The test results are shown in Table 1.

Table 1 below shows the inhibitory activity of compounds in examples on the proliferation of HCT116 MTAP(-/-)deficient cell and HCT116 wild-type cells.

**Table 1**

| Compounds | IC₅₀ (nM) | |
|---|---|---|
| | HCT-116 MTAP(-/-)deficient cells | HCT-116 wild-type cells |
| Example 1 | 99 | 8800 |
| Example 2 | 3029 | >20000 |
| Example 3 | 12522 | 10443 |
| Example 4 | 322 | >20000 |
| Example 5 | 899 | >20000 |
| Example 6 | >20000 | >20000 |
| Example 7 | 2315.4 | 5188.4 |
| Example 8 | 5079 | >20000 |
| Example 9 | 1477 | >20000 |
| Example 10 | 7123 | 16030 |
| Example 11 | 76.1 | 7896 |
| Example 12 | 160 | 6781 |
| Example 13 | 4296 | >20000 |
| Example 14 | 15.2 | 944.6 |
| Example 15 | 15.6 | 144.4 |
| Example 16 | 66.6 | 4704 |
| Example 17 | 558.5 | >10000 |
| Example 18 | 17.8 | 3229.3 |
| Example 19 | 13.3 | 2818.6 |
| Example 20 | 11.5 | 2004 |
| Example 21 | 216.4 | >10000 |
| Example 22 | 20.7 | 4697.4 |
| Example 23 | 7.9 | 1119.2 |
| Example 24 | 4.5 | 91 |
| Example 25 | >3000 | >10000 |
| Example 26 | 36 | 933 |
| Example 27 | 6.6 | 796.3 |
| Example 28 | 72.4 | 4237.7 |
| Example 29 | 66.6 | 4704.5 |
| Example 30 | 9.7 | 1481 |
| Example 31 | 52.7 | 4327.5 |
| Example 32 | 92.7 | >10000 |
| Example 33 | 89.2 | >10000 |
| Example 36 | 35 | 4659.4 |
| Example 38 | >3000 | >3000 |
| Example 39 | >3000 | >10000 |
| Example 40 | 64190.3 | 8699.4 |
| Example 41 | 96.2 | >10000 |
| Example 42 | >3000 | >10000 |
| Example 43 | 1612 | 13506 |
| Example 44 | 153 | >10000 |
| Example 45 | 13.3 | 193.2 |
| Example 46 | 8.8 | 212.5 |
| Example 47 | 91.1 | >10000 |
| Example 48 | 11.2 | 107 |
| Example 49 | >3000 | >3000 |
| Example 51 | 93.9 | >10000 |
| Example 52 | 143.9 | 4858.3 |
| Example 53 | 227.3 | >10000 |
| Example 54 | 34.6 | 1538.5 |
| Example 55 | 35.5 | 2892.7 |
| Example 56 | 35.4 | 1448.5 |
| Example 57 | 10.5 | 740.7 |
| Example 58 | 12.6 | 796.8 |
| Example 59 | 12.7 | 837 |
| Example 60 | 13.1 | 894 |
| Example 61 | 5085 | 14100 |
| Example 62 | 63.4 | 4109.5 |
| Example 63 | 89.6 | 8357 |
| Example 64 | 43.9 | 2321 |
| Example 65 | 337 | >10000 |
| Example 66 | 186.5 | 2270 |
| Example 68 | 24.7 | - |
| Example 69 | 51.1 | 4331.2 |
| Example 70 | 20.3 | 2630.2 |
| Example 71 | 36.4 | 2875.1 |
| Example 72 | 14.2 | 957.2 |
| Example 73 | 39 | 1322.4 |
| Example 74 | 11.2 | 566.7 |
| Example 75 | 66.9 | 1561.1 |
| Example 76 | 57.9 | 6338 |
| Example 77 | 17.5 | 1768.2 |
| Example 78 | 94.6 | 9850.2 |
| Example 79 | 20.1 | 1645 |
| Example 80 | 3841 | >10000 |
| Example 81 | 63.4 | 4109.5 |
| Example 82 | 5883 | >20000 |
| Example 83 | 71.7 | >10000 |
| Example 84 | >3000 | >10000 |
| Example 85 | >3000 | >10000 |
| Example 86 | 38 | >10000 |
| Example 87 | 8.7 | 1078.9 |
| Example 88 | 1932.8 | >10000 |
| Example 89 | 12.5 | 1762 |
| Example 90 | >3000 | >10000 |
| Example 91 | >3000 | >10000 |
| Example 92 | 32 | 8048 |
| Example 93 | 36.3 | 7628.3 |
| Example 94 | 2868 | >10000 |
| Example 95 | 7.7 | 823.4 |
| Example 96 | 630 | >10000 |
| Example 97 | 98.6 | >10000 |
| Example 98 | 21.9 | 3159.3 |
| Example 100 | 34.6 | >10000 |
| Example 101 | 91.4 | >10000 |
| Example 102 | 19.9 | >10000 |
| Example 103 | 48.2 | >10000 |
| Example 104 | 71.1 | >10000 |
| Example 105 | 84.6 | >10000 |
| Example 106 | 94.2 | >10000 |
| Example 107 | 120.3 | >10000 |
| Example 108 | 21.8 | 1638.1 |
| Example 109 | 56.9 | >10000 |
| Example 110 | 18.9 | 1608.4 |
| Example 111 | 2512.3 | >10000 |
| Example 112 | 4.7 | 405.4 |

### II. Pharmacokinetic Test in Mice

### 1. Test Compounds

The test compounds are compounds selected from specific examples of the present invention.

### 2. Test Animals

ICR male mice N=3/group; original source: Zhejiang Vital River Laboratory Animal Technology Co., Ltd.

### 3. Drug Preparation and Administration

Single oral (PO) administration to ICR mice: The compound was weighed and dissolved with DMSO, a certain volume of polyethylene glycol 400 and water for injection (WFI) were added, and then a small volume of 1 mol/L hydrochloric acid solution was added to adjust to a clear solution. 3 mice were intragastrically administered after fasting overnight at a dosage of 10 mg/kg.

Single intravenous (IV) administration to ICR mice: The compound was weighed and dissolved with DMSO, a certain volume of polyethylene glycol 400 and WFI were added, and then a small volume of 1 mol/L hydrochloric acid solution was added to adjust to a clear solution. 3 mice were administered via tail vein injection after fasting overnight at a dosage of 3 mg/kg.

### 4. Sample Collection

Blood was collected from the dorsalis pedis vein at approximately 30 µL/time point, anticoagulated with dipotassium ethylenediaminetetraacetate and kept on ice until plasma was separated by centrifugation (4000 g/min; 5 min; 4°C) within 1 h. The blood sampling time points were 0.0833 (intravenous injection), 0.25, 0.5, 1, 2, 4, 6, 8, and 24 h. The samples were stored in a refrigerator at -20°C.

30 µL of plasma sample (10 µL sample + 20 µL blank plasma sample) and 200 µL ice-cold ACN containing internal standard were mixed, and then drawn into vortex for 30 s and centrifuged at 4000 g/min for 20 min. 100 µL of the supernatant was transferred to a 96-well plate, and then 200 µL of ultrapure water was added. After drawing into vortex for 30 s, 5 µL or 10 µL was injected into LC-MS/MS for analysis.

**Table 2: Pharmacokinetic Data**

| Example No. | Mode of Administration (Dosage) | AUCₗₐₛₜ(hr*ng/mL) | T_{1/2}(h) | Bioavailability (F,%) |
|---|---|---|---|---|
| **56** | IV (3mg/kg) | 34550 | 4.1 | |
| | PO (10mg/kg) | 88326 | 4.6 | 78 |
| **57** | IV (3mg/kg) | 23746 | 7.5 | |
| | PO (10mg/kg) | 55042 | 10.6 | 69 |
| **92** | IV (3mg/kg) | 5699 | 1.7 | |
| | PO (10mg/kg) | 31071 | 2 | 161 |

Although preferred examples have been described above, it is obvious to those skilled in the art that modifications may be made without departing from the present invention. Such modifications are considered to be possible variants encompassed within the scope of the present invention.

## Claims

1. A compound of formula (I), a pharmaceutically acceptable salt, an ester, a prodrug, a stereoisomer or an isotopic derivative thereof, 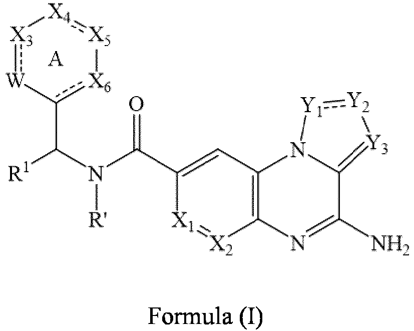
wherein W represents N or CR^{W};
wherein X₃ represents N or CR^{X3}; X₄ represents N or CR^{X4}; X₅ represents N or CR^{X5}; and X₆ represents N or CR^{X6};
wherein, when X₃ represents CR^{X3}, R^{X3} represents hydrogen, deuterium, C₁-C₆ alkyl, deuterated C₁-C₆ alkyl, C₂-C₆ alkenyl, C₂-C₆ alkynyl, halogen, -OR^{a}, -SR^{a}, -S(O)₂R^{a}, -S(O)R^{a}, -P(O)R^{a}R^{b}, -CN, -SF₅, -NR^{a}R^{b}, C₁-C₆ haloalkyl, C₁-C₆ haloalkoxy, hydroxy(C₁-C₆ alkyl) or C₃-C₁₀ cycloalkyl, C₆-C₁₀ cycloalkenyl, 4- to 10-membered heterocycloalkyl, 6- to 10-membered heterocycloalkenyl, C₆-C₁₀ aryl, 5- to 10-membered heteroaryl, wherein the said C₃-C₁₀ cycloalkyl, C₆-C₁₀ cycloalkenyl, 4- to 10-membered heterocycloalkyl, 6- to 10-membered heterocycloalkenyl, C₆-C₁₀ aryl and 5- to 10-membered heteroaryl can be furtherly substituted with 0-4 substituents selected from: deuterium, halogen, C₁-C₆ alkyl, C₂-C₆ alkenyl, C₂-C₆ alkynyl, C₃-C₁₀ cycloalkyl, -OR^{a}, oxo, hydroxy(C₁-C₆ alkyl), NR^{a}R^{b}, -CN, C₁-C₆ haloalkyl, C₁-C₆ haloalkoxy, -SO₃R^{a}, -SR^{a}, -SF₅, -C(O)R^{a}, -C(O)OR^{a}, -OC(O)R^{a}, OC(O)NR^{a}R^{b}, -NR^{a}COR^{b} or -CONR^{a}R^{b};
wherein, when X₄ represents CR^{X4}, R^{X4} represents hydrogen, deuterium, C₁-C₆ alkyl, deuterated C₁-C₆ alkyl, C₂-C₆ alkenyl, C₂-C₆ alkynyl, halogen, -OR^{a}, -SR^{a}, -S(O)₂R^{a}, -S(O)R^{a}, -P(O) R^{a}R^{b}, -CN, -SF₅, -NR^{a}R^{b}, C₁-C₆ haloalkyl, C₁-C₆ haloalkoxy, hydroxy(C₁-C₆ alkyl) or C₃-C₁₀ cycloalkyl, C₆-C₁₀ cycloalkenyl, 4- to 10-membered heterocycloalkyl, 6- to 10-membered heterocycloalkenyl, C₆-C₁₀ aryl, 5- to 10-membered heteroaryl, wherein the said C₃-C₁₀ cycloalkyl, C₆-C₁₀ cycloalkenyl, 4- to 10-membered heterocycloalkyl, 6- to 10-membered heterocycloalkenyl, C₆-C₁₀ aryl and 5- to 10-membered heteroaryl can be furtherly substituted with 0-4 substituents selected from: deuterium, halogen, C₁-C₆ alkyl, C₂-C₆ alkenyl, C₂-C₆ alkynyl, C₃-C₆ cycloalkyl, -OR^{a}, oxo, hydroxy(C₁-C₆ alkyl), NR^{a}R^{b}, -CN, C₁-C₆ haloalkyl, C₁-C₆ haloalkoxy, -SO₃R^{a}, -SR^{a}, -SF₅, -C(O)R^{a}, -C(O)OR^{a}, -OC(O)R^{a}, OC(O)NR^{a}R^{b}, -NR^{a}COR^{b} or -CONR^{a}R^{b};
wherein, when X₅ represents CR^{X5}, R^{X5} represents hydrogen, deuterium, C₁-C₆ alkyl, deuterated C₁-C₆ alkyl, C₂-C₆ alkenyl, C₂-C₆ alkynyl, halogen, -OR^{a}, -SR^{a}, -S(O)₂R^{a}, -S(O)R^{a}, -P(O) R^{a}R^{b}, -CN, -SF₅, -NR^{a}R^{b}, C₁-C₆ haloalkyl, C₁-C₆ haloalkoxy, hydroxy(C₁-C₆ alkyl) or C₃-C₁₀ cycloalkyl, C₆-C₁₀ cycloalkenyl, 4- to 10-membered heterocycloalkyl, 6- to 10-membered heterocycloalkenyl, C₆-C₁₀ aryl, 5- to 10-membered heteroaryl, wherein the said C₃-C₁₀ cycloalkyl, C₆-C₁₀ cycloalkenyl, 4- to 10-membered heterocycloalkyl, 6- to 10-membered heterocycloalkenyl, C₆-C₁₀ aryl and 5- to 10-membered heteroaryl can be furtherly substituted with 0-4 substituents selected from: deuterium, halogen, C₁-C₆ alkyl, C₂-C₆ alkenyl, C₂-C₆ alkynyl, C₃-C₁₀ cycloalkyl, -OR^{a}, oxo, hydroxy(C₁-C₆ alkyl), NR^{a}R^{b}, -CN, C₁-C₆ haloalkyl, C₁-C₆ haloalkoxy, -SO₃R^{a}, -SR^{a}, -SF₅, -C(O)R^{a}, -C(O)OR^{a}, -OC(O)R^{a}, OC(O)NR^{a}R^{b}, -NR^{a}COR^{b} or -CONR^{a}R^{b};
wherein, when X₆ represents CR^{X6}, R^{X6} represents hydrogen, deuterium, C₁-C₆ alkyl, deuterated C₁-C₆ alkyl, C₂-C₆ alkenyl, C₂-C₆ alkynyl, halogen, -OR^{a}, -SR^{a}, -S(O)₂R^{a}, -S(O)R^{a}, -P(O) R^{a}R^{b}, -CN, -SF₅, -NR^{a}R^{b}, C₁-C₆ haloalkyl, C₁-C₆ haloalkoxy, hydroxy(C₁-C₆ alkyl) or C₃-C₁₀ cycloalkyl, C₆-C₁₀ cycloalkenyl, 4- to 10-membered heterocycloalkyl, 6- to 10-membered heterocycloalkenyl, C₆-C₁₀ aryl, 5- to 10-membered heteroaryl, wherein the said C₃-C₁₀ cycloalkyl, C₆-C₁₀ cycloalkenyl, 4- to 10-membered heterocycloalkyl, 6- to 10-membered heterocycloalkenyl, C₆-C₁₀ aryl and 5- to 10-membered heteroaryl can be furtherly substituted with 0-4 substituents selected from: deuterium, halogen, C₁-C₆ alkyl, C₂-C₆ alkenyl, C₂-C₆ alkynyl, C₃-C₁₀ cycloalkyl, -OR^{a}, oxo, hydroxy(C₁-C₆ alkyl), NR^{a}R^{b}, -CN, C₁-C₆ haloalkyl, C₁-C₆ haloalkoxy, -SO₃R^{a}, -SR^{a}, -SF₅, -C(O)R^{a}, -C(O)OR^{a}, -OC(O)R^{a}, OC(O)NR^{a}R^{b}, -NR^{a}COR^{b} or -CONR^{a}R^{b};
wherein, the ring A may further optionally fuse a 5- to 6-membered saturated or unsaturated ring at the chemical bond between X₃ and X₄, and may further contain 0-3 heteroatoms selected from O, N, and S; further, the ring may be substituted with 0-3 substitutions selected from the group consisting of: deuterium, C₁-C₆ alkyl, deuterated C₁-C₆ alkyl, C₂-C₆ alkenyl, C₂-C₆ alkynyl, halogen, -OR^{a}, -SR^{a}, -S(O)₂R^{a}, -S(O)R^{a}, -P(O)R^{a}R^{b}, -CN, -SF₅, -NR^{a}R^{b}, C₁-C₆ haloalkyl, C₁-C₆ haloalkoxy, hydroxy(C₁-C₆ alkyl), or C₃-C₁₀ cycloalkyl, C₆-C₁₀ cycloalkenyl, 4- to 10-membered heterocycloalkyl, 6- to 10-membered heterocycloalkenyl, C₆-C₁₀ aryl, or 5-to 10-membered heteroaryl, wherein the said C₃-C₁₀ cycloalkyl, C₆-C₁₀ cycloalkenyl, 4- to 10-membered heterocycloalkyl, 6- to 10-membered heterocycloalkenyl, C₆-C₁₀ aryl, or 5- to 10-membered heteroaryl can be furtherly substituted with 0-4 substituents selected from the group consisting of: deuterated, halogen, C₁-C₆ alkyl, C₂-C₆ alkenyl, C₂-C₆ alkynyl, C₃-C₁₀ cycloalkyl, -OR^{a}, oxo, hydroxy(C₁-C₆ alkyl), NR^{a}R^{b}, -CN, C₁-C₆ haloalkyl, C₁-C₆ haloalkoxy, -SO₅R^{a}, -SR^{a}, -SF₅, -C(O)R^{a}, -C(O)OR^{a}, -OC(O)R^{a}, -OC(O)NR^{a}R^{b}, -NR^{a}COR^{b}, and -CONR^{a}R^{b};
wherein, the ring A may further optionally fuse a 5- to 6-membered saturated or unsaturated ring at the chemical bond between X₄ and X₅, and may further contain 0-3 heteroatoms selected from O, N, and S; further, the ring may be substituted with 0-3 substitutions selected from the group consisting of: deuterium, C₁-C₆ alkyl, deuterated C₁-C₆ alkyl, C₂-C₆ alkenyl, C₂-C₆ alkynyl, halogen, -OR^{a}, -SR^{a}, -S(O)₂R^{a}, -S(O)R^{a}, -P(O)R^{a}R^{b}, -CN, -SF₅, -NR^{a}R^{b}, C₁-C₆ haloalkyl, C₁-C₆ haloalkoxy, hydroxy(C₁-C₆ alkyl), or C₃-C₁₀ cycloalkyl, C₆-C₁₀ cycloalkenyl, 4- to 10-membered heterocycloalkyl, 6- to 10-membered heterocycloalkenyl, C₆-C₁₀ aryl, or 5-to 10-membered heteroaryl, wherein the said C₃-C₁₀ cycloalkyl, C₆-C₁₀ cycloalkenyl, 4- to 10-membered heterocycloalkyl, 6- to 10-membered heterocycloalkenyl, C₆-C₁₀ aryl, or 5- to 10-membered heteroaryl can be furtherly substituted with 0-4 substituents selected from the group consisting of: deuterated, halogen, C₁-C₆ alkyl, C₂-C₆ alkenyl, C₂-C₆ alkynyl, C₃-C₁₀ cycloalkyl, -OR^{a}, oxo, hydroxy(C₁-C₆ alkyl), NR^{a}R^{b}, -CN, C₁-C₆ haloalkyl, C₁-C₆ haloalkoxy, -SO₄R^{a}, -SR^{a}, -SF₅, -C(O)R^{a}, -C(O)OR^{a}, -OC(O)R^{a}, OC(O)NR^{a}R^{b}, -NR^{a}COR^{b}, and -CONR^{a}R^{b};
wherein, the ring A may further optionally fuse a 5- to 6-membered saturated or unsaturated ring at the chemical bond between X₅ and X₆, and may further contain 0-3 heteroatoms selected from O, N, and S; further, the ring may be substituted with 0-3 substitutions selected from the group consisting of: deuterium, C₁-C₆ alkyl, deuterated C₁-C₆ alkyl, C₂-C₆ alkenyl, C₂-C₆ alkynyl, halogen, -OR^{a}, -SR^{a}, -S(O)₂R^{a}, -S(O)R^{a}, -P(O)R^{a}R^{b}, -CN, -SF₅, -NR^{a}R^{b}, C₁-C₆ haloalkyl, C₁-C₆ haloalkoxy, hydroxy(C₁-C₆ alkyl), or C₃-C₁₀ cycloalkyl, C₆-C₁₀ cycloalkenyl, 4- to 10-membered heterocycloalkyl, 6- to 10-membered heterocycloalkenyl, C₆-C₁₀ aryl, or 5-to 10-membered heteroaryl substituted with 0-4 substituents selected from the group consisting of: deuterated, halogen, C₁-C₆ alkyl, C₂-C₆ alkenyl, C₂-C₆ alkynyl, C₃-C₁₀ cycloalkyl, -OR^{a}, oxo, hydroxy(C₁-C₆ alkyl), NR^{a}R^{b}, -CN, C₁-C₆ haloalkyl, C₁-C₆ haloalkoxy, -SO₅R^{a}, -SR^{a}, -SF₅, - C(O)R^{a}, -C(O)OR^{a}, -OC(O)R^{a}, OC(O)NR^{a}R^{b}, -NR^{a}COR^{b}, and -CONR^{a}R^{b};
wherein, R' represents C₁-C₆ alkyl, C₃-C₁₀ cycloalkyl, 4- to 10-membered heterocycloalkyl, C₆-C₁₀ aryl, or 5- to 10-membered heteroaryl, wherein the said C₁-C₆ alkyl, C₃-C₁₀ cycloalkyl, 4- to 10-membered heterocycloalkyl, C₆-C₁₀ aryl, or 5- to 10-membered heteroaryl can be furtherly substituted with 0-3 substituents optionally selected from the group consisting of: deuterated, halogen, C₁-C₆ alkyl, C₂-C₆ alkenyl, C₂-C₆ alkynyl, C₃-C₆ cycloalkyl, 3- to 6-membered saturated or unsaturated aliphatic monoheterocyclic group, -OR^{a}, oxo, hydroxy(C₁-C₆ alkyl), NR^{a}R^{b}, -CN, C₁-C₆ haloalkyl, C₁-C₆ haloalkoxy, -SO₃R^{a}, -SR^{a}, -S(O)₂R^{a}, -S(O)R^{a}, -SF₅, - C(O)R^{a}, -C(O)OR^{a}, -OC(O)R^{a}, -OC(O)NR^{a}R^{b}, -NR^{a}COR^{b}, and -CONR^{a}R^{b};
preferably, R' represents -CHR²R³;
wherein, R¹ represents hydrogen, deuterium, halogen, C₁-C₆ alkyl, deuterated C₁-C₆ alkyl, C₁-C₆ haloalkyl, hydroxy(C₁-C₆ alkyl), or C₃-C₁₀ cycloalkyl;
wherein, R² and R³ respectively represent hydrogen, deuterium, -OR^{a}, halogen, -CN, C₁-C₆ alkyl, C₁-C₆ haloalkyl, C₁-C₆ haloalkoxy, hydroxy(C₁-C₆ alkyl), or C₃-C₁₀ cycloalkyl, 4- to 10-membered heterocycloalkyl, C₆-C₁₀ aryl, or 5- to 10-membered heteroaryl, wherein the said C₃-C₁₀ cycloalkyl, 4- to 10-membered heterocycloalkyl, C₆-C₁₀ aryl, or 5- to 10-membered heteroaryl can be furtherly substituted with 0-3 substituents optionally selected from the group consisting of: deuterated, halogen, C₁-C₆ alkyl, C₂-C₆ alkenyl, C₂-C₆ alkynyl, C₃-C₆ cycloalkyl, -OR^{a}, oxo, hydroxy(C₁-C₆ alkyl), NR^{a}R^{b}, -CN, C₁-C₆ haloalkyl, C₁-C₆ haloalkoxy, -SO₃R^{a}, -SR^{a}, -S(O)₂R^{a}, -S(O)R^{a}, -SF₅, -C(O)R^{a}, -C(O)OR^{a}, -OC(O)R^{a}, -OC(O)NR^{a}R^{b}, -NR^{a}COR^{b}, and - CONR^{a}R^{b};
wherein, when W represents CR^{W}, R^{W} is selected from hydrogen, deuterium, C₁-C₆ alkyl, deuterated C₁-C₆ alkyl, halogen, -OR^{a}, -SR^{a}, -S(O)₂R^{a}, -S(O)R^{a}, -CN, -SF₅, -NR^{a}R^{b}, C₁-C₆ haloalkyl, hydroxy(C₁-C₆ alkyl), and C₁-C₆ haloalkoxy;
wherein X₁ represents N or CR^{X1};
wherein X₂ represents N or CR^{X2};
wherein Y₁ represents CR^{Y1}R^{Y1'}, NR^{Y1}, O, S, or Se;
wherein Y₂ represents CR^{Y2}R^{Y2'}, NR^{Y2}, O, S, or Se;
wherein Y₃ represents CR^{Y3}R^{Y3'}**,** NR^{Y3}, O, S, or Se;
wherein R^{X1} and R^{X2} respectively represent hydrogen, deuterium, C₁-C₆ alkyl, deuterated C₁-C₆ alkyl, C₁-C₆ haloalkyl, C₁-C₆ haloalkoxy, hydroxy(C₁-C₆ alkyl), C₂-C₆ alkenyl, C₂-C₆ alkynyl, - OR^{a}, -SR^{a}, -S(O)₂R^{a}, -S(O)R^{a}, -CN, -OC(O)R^{a}, -OCONR^{a}R^{b}, halogen, -SO₃R^{a}, -NR^{a}R^{b}, or -SF₅; wherein R^{Y1}, R^{Y1'}, R^{Y2}, R^{Y2'}, R^{Y3}, and R^{Y3'} respectively represent absent, hydrogen, deuterium, C₁-C₆ alkyl, deuterated C₁-C₆ alkyl, C₁-C₆ haloalkyl, C₁-C₆ haloalkoxy, hydroxy(C₁-C₆ alkyl), C₂-C₆ alkenyl, C₂-C₆ alkynyl, C₃-C₆ cycloalkyl, -OR^{a}, -SR^{a}, -S(O)₂R^{a}, -S(O)R^{a}, -CN, -OC(O)R^{a}, -OCONR^{a}R^{b}, halogen, -SO₃R^{a}, -NR^{a}R^{b}, or -SF₅;
wherein, -̅ -̅ -̅ represents a single bond or a double bond; and
wherein R^{a} and R^{b} respectively represent hydrogen, deuterium, halogen, C₁-C₆ alkyl, C₃-C₆ cycloalkyl, C₁-C₆ haloalkyl, or R^{a} and R^{b} can be taken together with the atom to which they are attached form a 3- to 14-membered saturated or unsaturated ring , wherein the ring may optionally contain 0-2 heteroatoms selected from O, S, and N.

2. The compound, the pharmaceutically acceptable salt, the ester, the prodrug, the stereoisomer or the isotopic derivative thereof according to claim 1, the structure of formula (II) is provided below: 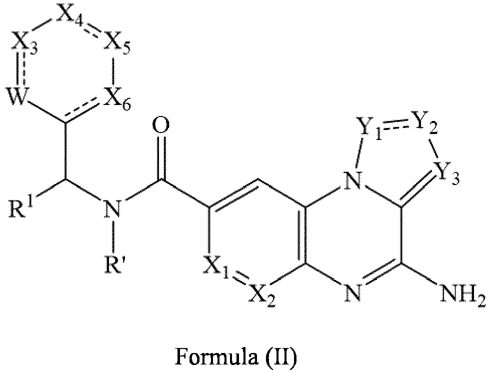
wherein W represents N or CR^{W};
wherein X₃ represents N or CR^{X3}; X₄ represents N or CR^{X4}; X₅ represents N or CR^{X5}; and X₆ represents N or CR^{X6};
wherein, when X₃ represents CR^{X3}, R^{X3} represents hydrogen, deuterium, C₁-C₆ alkyl, deuterated C₁-C₆ alkyl, C₂-C₆ alkenyl, C₂-C₆ alkynyl, halogen, -OR^{a}, -SR^{a}, -S(O)₂R^{a}, -S(O)R^{a}, -P(O) R^{a}R^{b}, -CN, -SF₅, -NR^{a}R^{b}, C₁-C₆ haloalkyl, C₁-C₆ haloalkoxy, hydroxy(C₁-C₆ alkyl) or C₃-C₁₀ cycloalkyl, C₆-C₁₀ cycloalkenyl, 4- to 10-membered heterocycloalkyl, 6- to 10-membered heterocycloalkenyl, C₆-C₁₀ aryl, 5- to 10-membered heteroaryl, wherein the said C₃-C₁₀ cycloalkyl, C₆-C₁₀ cycloalkenyl, 4- to 10-membered heterocycloalkyl, 6- to 10-membered heterocycloalkenyl, C₆-C₁₀ aryl and 5- to 10-membered heteroaryl can be furtherly substituted with 0-4 substituents selected from: deuterium, halogen, C₁-C₆ alkyl, C₂-C₆ alkenyl, C₂-C₆ alkynyl, C₃-C₁₀ cycloalkyl, -OR^{a}, oxo, hydroxy(C₁-C₆ alkyl), NR^{a}R^{b}, -CN, C₁-C₆ haloalkyl, C₁-C₆ haloalkoxy, -SO₃R^{a}, -SR^{a}, -SF₅, -C(O)R^{a}, -C(O)OR^{a}, -OC(O)R^{a}, OC(O)NR^{a}R^{b}, -NR^{a}COR^{b} or -CONR^{a}R^{b};
wherein, when X₄ represents CR^{X4}, R^{X4} represents hydrogen, deuterium, C₁-C₆ alkyl, deuterated C₁-C₆ alkyl, C₂-C₆ alkenyl, C₂-C₆ alkynyl, halogen, -OR^{a}, -SR^{a}, -S(O)₂R^{a}, -S(O)R^{a}, -P(O) R^{a}R^{b}, -CN, -SF₅, -NR^{a}R^{b}, C₁-C₆ haloalkyl, C₁-C₆ haloalkoxy, hydroxy(C₁-C₆ alkyl) or C₃-C₁₀ cycloalkyl, C₆-C₁₀ cycloalkenyl, 4- to 10-membered heterocycloalkyl, 6- to 10-membered heterocycloalkenyl, C₆-C₁₀ aryl, 5- to 10-membered heteroaryl, wherein the said C₃-C₁₀ cycloalkyl, C₆-C₁₀ cycloalkenyl, 4- to 10-membered heterocycloalkyl, 6- to 10-membered heterocycloalkenyl, C₆-C₁₀ aryl and 5- to 10-membered heteroaryl can be furtherly substituted with 0-4 substituents selected from: deuterium, halogen, C₁-C₆ alkyl, C₂-C₆ alkenyl, C₂-C₆ alkynyl, C₃-C₆ cycloalkyl, -OR^{a}, oxo, hydroxy(C₁-C₆ alkyl), NR^{a}R^{b}, -CN, C₁-C₆ haloalkyl, C₁-C₆ haloalkoxy, -SO₃R^{a}, -SR^{a}, -SF₅, -C(O)R^{a}, -C(O)OR^{a}, -OC(O)R^{a}, OC(O)NR^{a}R^{b}, -NR^{a}COR^{b} or -CONR^{a}R^{b};
wherein, when X₅ represents CR^{X5}, R^{X5} represents hydrogen, deuterium, C₁-C₆ alkyl, deuterated C₁-C₆ alkyl, C₂-C₆ alkenyl, C₂-C₆ alkynyl, halogen, -OR^{a}, -SR^{a}, -S(O)₂R^{a}, -S(O)R^{a}, -P(O) R^{a}R^{b}, -CN, -SF₅, -NR^{a}R^{b}, C₁-C₆ haloalkyl, C₁-C₆ haloalkoxy, hydroxy(C₁-C₆ alkyl) or C₃-C₁₀ cycloalkyl, C₆-C₁₀ cycloalkenyl, 4- to 10-membered heterocycloalkyl, 6- to 10-membered heterocycloalkenyl, C₆-C₁₀ aryl, 5- to 10-membered heteroaryl, wherein the said C₃-C₁₀ cycloalkyl, C₆-C₁₀ cycloalkenyl, 4- to 10-membered heterocycloalkyl, 6- to 10-membered heterocycloalkenyl, C₆-C₁₀ aryl and 5- to 10-membered heteroaryl can be furtherly substituted with 0-4 substituents selected from: deuterium, halogen, C₁-C₆ alkyl, C₂-C₆ alkenyl, C₂-C₆ alkynyl, C₃-C₁₀ cycloalkyl, -OR^{a}, oxo, hydroxy(C₁-C₆ alkyl), NR^{a}R^{b}, -CN, C₁-C₆ haloalkyl, C₁-C₆ haloalkoxy, -SO₃R^{a}, -SR^{a}, -SF₅, -C(O)R^{a}, -C(O)OR^{a}, -OC(O)R^{a}, OC(O)NR^{a}R^{b}, -NR^{a}COR^{b} or -CONR^{a}R^{b};
wherein, when X₆ represents CR^{X6}, R^{X6} represents hydrogen, deuterium, C₁-C₆ alkyl, deuterated C₁-C₆ alkyl, C₂-C₆ alkenyl, C₂-C₆ alkynyl, halogen, -OR^{a}, -SR^{a}, -S(O)₂R^{a}, -S(O)R^{a}, -P(O) R^{a}R^{b}, -CN, -SF₅, -NR^{a}R^{b}, C₁-C₆ haloalkyl, C₁-C₆ haloalkoxy, hydroxy(C₁-C₆ alkyl) or C₃-C₁₀ cycloalkyl, C₆-C₁₀ cycloalkenyl, 4- to 10-membered heterocycloalkyl, 6- to 10-membered heterocycloalkenyl, C₆-C₁₀ aryl, 5- to 10-membered heteroaryl, wherein the said C₃-C₁₀ cycloalkyl, C₆-C₁₀ cycloalkenyl, 4- to 10-membered heterocycloalkyl, 6- to 10-membered heterocycloalkenyl, C₆-C₁₀ aryl and 5- to 10-membered heteroaryl can be furtherly substituted with 0-4 substituents selected from: deuterium, halogen, C₁-C₆ alkyl, C₂-C₆ alkenyl, C₂-C₆ alkynyl, C₃-C₁₀ cycloalkyl, -OR^{a}, oxo, hydroxy(C₁-C₆ alkyl), NR^{a}R^{b}, -CN, C₁-C₆ haloalkyl, C₁-C₆ haloalkoxy, -SO₃R^{a}, -SR^{a}, -SF₅, -C(O)R^{a}, -C(O)OR^{a}, -OC(O)R^{a}, OC(O)NR^{a}R^{b}, -NR^{a}COR^{b} or -CONR^{a}R^{b};
wherein, R' represents C₁-C₆ alkyl, C₃-C₁₀ cycloalkyl, 4- to 10-membered heterocycloalkyl, C₆-C₁₀ aryl, or 5- to 10-membered heteroaryl, wherein the said C₁-C₆ alkyl, C₃-C₁₀ cycloalkyl, 4- to 10-membered heterocycloalkyl, C₆-C₁₀ aryl, or 5- to 10-membered heteroaryl can be furtherly substituted with 0-3 substituents optionally selected from the group consisting of: deuterated, halogen, C₁-C₆ alkyl, C₂-C₆ alkenyl, C₂-C₆ alkynyl, C₃-C₆ cycloalkyl, 3- to 6-membered saturated or unsaturated aliphatic monoheterocyclic group, -OR^{a}, oxo, hydroxy(C₁-C₆ alkyl), NR^{a}R^{b}, -CN, C₁-C₆ haloalkyl, C₁-C₆ haloalkoxy, -SO₃R^{a}, -SR^{a}, -S(O)₂R^{a}, -S(O)R^{a}, -SF₅, - C(O)R^{a}, -C(O)OR^{a}, -OC(O)R^{a}, -OC(O)NR^{a}R^{b}, -NR^{a}COR^{b}, and -CONR^{a}R^{b};
preferably, R' represents -CHR²R³;
wherein, R¹ represents hydrogen, deuterium, halogen, C₁-C₆ alkyl, deuterated C₁-C₆ alkyl, C₁-C₆ haloalkyl, hydroxy(C₁-C₆ alkyl), or C₃-C₁₀ cycloalkyl;
wherein, R² and R³ respectively represent hydrogen, deuterium, -OR^{a}, halogen, -CN, -C₁-C₆ alkyl, C₁-C₆ haloalkyl, C₁-C₆ haloalkoxy, hydroxy(C₁-C₆ alkyl) or C₃-C₁₀ cycloalkyl, 4- to 10-membered heterocycloalkyl, C₆-C₁₀ aryl, or 5- to 10-membered heteroaryl, wherein the said C₃-C₁₀ cycloalkyl, 4- to 10-membered heterocycloalkyl, C₆-C₁₀ aryl, or 5- to 10-membered heteroaryl can be furtherly substituted with 0-3 substituents optionally selected from the group consisting of: deuterated, halogen, C₁-C₆ alkyl, C₂-C₆ alkenyl, C₂-C₆ alkynyl, C₃-C₆ cycloalkyl, -OR^{a}, oxo, hydroxy(C₁-C₆ alkyl), NR^{a}R^{b}, -CN, C₁-C₆ haloalkyl, C₁-C₆ haloalkoxy, -SO₃R^{a}, -SR^{a}, -S(O)₂R^{a}, -S(O)R^{a}, -SF₅, -C(O)R^{a}, -C(O)OR^{a}, -OC(O)R^{a}, -OC(O)NR^{a}R^{b}, -NR^{a}COR^{b} and - CONR^{a}R^{b};
wherein, when W represents CR^{W}, R^{W} is selected from hydrogen, deuterium, C₁-C₆ alkyl, halogen, -OR^{a}, -SR^{a}, -S(O)₂R^{a}, -S(O)R^{a}, -CN, -SF₅, -NR^{a}R^{b}, C₁-C₆ haloalkyl, hydroxy(C₁-C₆ alkyl), and C₁-C₆ haloalkoxy;
wherein X₁ represents N or CR^{X1};
wherein X₂ represents N or CR^{X2};
wherein Y₁ represents CR^{Y1}R^{Y1'}, NR^{Y1}, O, S, or Se;
wherein Y₂ represents CR^{Y2}R^{Y2'}, NR^{Y2}, O, S, or Se;
wherein Y₃ represents CR^{Y3}R^{Y3'}, NR^{Y3}, O, S, or Se;
wherein R^{X1} and R^{X2} respectively represent hydrogen, deuterium, C₁-C₆ alkyl, deuterated C₁-C₆ alkyl, C₁-C₆ haloalkyl, C₁-C₆ haloalkoxy, hydroxy(C₁-C₆ alkyl), C₂-C₆ alkenyl, C₂-C₆ alkynyl, - OR^{a}, -SR^{a}, -S(O)₂R^{a}, -S(O)R^{a}, -CN, -OC(O)R^{a}, -OCONR^{a}R^{b}, halogen, -SO₃R^{a}, -NR^{a}R^{b}, or -SF₅; wherein R^{Y1}, R^{Y1'}, R^{Y2}, R^{Y2'}, R^{Y3}, and R^{Y3'} respectively represent absent, hydrogen, deuterium, C₁-C₆ alkyl, deuterated C₁-C₆ alkyl, C₁-C₆ haloalkyl, C₁-C₆ haloalkoxy, hydroxy(C₁-C₆ alkyl), C₂-C₆ alkenyl, C₂-C₆ alkynyl, C₃-C₆ cycloalkyl, -OR^{a}, -SR^{a}, -S(O)₂R^{a}, -S(O)R^{a}, -CN, -OC(O)R^{a}, -OCONR^{a}R^{b}, halogen, -SO₃R^{a}, -NR^{a}R^{b}, or -SF₅;
wherein, -̅ -̅ -̅ represents a single bond or a double bond; and
wherein R^{a} and R^{b} respectively represent hydrogen, deuterium, halogen, C₁-C₆ alkyl, C₃-C₆ cycloalkyl, C₁-C₆ haloalkyl, or R^{a} and R^{b} can be taken together with the atom to which they are attached form a 3- to 14-membered saturated or unsaturated ring , wherein the ring may optionally contain 0-2 heteroatoms selected from O, S, and N.

3. The compound, the pharmaceutically acceptable salt, the ester, the prodrug, the stereoisomer or the isotopic derivative thereof according to claim 1 or 2, wherein W represents CH or N.

4. The compound, the pharmaceutically acceptable salt, the ester, the prodrug, the stereoisomer or the isotopic derivative thereof according to any one of claims 1 to 3, wherein X₁ represents CR^{X1} or N, wherein R^{X1} represents hydrogen, deuterium, halogen, -CN, C₁-C₆ alkyl, deuterated C₁-C₆ alkyl, C₁-C₆ alkoxy, or C₁-C₆ haloalkyl.

5. The compound, the pharmaceutically acceptable salt, the ester, the prodrug, the stereoisomer or the isotopic derivative thereof according to any one of claims 1 to 4, wherein X₂ represents CH or CD.

6. The compound, the pharmaceutically acceptable salt, the ester, the prodrug, the stereoisomer or the isotopic derivative thereof according to any one of claims 1 to 5, wherein X₃ represents CH, CD, or N.

7. The compound, the pharmaceutically acceptable salt, the ester, the prodrug, the stereoisomer or the isotopic derivative thereof according to any one of claims 1 to 6, wherein X₄ represents CR^{X4} or N, wherein R^{X4} represents hydrogen, deuterium, C₁-C₆ alkyl, deuterated C₁-C₆ alkyl, C₁-C₆ alkoxy, C₁-C₆ haloalkyl, C₁-C₆ haloalkoxy, halogen, SF₅, SO₃R^{a}, -SR^{a}, cyano, or C₃-C₁₀ cycloalkyl, C₆-C₁₀ cycloalkenyl, 4-10 membered heterocycloalkyl, 6- to 10-membered heterocycloalkenyl, C₆-C₁₀ aryl, or 5- to 10-membered heteroaryl, wherein the said C₃-C₁₀ cycloalkyl, C₆-C₁₀ cycloalkenyl, 4-10 membered heterocycloalkyl, 6- to 10-membered heterocycloalkenyl, C₆-C₁₀ aryl, or 5- to 10-membered heteroaryl can be furtherly substituted with 0-4 substituents selected from the group consisting of: deuterated, halogen, C₁-C₆ alkyl, C₂-C₆ alkenyl, C₂-C₆ alkynyl, C₃-C₆ cycloalkyl, -OR^{a}, oxo, hydroxy(C₁-C₆ alkyl), NR^{a}R^{b}, - P(O)R^{a}R^{b},-CN, C₁-C₆ haloalkyl, C₁-C₆ haloalkoxy, -SO₃R^{a}, -SR^{a}, -SF₅, -C(O)R^{a}, -C(O)OR^{a}, - OC(O)R^{a}, -OC(O)NR^{a}R^{b}, -NR^{a}COR^{b}, and -CONR^{a}R^{b}.

8. The compound, the pharmaceutically acceptable salt, the ester, the prodrug, the stereoisomer or the isotopic derivative thereof according to any one of claims 1 to 7, wherein X₅ represents CR^{X5} or N, wherein R^{X5} represents hydrogen, deuterium, C₁-C₆ alkyl, C₁-C₆ alkoxy, C₁-C₆ haloalkyl, C₁-C₆ haloalkoxy, halogen, SF₅, or cyano.

9. The compound, the pharmaceutically acceptable salt, the ester, the prodrug, the stereoisomer or the isotopic derivative thereof according to any one of claims 1 to 8, wherein X₆ represents CH, CD, or N.

10. The compound, the pharmaceutically acceptable salt, the ester, the prodrug, the stereoisomer or the isotopic derivative thereof according to any one of claims 1 to 9, wherein the chemical bond between Y₁ and Y₂ is a double bond.

11. The compound, the pharmaceutically acceptable salt, the ester, the prodrug, the stereoisomer or the isotopic derivative thereof according to claim 10, wherein Y₁ represents CR^{Y1}, wherein R^{Y1} represents hydrogen, deuterium, C₁-C₆ alkyl, deuterated C₁-C₆ alkyl, C₃-C₆ cycloalkyl, C₁-C₆ alkoxy, C₁-C₆ haloalkyl, C₁-C₆ haloalkoxy, halogen, SF₅, -S(O)₂CH₃, or cyano.

12. The compound, the pharmaceutically acceptable salt, the ester, the prodrug, the stereoisomer or the isotopic derivative thereof according to claim 11, wherein Y₂ represents N.

13. The compound, the pharmaceutically acceptable salt, the ester, the prodrug, the stereoisomer or the isotopic derivative thereof according to any one of claims 1 to 12, wherein Y₃ represents CH or CD.

14. The compound, the pharmaceutically acceptable salt, the ester, the prodrug, the stereoisomer or the isotopic derivative thereof according to any one of claims 1 to 13, wherein R' represents - CHR²R³, wherein R² and R³ respectively represent hydrogen, deuterium, C₁-C₆ alkyl, or C₃-C₁₀ cycloalkyl, 4- to 10- membered heterocycloalkyl, C₆-C₁₀ aryl, or 5- to 10- membered heteroaryl substituted, wherein the said C₃-C₁₀ cycloalkyl, 4- to 10- membered heterocycloalkyl, C₆-C₁₀ aryl, or 5- to 10- membered heteroaryl can be furtherly substituted with 0-3 substituents selected from the group consisting of: halogen, C₁-C₆ alkyl, C₂-C₆ alkenyl, C₂-C₆ alkynyl, C₃-C₆ cycloalkyl, -OR^{a}, oxo, hydroxy(C₁-C₆ alkyl), NR^{a}R^{b}, -CN, C₁-C₆ haloalkyl, C₁-C₆ haloalkoxy, - SO₃R^{a}, -SR^{a}, -S(O)₂R^{a}, -S(O)R^{a}, -SF₅, -C(O)R^{a}, -C(O)OR^{a}, -OC(O)R^{a}, -OC(O)NR^{a}R^{b}, - NR^{a}COR^{b}, and -CONR^{a}R^{b}.

15. The compound, the pharmaceutically acceptable salt, the ester, the prodrug, the stereoisomer or the isotopic derivative thereof according to claim 14, wherein R' represents - CHR²R³, wherein, R² represents hydrogen, deuterium, or C₁-C₆ alkyl; and R³ represents hydrogen, C₁-C₆ alkyl, or C₃-C₁₀ cycloalkyl, 4- to 10- membered heterocycloalkyl, C₆-C₁₀ aryl, or 5- to 10- membered heteroaryl, wherein the said C₃-C₁₀ cycloalkyl, 4- to 10- membered heterocycloalkyl, C₆-C₁₀ aryl, or 5- to 10- membered heteroaryl can be furtherly substituted with 0-3 substituents selected from the group consisting of: halogen, C₁-C₆ alkyl, C₂-C₆ alkenyl, C₂-C₆ alkynyl, C₃-C₆ cycloalkyl, -OR^{a}, oxo, hydroxy(C₁-C₆ alkyl), NR^{a}R^{b}, -CN, C₁-C₆ haloalkyl, C₁-C₆ haloalkoxy, -SO₃R^{a}, -SR^{a}, -S(O)₂R^{a}, -S(O)R^{a}, -SF₅, -C(O)R^{a}, -C(O)OR^{a}, - OC(O)R^{a}, -OC(O)NR^{a}R^{b}, -NR^{a}COR^{b}, and -CONR^{a}R^{b}.

16. The compound, the pharmaceutically acceptable salt, the ester, the prodrug, the stereoisomer, or the isotopic derivative thereof according to claim 15, wherein R' represents -CHR²R³, wherein, R² represents hydrogen, deuterium, or C₁-C₆ alkyl; and R³ represents C₃-C₁₀ cycloalkyl, 4- to 10- membered heterocycloalkyl, C₆-C₁₀ aryl, or 5- to 10- membered heteroaryl, wherein the said C₃-C₁₀ cycloalkyl, 4- to 10- membered heterocycloalkyl, C₆-C₁₀ aryl, or 5- to 10-membered heteroaryl can be furtherly substituted with 0-3 substituents selected from the group consisting of: deuterated, halogen, C₁-C₆ alkyl, C₂-C₆ alkenyl, C₂-C₆ alkynyl, C₃-C₆ cycloalkyl, -OR^{a}, oxo, hydroxy(C₁-C₆ alkyl), NR^{a}R^{b}, S(O)₂R^{a}, -S(O)R^{a}, -CN, C₁-C₆ haloalkyl, C₁-C₆ haloalkoxy, -SO₃R^{a}, -SR^{a}, -S(O)₂R^{a}, -S(O)R^{a}, -SF₅, -C(O)R^{a}, -C(O)OR^{a}, -OC(O)R^{a}, - OC(O)NR^{a}R^{b}, -NR^{a}COR^{b}, and -CONR^{a}R^{b}.

17. The compound, the pharmaceutically acceptable salt, the ester, the prodrug, the stereoisomer, or the isotopic derivative thereof according to claim 16, wherein R' represents -CHR²R³, wherein, R² represents hydrogen, deuterium, or C₁-C₆ alkyl; and R³ represents the following groups: 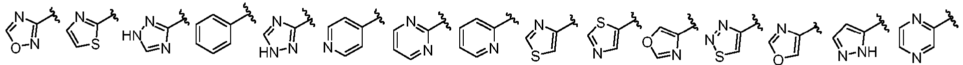 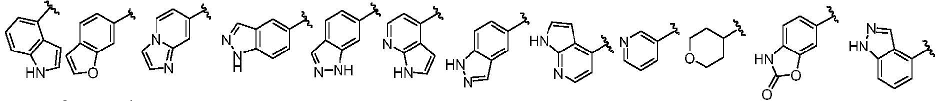 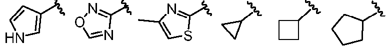 wherein, the above groups can be furtherly substituted with 0-3 substituents selected from the group consisting of: deuterated, halogen, C₁-C₆ alkyl, C₂-C₆ alkenyl, C₂-C₆ alkynyl, C₃-C₆ cycloalkyl, -OR^{a}, oxo, hydroxy(C₁-C₆ alkyl), NR^{a}R^{b}, S(O)₂R^{a}, -S(O)R^{a}, -CN, C₁-C₆ haloalkyl, C₁-C₆ haloalkoxy, -SO₃R^{a}, -SR^{a}, -SF₅, -C(O)R^{a}, -C(O)OR^{a}, -OC(O)R^{a}, -OC(O)NR^{a}R^{b}, - NR^{a}COR^{b}, and -CONR^{a}R^{b}.

18. The compound, the pharmaceutically acceptable salt, the ester, the prodrug, the stereoisomer, or the isotopic derivative thereof according to any one of claims 1 to 13, wherein R' represents C₁-C₆ alkyl substituted with 0-3 substituents selected from the group consisting of: deuterated, halogen, C₁-C₆ alkyl, hydroxy(C₁-C₆ alkyl), -OR^{a}, -CN, NR^{a}R^{b}, C₁-C₆ haloalkyl, and C₁-C₆ haloalkoxy.

19. The compound, the pharmaceutically acceptable salt, the ester, the prodrug, the stereoisomer, or the isotopic derivative thereof according to claim 17, wherein R' represents C₃-C₁₀ cycloalkyl substituted with 0-3 substituents selected from the group consisting of: deuterated, halogen, C₁-C₆ alkyl, hydroxy(C₁-C₆ alkyl), -OR^{a}, -CN, NR^{a}R^{b}, C₁-C₆ haloalkyl, and C₃-C₁₀ haloalkoxy.

20. The compound, the pharmaceutically acceptable salt, the ester, the prodrug, the stereoisomer, or the isotopic derivative thereof according to claim 19, wherein R' represents cyclopropyl, cyclobutyl, cyclopentyl, or cyclohexyl, wherein the said cyclopropyl, cyclobutyl, cyclopentyl, or cyclohexyl can be furtherly substituted with 0-3 substituents selected from the group consisting of: deuterated, halogen, C₁-C₆ alkyl, hydroxy(C₁-C₆ alkyl), -OR^{a}, -CN, NR^{a}R^{b}, C₁-C₆ haloalkyl, and C₁-C₆ haloalkoxy.

21. The compound, the pharmaceutically acceptable salt, the ester, the prodrug, the stereoisomer, or the isotopic derivative thereof according to any one of claims 1 to 13, wherein R' represents 4- to 10- membered heterocycloalkyl, C₆-C₁₀ aryl, or 5- to 10- membered heteroaryl, wherein the said 4- to 10- membered heterocycloalkyl, C₆-C₁₀ aryl, or 5- to 10-membered heteroaryl can be furtherly substituted with 0-3 substituents selected from the group consisting of: deuterated, halogen, C₁-C₆ alkyl, hydroxy(C₁-C₆ alkyl), -OR^{a}, -CN, NR^{a}R^{b}, C₁-C₆ haloalkyl, and C₁-C₆ haloalkoxy.

22. The compound, the pharmaceutically acceptable salt, the ester, the prodrug, the stereoisomer, or the isotopic derivative thereof according to claim 21, wherein R' represents the following groups: 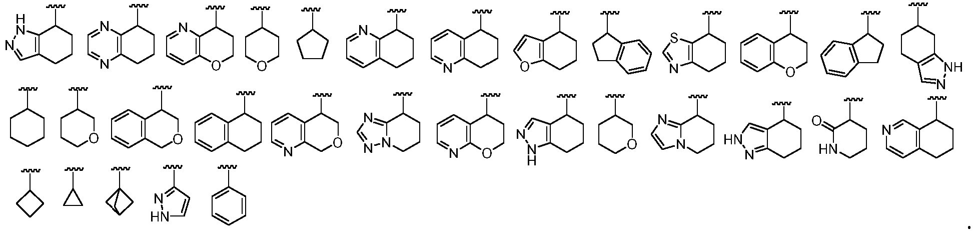 wherein, the above groups can be furtherly substituted with 0-3 substituents selected from the group consisting of: deuterated, halogen, C₁-C₆ alkyl, hydroxy(C₁-C₆ alkyl), -OR^{a}, -CN, NR^{a}R^{b}, C₁-C₆ haloalkyl, and C₁-C₆ haloalkoxy:

23. The compound, the pharmaceutically acceptable salt, the ester, the prodrug, the stereoisomer, or the isotopic derivative thereof according to claim 1, wherein R' represents any one of the following groups:

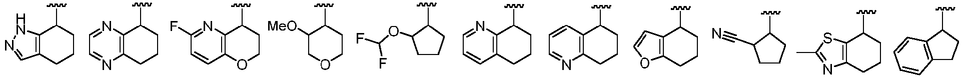

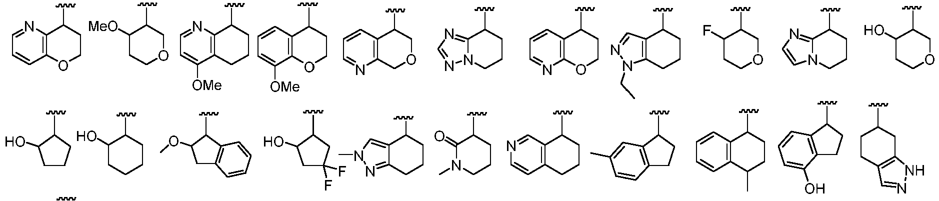

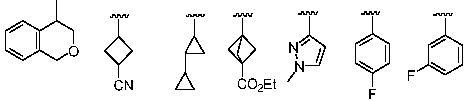

24. The compound, the pharmaceutically acceptable salt, the ester, the prodrug, the stereoisomer, or the isotopic derivative thereof according to any one of claims 1 to 23, **characterized in that** the compound structure is optionally selected from the following group:

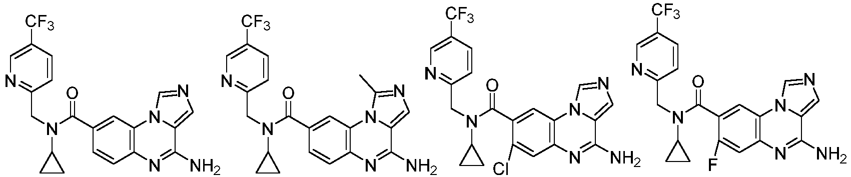

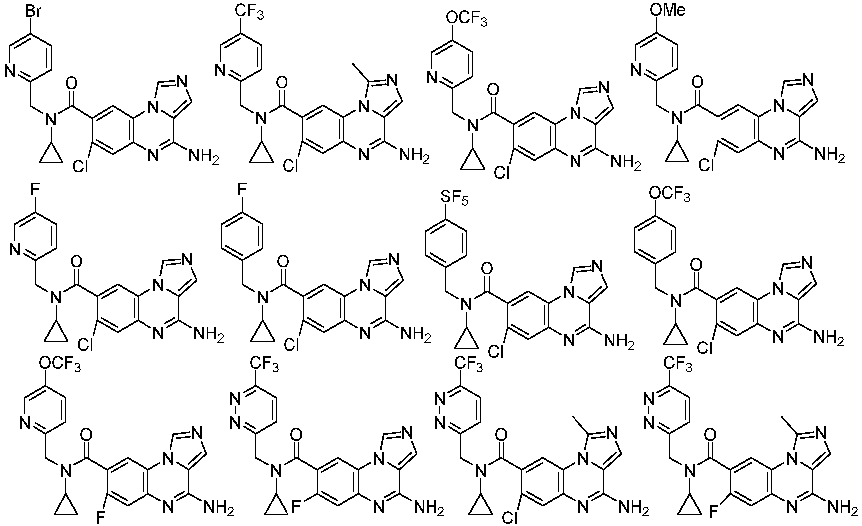

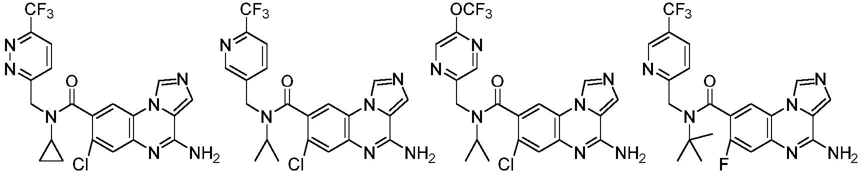

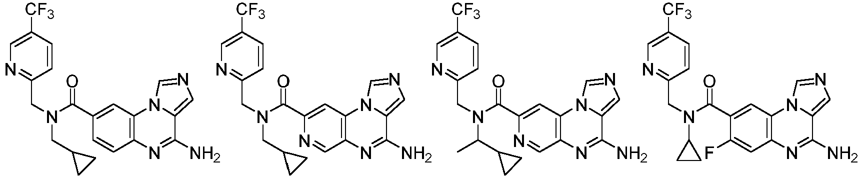

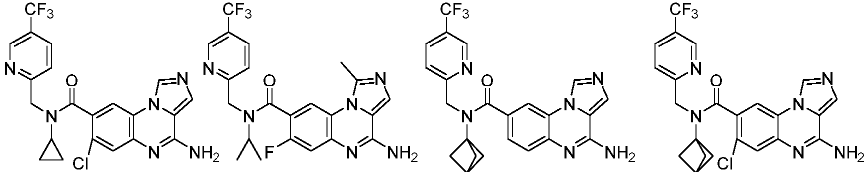

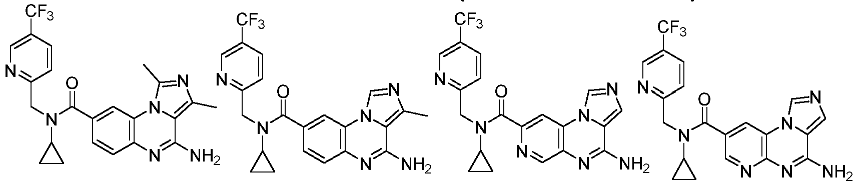

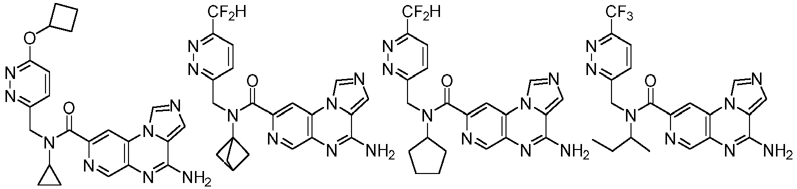

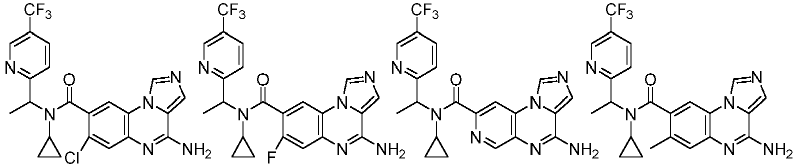

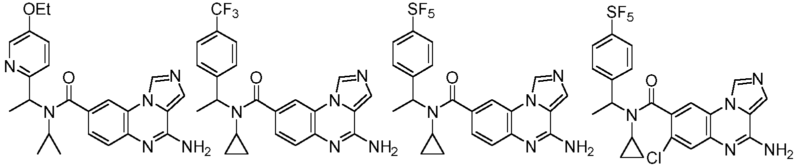

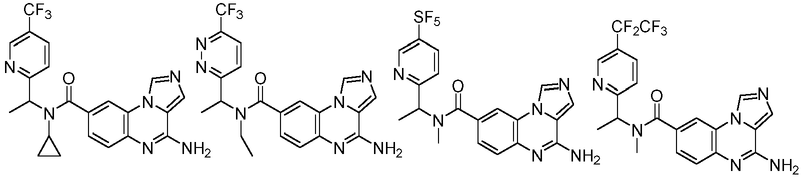

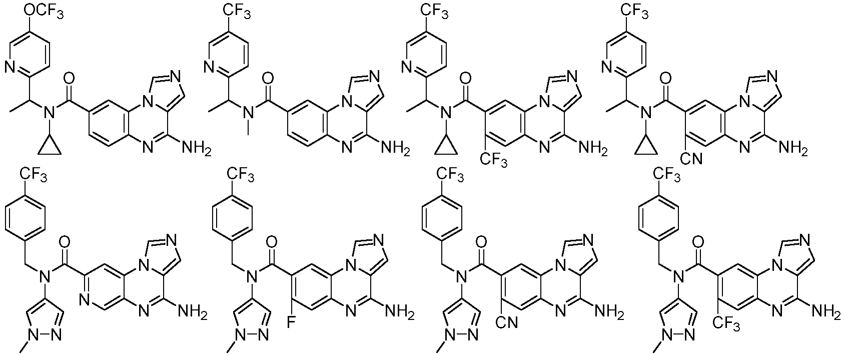

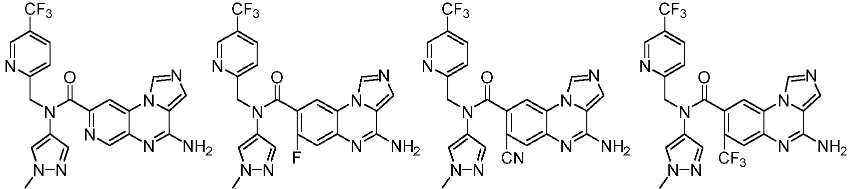

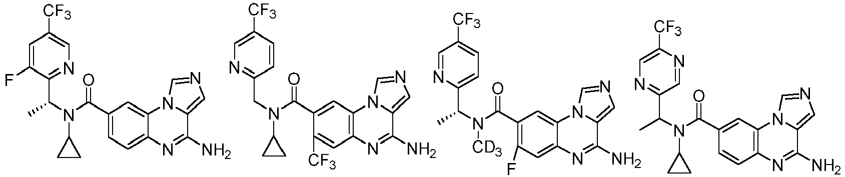

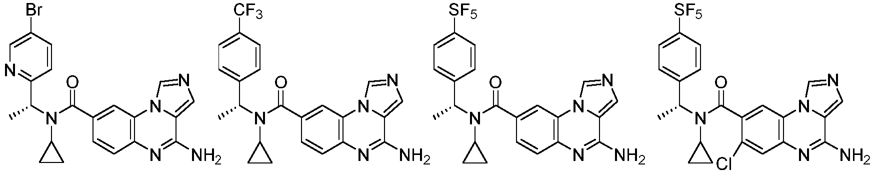

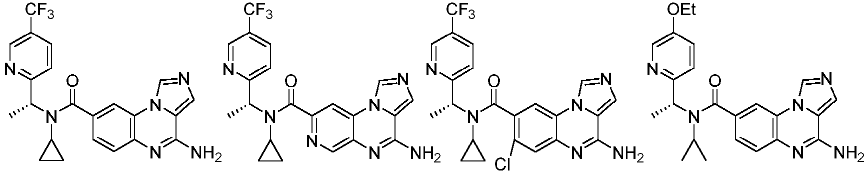

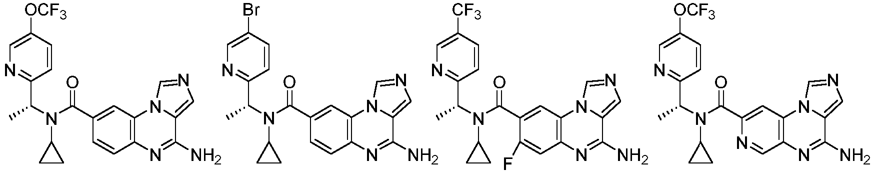

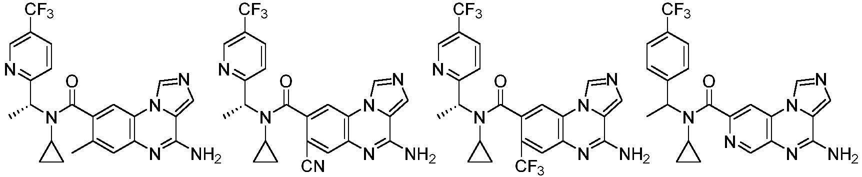

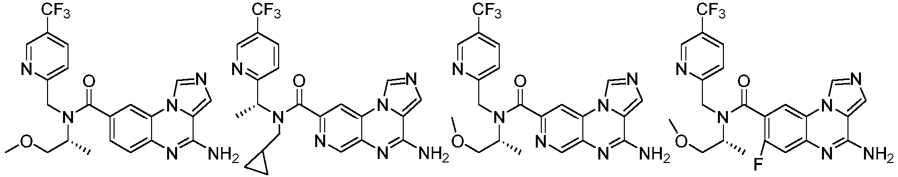

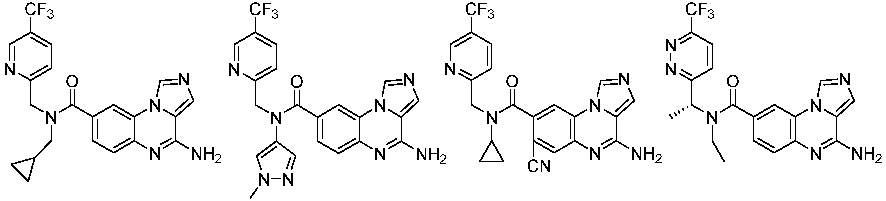

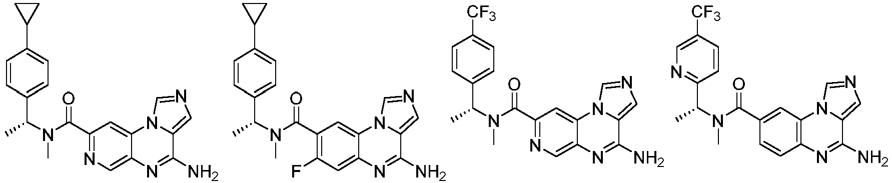

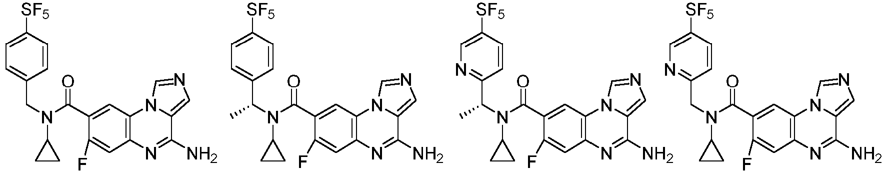

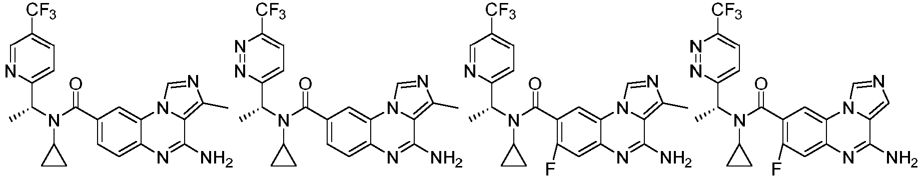

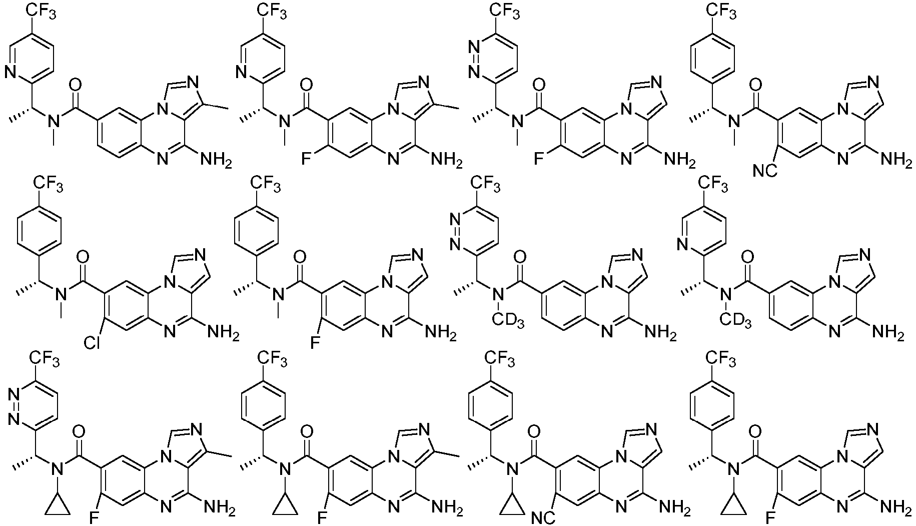

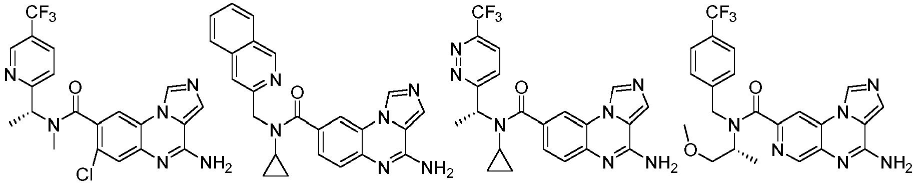

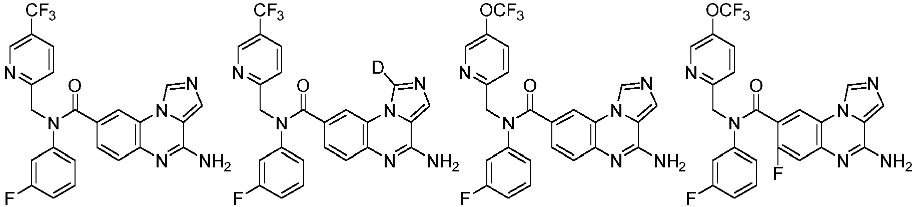

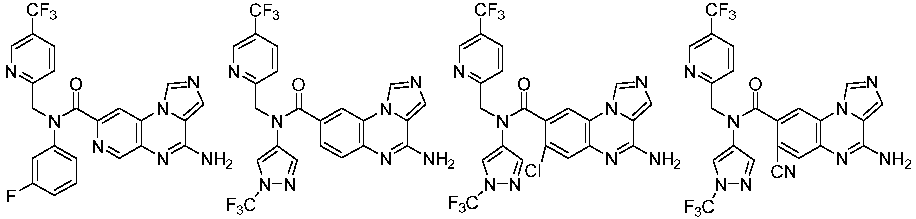

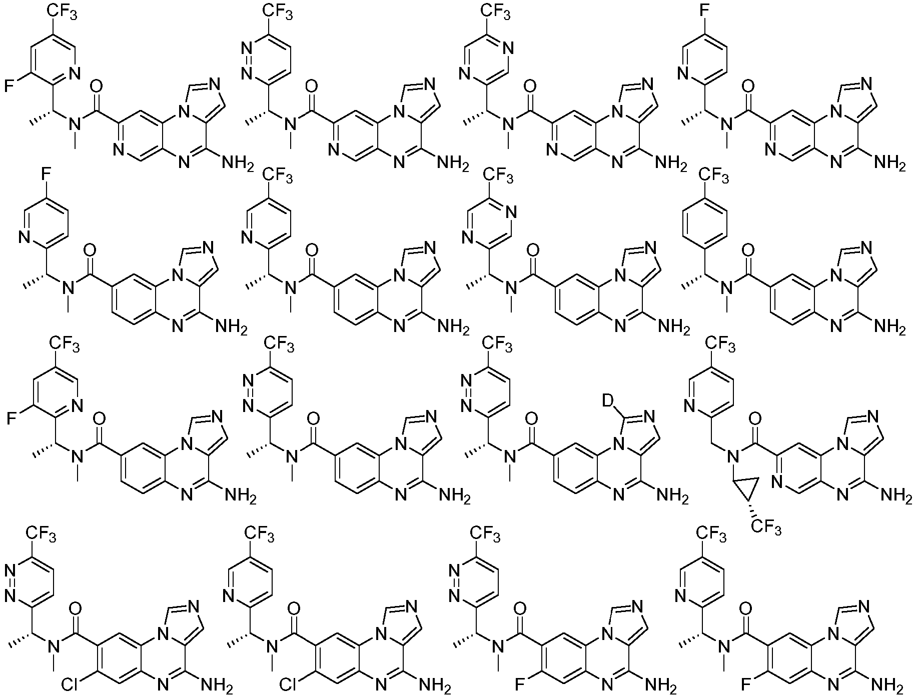

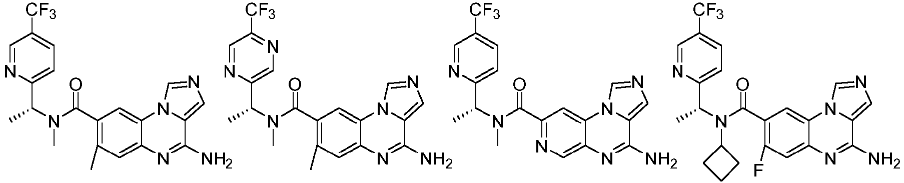

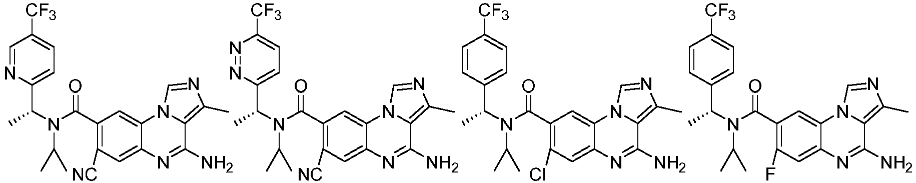

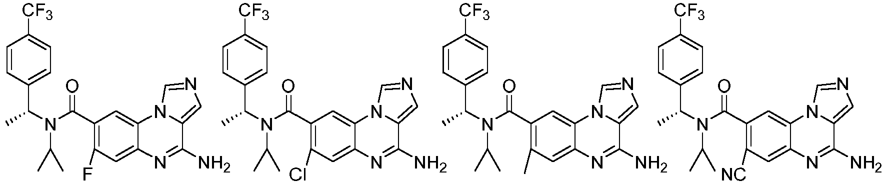

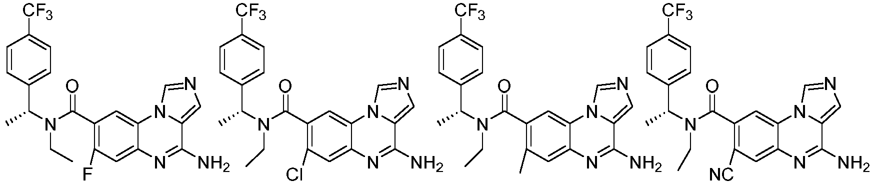

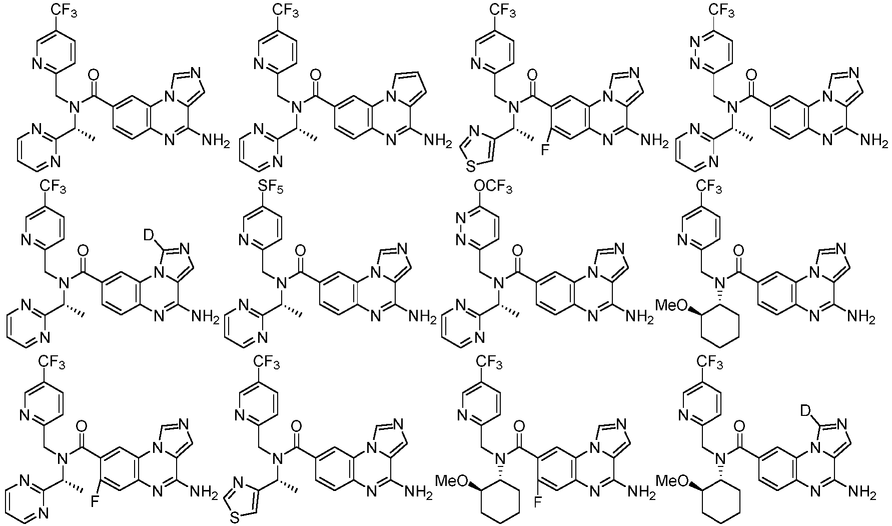

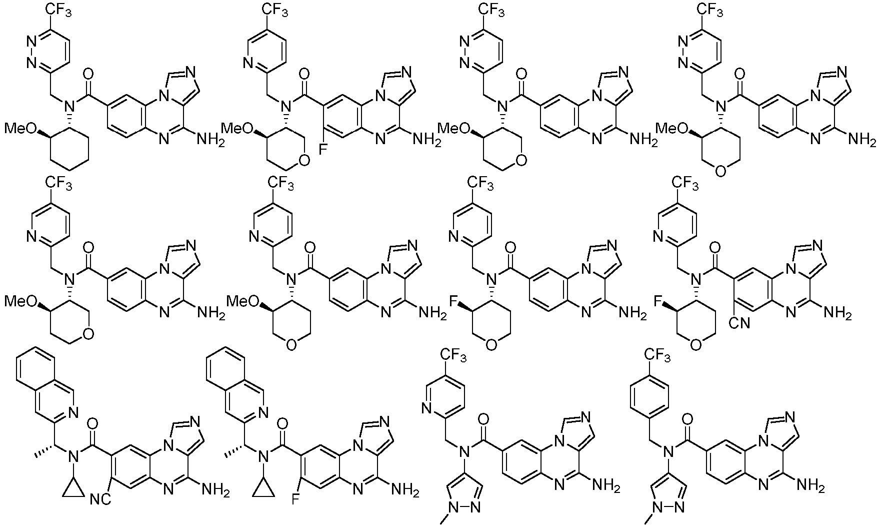

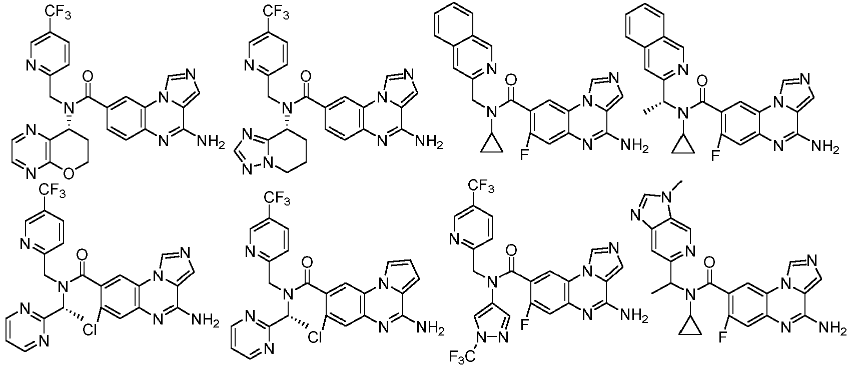

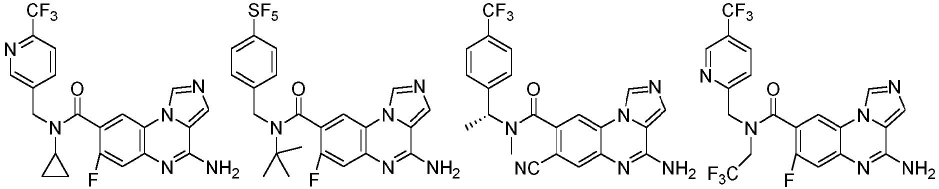

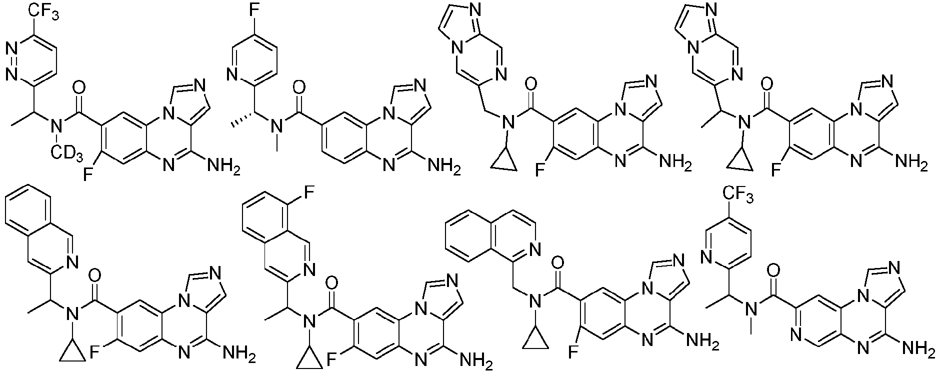

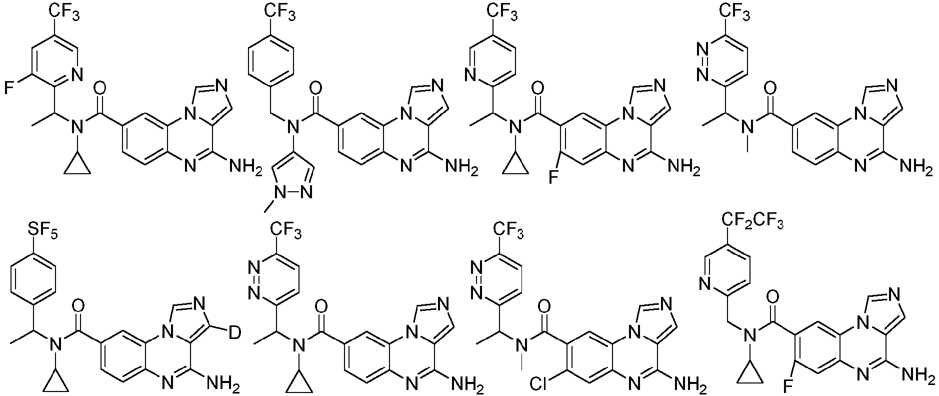

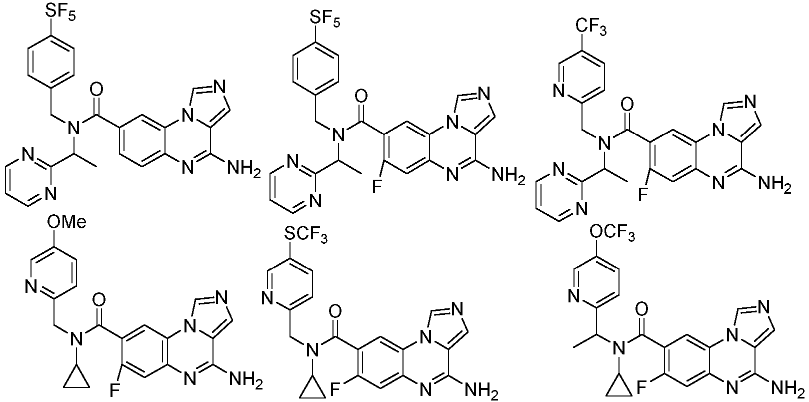

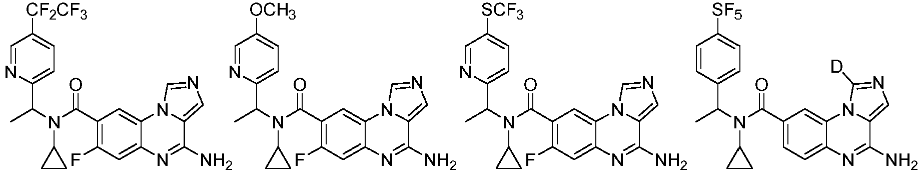

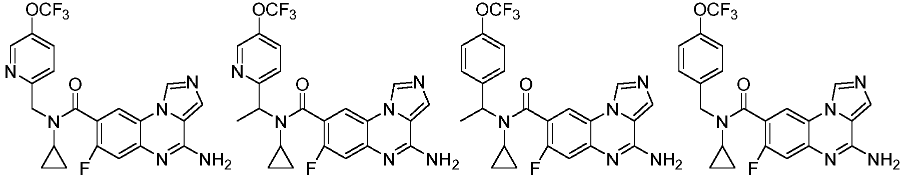

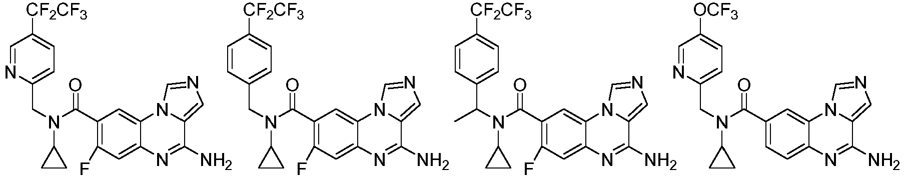

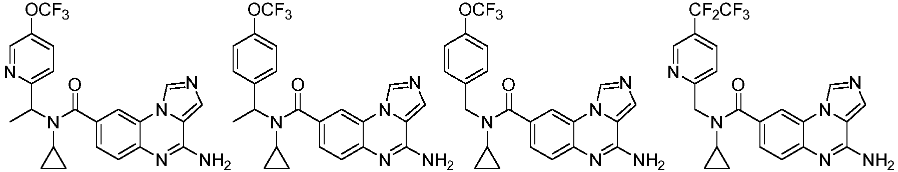

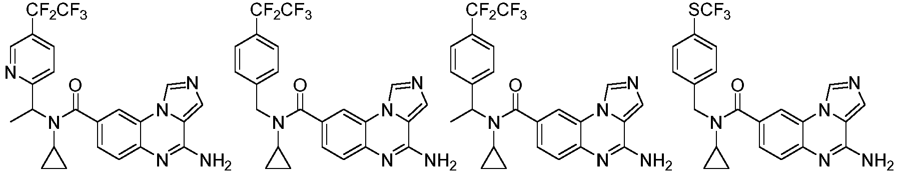

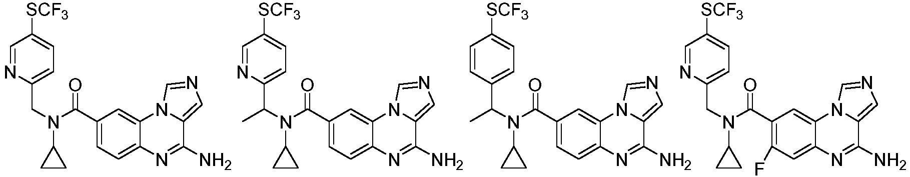

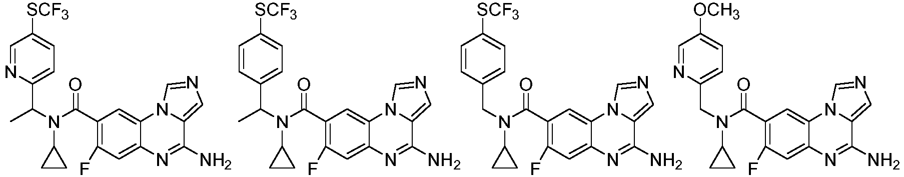

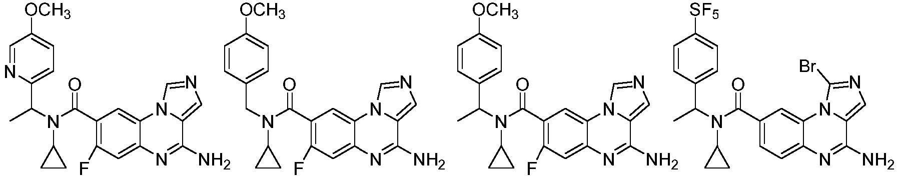

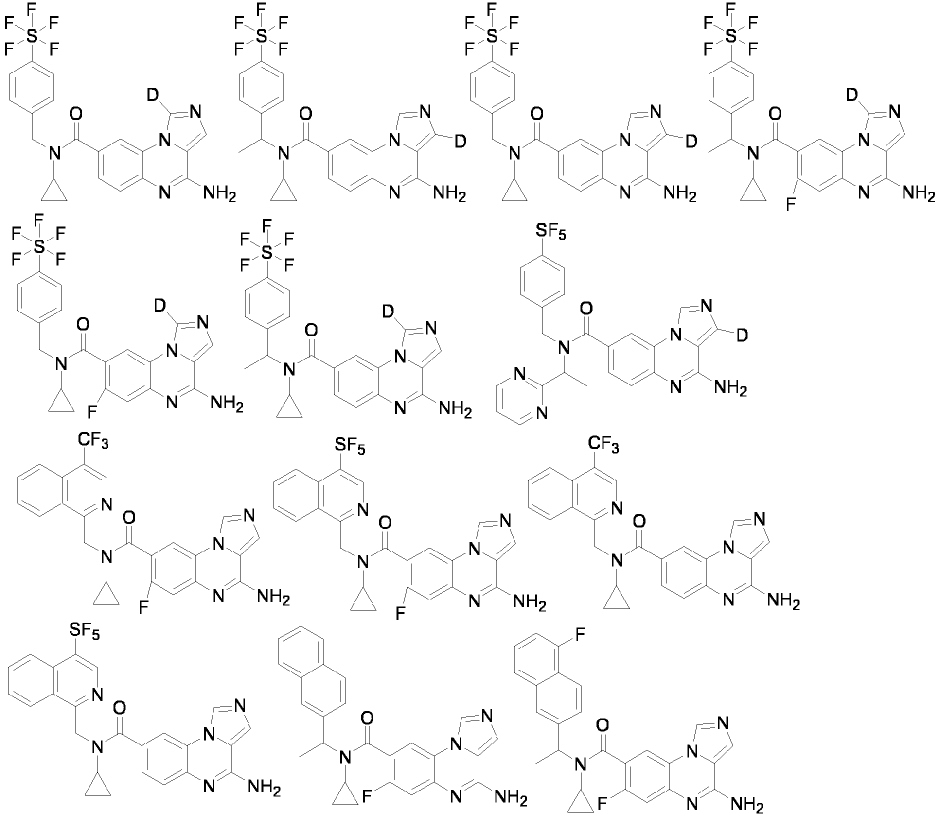

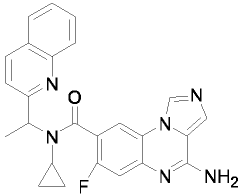

25. An isotopic derivative of the compound according to any one of claims 1 to 24, wherein at least one or more hydrogen atoms contained in the isotopic derivative are deuterium atoms.

26. A pharmaceutical composition, comprising the compound, the pharmaceutically acceptable salt, the ester, the prodrug, the stereoisomer, or the isotopic derivative thereof according to any one of claims 1 to 25 and the pharmaceutically acceptable carrier thereof.

27. The pharmaceutical composition according to claim 26, further comprising a second active substance, wherein the second active substance is an anti-tumor drug comprising one or more of chemotherapeutic drugs, targeted tumor therapy drugs and tumor therapy antibody drugs.

28. The compound, the pharmaceutically acceptable salt, the ester, the prodrug, the stereoisomer, or the isotopic derivative thereof according to any one of claims 1 to 27, a method for treating a disease by inhibiting the action of PRMT5 is provided.

29. The method according to claim 28, wherein the disease is a tumor.
